# EUROPEAN PATENT APPLICATION

(11) **EP 4 378 955 A1**
(43) Date of publication of application: **05.06.2024**
(21) Application number: 22848688.2
(22) Date of filing: 29.07.2022
(51) Int. Cl.: C07K 16/28, C07K 16/30, C12N 15/13, C12N 15/85, A61K 47/68, A61K 39/395, A61P 35/00, G01N 33/574

(54) **ANTI-DLL3 ANTIBODY AND PREPARATION METHOD THEREFOR, DRUG CONJUGATE AND APPLICATION THEREOF**

(30) Priority: 30.07.2021 WO PCT/CN2021/109517; 11.03.2022 CN 202210239591
(71) Applicant: Shanghai Fudan-Zhangjiang Bio-Pharmaceutical Co., Ltd., Shanghai 201210 (CN)
(72) Inventor: GUO, Qingsong, Shanghai 201210 (CN); SHEN, Yijun, Shanghai 201210 (CN); YANG, Tong, Shanghai 201210 (CN); GAO, Bei, Shanghai 201210 (CN); WU, Fang, Shanghai 201210 (CN); MENG, Likai, Shanghai 201210 (CN); WANG, Baoxia, Shanghai 201210 (CN); ZHANG, Wenbo, Shanghai 201210 (CN)
(74) Representative: Dehns
(86) International application number: PCT/CN2022/109050
(87) International publication number: WO 2023/006084

(57) **Abstract**

Disclosed are an anti-DLL3 antibody and a preparation method therefor, a drug conjugate and application thereof. The anti-DLL3 antibody in the present invention has good internalization activity, has better binding activity with human DLL3 protein, and has strong affinity at the protein level. The antibody conjugated drug targeting DLL3 in the present invention has good druggability, biological activity, and anti-tumor activity *in vitro* and *vivo,* and can achieve an application of cytotoxic drugs in treating tumor patients comprising SCLC and having neuroendocrine characteristics.

## Description

The present application claims priority to PCT patent application PCT/CN2021/109517 filed on July 30, 2021, and Chinese patent application CN2022102395912 filed on March 11, 2022. The contents of the above patent applications are incorporated herein by reference in their entireties.

### TECHNICAL FIELD

The present disclosure relates to the fields of biotechnology and medicine, and particularly relates to an anti-DLL3 antibody and preparation method therefor, drug conjugate and application thereof.

### BACKGROUND

Small cell lung cancer (SCLC) is the most malignant type of lung cancer, characterized by rapid progression, early metastasis, and easy recurrence. It accounts for approximately 15%-20% of new-onset lung cancer cases and is closely associated with long-term smoking. SCLC patients are primarily divided into patients in limited-stage and extensive-stage, of which patients in the extensive-stage are the majority, accounting for about 70% of SCLC patients. At present, the treatment of SCLC is primarily based on chemotherapy. The first-line chemotherapy is mainly a combination therapy based on platinum drugs, with etoposide + cisplatin or carboplatin (EP or CE regimen) being the most commonly used. In guidelines, it is recommended that both limited-stage and extensive-stage SCLC patients should receive 4-6 courses of combination therapy with cisplatin/carboplatin. The response rate for first-line treatment in limited-stage SCLC is 70%-90%, while in extensive-stage SCLC, it is 50%-60%. Currently, within one year of first-line treatment for SCLC, about 80% of patients with limited-stage and nearly all patients with extensive-stage experience relapse or progression. Therefore, the efficacy of second-line treatment significantly impacts the ultimate survival outcomes of SCLC patients. Topotecan, as a standard monotherapy for second-line treatment of SCLC, has a response rate of about 22%, with an overall survival of approximately 8 months. However, for patients with drug-resistant relapse, the response rate drops to around 4%, and the median survival is only 5 months, indicating extremely limited clinical benefit and a significant unmet clinical need. In summary, due to the high recurrence and drug resistance rates of SCLC, coupled with limited options for second-line treatment, it results in limited survival benefits, involving numerous challenges in treatment. There is an urgent need for new therapeutic methods to address these unmet clinical needs. Current research indicates that the inhibition of the Notch pathway is highly associated with the development and progression of SCLC. Therefore, its ligand, DLL3, is considered one of the most promising targets for the treatment of SCLC.

DLL3 is an atypical Notch pathway ligand identified through high-throughput sequencing. It is a single transmembrane protein composed of 619 amino acids. Its complete structure includes one DSL domain, one intracellular domain, and six epidermal growth factor-like domains (namely domains EGF1, EGF2, EGF3, EGF4, EGF5, and EGF6), making it a crucial target in the development and progression of SCLC. Immunohistochemistry reveals that this target is hardly expressed in normal tissues and is not expressed in other types of tumors. However, it is specifically and abundantly expressed in neuroendocrine tumors. Approximately 80% of SCLC tumor tissues and cancer cell surfaces present high levels of DLL3 expression. Similarly, about 85% of recurrent SCLC cases exhibit high expression of the DLL3 protein, making it a constitutive expression receptor of SCLC. The lack of DLL3 expression in normal tissues and its specific expression in neuroendocrine tumors such as SCLC making it a viable target for the development of antibodies or antibody-drug conjugates (ADCs).

Currently, various drugs targeting the DLL3 target are under development, mainly including bispecific antibodies, cell therapies, and ADCs (antibody-drug conjugates)(e.g., see CN104520324A). Rova-T, developed by AbbVie, is the first ADC drug targeting the DLL3 target and the first targeting therapeutic drug for SCLC in clinical research. This drug utilizes DLL3 expressed on the surface of tumor cells to recognize the tumor cells and deliver the cytotoxic drug PBD into the tumor cells, achieving the effect of targeted killing of the tumor cells. However, in phase III clinical trials, due to the serious toxic side effects of the cytotoxic agent PBD, which results in the intolerance of the patients, leading to insufficient efficacy, the development of this drug has been terminated.

### CONTENT OF THE PRESENT INVENTION

The technical problem addressed by the present disclosure is the inadequacies of existing anti-DLL3 antibodies and the current clinical situation that ADC drugs targeting DLL3 have not been successfully developed. To address these issues, the present disclosure provides a novel anti-DLL3 antibody, an ADC targeting DLL3, an intermediate thereof, a preparation method therefor, and a use thereof. Addressing the deficiency in prior art of lacking antibody-drug conjugates targeting DLL3, the antibody-drug conjugate described in the present disclosure can effectively treat patients with tumors exhibiting neuroendocrine characteristics, including SCLC. The present disclosure primarily employs the following technical means to solve the aforementioned technical problems.

The present disclosure provides an anti-DLL3 antibody comprising a heavy chain variable region (VH) and a light chain variable region (VL); wherein

the VH comprises complementarity-determining regions (CDRs) as follows or mutations thereof: VH CDR1 as shown in amino acid sequence of SEQ ID NO: 9, 19, 29, 39, 59, 69, 79, 89, 99, or 63, VH CDR2 as shown in amino acid sequence of SEQ ID NO: 10, 20, 30, 40, 60, 70, 80, 90, 100, 110, or 83, and/or VH CDR3 as shown in amino acid sequence of SEQ ID NO: 11, 21, 31, 41, 61, 71, 81, 91, 101, 111, or 93;

the VL comprises CDRs as follows or mutations thereof: VL CDR1 as shown in amino acid sequence of SEQ ID NO: 12, 32, 42, 62, 72, 82, 92, 102, 112, or 103, VL CDR2 as shown in GAS, GAT, TTS, NAK, YTS, RAN, WAS, FTS, or NAN, and/or VL CDR3 as shown in amino acid sequence of SEQ ID NO: 14, 24, 34, 44, 64, 74, 84, 94, 104, 114, or 113;

wherein the mutations having 3, 2, or 1 amino acid insertions, deletions, or substitutions in the amino acid sequence of the CDRs.

In the present application, the term "amino acid mutation" in a context like "having 3, 2, or 1 amino acid insertions, deletions, or substitutions" refers to an amino acid mutation in a varied sequence relative to the original amino acid sequence, which includes insertion, deletion, or substitution of amino acids based on the original amino acid sequence. An exemplary explanation is that the mutations in CDR can involve mutations of 3, 2, or 1 amino acid(s), and the same or different number of amino acid residues can optionally be selected for mutation between these CDRs. For example, there could be a mutation of one amino acid in CDR1, with no amino acid mutations in CDR2 and CDR3.

In the present application, the mutations may include those currently known to those skilled in the art, such as mutations that might be made to antibodies during their production or application. For instance, mutations may be made at sites of potential post-translational modifications (PTMs), especially in the CDR regions. These include mutations related to antibody aggregation, susceptible to asparagine deamidation (at sites like NG, NS, NH, etc.), susceptible to aspartic acid isomerization (at DG, DP sites), susceptible to N-glycosylation (at N-{P}S/T sites), and susceptible to oxidation, and other related mutations.

Preferably, the amino acid sequences of VH CDR1, VH CDR2, and VH CDR3 comprised in the VH are respectively as shown in SEQ ID NO: 9, 10, and 11; or the amino acid sequences of VH CDR1, VH CDR2, and VH CDR3 comprised in the VH are respectively as shown in SEQ ID NO: 19, 20, and 21; or the amino acid sequences of VH CDR1, VH CDR2, and VH CDR3 comprised in the VH are respectively as shown in SEQ ID NO: 29, 30, and 31; or the amino acid sequences of VH CDR1, VH CDR2, and VH CDR3 comprised in the VH are respectively as shown in SEQ ID NO: 39, 40, and 41; or the amino acid sequences of VH CDR1, VH CDR2, and VH CDR3 comprised in the VH are respectively as shown in SEQ ID NO: 59, 60, and 61; or the amino acid sequences of VH CDR1, VH CDR2, and VH CDR3 comprised in the VH are respectively as shown in SEQ ID NO: 69, 70, and 71; or the amino acid sequences of VH CDR1, VH CDR2, and VH CDR3 comprised in the VH are respectively as shown in SEQ ID NO: 79, 80, and 81; or the amino acid sequences of VH CDR1, VH CDR2, and VH CDR3 comprised in the VH are respectively as shown in SEQ ID NO: 89, 90, and 91; or the amino acid sequences of VH CDR1, VH CDR2, and VH CDR3 comprised in the VH are respectively as shown in SEQ ID NO: 99, 100, and 101; or the amino acid sequences of VH CDR1, VH CDR2, and VH CDR3 comprised in the VH are respectively as shown in SEQ ID NO: 89, 110, and 111; or the amino acid sequences of VH CDR1, VH CDR2, and VH CDR3 comprised in the VH are respectively as shown in SEQ ID NO: 63, 83, and 93.

Preferably, the amino acid sequences of VL CDR1, VL CDR2, and VL CDR3 comprised in the VL are respectively as shown in SEQ ID NO: 12, GAS, and SEQ ID NO: 14; or the amino acid sequences of VL CDR1, VL CDR2, and VL CDR3 comprised in the VL are respectively as shown in SEQ ID NO: 12, GAS, and SEQ ID NO: 24; or the amino acid sequences of VL CDR1, VL CDR2, and VL CDR3 comprised in the VL are respectively as shown in SEQ ID NO: 32, GAT, and SEQ ID NO: 34; or the amino acid sequences ofVL CDR1, VL CDR2, and VL CDR3 comprised in the VL are respectively as shown in SEQ ID NO: 42, TTS, and SEQ ID NO: 44; or the amino acid sequences of VL CDR1, VL CDR2, and VL CDR3 comprised in the VL are respectively as shown in SEQ ID NO: 62, NAK, and SEQ ID NO: 64; or the amino acid sequences of VL CDR1, VL CDR2, and VL CDR3 comprised in the VL are respectively as shown in SEQ ID NO: 72, NAK, and SEQ ID NO: 74; or the amino acid sequences of VL CDR1, VL CDR2, and VL CDR3 comprised in the VL are respectively as shown in SEQ ID NO: 82, YTS, and SEQ ID NO: 84; or the amino acid sequences of VL CDR1, VL CDR2, and VL CDR3 comprised in the VL are respectively as shown in SEQ ID NO: 92, RAN, and SEQ ID NO: 94; or the amino acid sequences of VL CDR1, VL CDR2, and VL CDR3 comprised in the VL are respectively as shown in SEQ ID NO: 102, WAS, and SEQ ID NO: 104; or the amino acid sequences of VL CDR1, VL CDR2, and VL CDR3 comprised in the VL are respectively as shown in SEQ ID NO: 112, FTS, and SEQ ID NO: 114; or the amino acid sequences of VL CDR1, VL CDR2, and VL CDR3 comprised in the VL are respectively as shown in SEQ ID NO: 103, NAN, and 113.

In one preferred embodiment, in the anti-DLL3 antibody, the amino acid sequences of VH CDR1, VH CDR2, and VH CDR3 comprised in the VH are respectively as shown in SEQ ID NO: 9, 10, and 11; and the amino acid sequences of VL CDR1, VL CDR2, and VL CDR3 comprised in the VL are respectively as shown in SEQ ID NO: 12, GAS, and SEQ ID NO: 14. In one preferred embodiment, in the anti-DLL3 antibody, the amino acid sequences of VH CDR1, VH CDR2, and VH CDR3 comprised in the VH are respectively as shown in SEQ ID NO: 19, 20, and 21; and the amino acid sequences of VL CDR1, VL CDR2, and VL CDR3 comprised in the VL are respectively as shown in SEQ ID NO: 12, GAS, and SEQ ID NO: 24. In one preferred embodiment, in the anti-DLL3 antibody, the amino acid sequences of VH CDR1, VH CDR2, and VH CDR3 comprised in the VH are respectively as shown in SEQ ID NO: 29, 30, and 31; and the amino acid sequences of VL CDR1, VL CDR2, and VL CDR3 comprised in the VL are respectively as shown in SEQ ID NO: 32, GAT, and SEQ ID NO: 34. In one preferred embodiment, in the anti-DLL3 antibody, the amino acid sequences of VH CDR1, VH CDR2, and VH CDR3 comprised in the VH are respectively as shown in SEQ ID NO: 39, 40, and 41; and the amino acid sequences of VL CDR1, VL CDR2, and VL CDR3 comprised in the VL are respectively as shown in SEQ ID NO: 42, TTS, and SEQ ID NO: 44. In one preferred embodiment, in the anti-DLL3 antibody, the amino acid sequences of VH CDR1, VH CDR2, and VH CDR3 comprised in the VH are respectively as shown in SEQ ID NO: 59, 60, and 61; and the amino acid sequences of VL CDR1, VL CDR2, and VL CDR3 comprised in the VL are respectively as shown in SEQ ID NO: 62, NAK, and SEQ ID NO: 64. In one preferred embodiment, in the anti-DLL3 antibody, the amino acid sequences of VH CDR1, VH CDR2, and VH CDR3 comprised in the VH are respectively as shown in SEQ ID NO: 69, 70, and 71; and the amino acid sequences of VL CDR1, VL CDR2, and VL CDR3 comprised in the VL are respectively as shown in SEQ ID NO: 72, NAK, and SEQ ID NO: 74. In one preferred embodiment, in the anti-DLL3 antibody, the amino acid sequences of VH CDR1, VH CDR2, and VH CDR3 comprised in the VH are respectively as shown in SEQ ID NO: 79, 80, and 81; and the amino acid sequences of VL CDR1, VL CDR2, and VL CDR3 comprised in the VL are respectively as shown in SEQ ID NO: 82, YTS, and SEQ ID NO: 84. In one preferred embodiment, in the anti-DLL3 antibody, the amino acid sequences of VH CDR1, VH CDR2, and VH CDR3 comprised in the VH are respectively as shown in SEQ ID NO: 89, 90, and 91; and the amino acid sequences of VL CDR1, VL CDR2, and VL CDR3 comprised in the VL are respectively as shown in SEQ ID NO: 92, RAN, and SEQ ID NO: 94. In one preferred embodiment, in the anti-DLL3 antibody, the amino acid sequences of VH CDR1, VH CDR2, and VH CDR3 comprised in the VH are respectively as shown in SEQ ID NO: 99, 100, and 101; and the amino acid sequences of VL CDR1, VL CDR2, and VL CDR3 comprised in the VL are respectively as shown in SEQ ID NO: 102, WAS, and SEQ ID NO: 104. In one preferred embodiment, in the anti-DLL3 antibody, the amino acid sequences of VH CDR1, VH CDR2, and VH CDR3 comprised in the VH are respectively as shown in SEQ ID NO: 89, 110, and 111; and the amino acid sequences of VL CDR1, VL CDR2, and VL CDR3 comprised in the VL are respectively as shown in SEQ ID NO: 112, FTS, and SEQ ID NO: 114. In one preferred embodiment, in the anti-DLL3 antibody, the amino acid sequences of VH CDR1, VH CDR2, and VH CDR3 comprised in the VH are respectively as shown in SEQ ID NO: 63, 83, and 93; and the amino acid sequences of VL CDR1, VL CDR2, and VL CDR3 comprised in the VL are respectively as shown in SEQ ID NO: 103, NAN, and 113.

Preferably, in the anti-DLL3 antibody, the heavy chain variable region (VH) further comprises a heavy chain variable region framework region (VH FWR), wherein the VH FWR is the heavy chain variable region framework region of a human or murine antibody. In one preferred embodiment of the present disclosure, the VH may comprise the amino acid sequence as shown in SEQ ID NO: 15, 25, 35, 45, 65, 75, 85, 95, 105, 13, or 22, or mutations thereof.

Preferably, in the anti-DLL3 antibody, the light chain variable region (VL) further comprises a light chain variable region framework region (VL FWR), wherein the VL FWR is the light chain variable region framework region of a human or murine antibody. In one preferred embodiment of the present disclosure, the VL may comprise the amino acid sequence as shown in SEQ ID NO: 16, 26, 36, 46, 66, 76, 86, 96, 106, 33, or 23, or mutations thereof.

In one preferred embodiment, the VH comprises the amino acid sequence as shown in SEQ ID NO: 15 or mutations thereof, and the VL comprises the amino acid sequence as shown in SEQ ID NO: 16 or mutations thereof. In one preferred embodiment, the VH comprises the amino acid sequence as shown in SEQ ID NO: 25 or mutations thereof, and the VL comprises the amino acid sequence as shown in SEQ ID NO: 26 or mutations thereof. In one preferred embodiment, the VH comprises the amino acid sequence as shown in SEQ ID NO: 35 or mutations thereof, and the VL comprises the amino acid sequence as shown in SEQ ID NO: 36 or mutations thereof. In one preferred embodiment, the VH comprises the amino acid sequence as shown in SEQ ID NO: 45 or mutations thereof, and the VL comprises the amino acid sequence as shown in SEQ ID NO: 46 or mutations thereof. In one preferred embodiment, the VH comprises the amino acid sequence as shown in SEQ ID NO: 65 or mutations thereof, and the VL comprises the amino acid sequence as shown in SEQ ID NO: 66 or mutations thereof. In one preferred embodiment, the VH comprises the amino acid sequence as shown in SEQ ID NO: 75 or mutations thereof, and the VL comprises the amino acid sequence as shown in SEQ ID NO: 76 or mutations thereof. In one preferred embodiment, the VH comprises the amino acid sequence as shown in SEQ ID NO: 85 or mutations thereof, and the VL comprises the amino acid sequence as shown in SEQ ID NO: 86 or mutations thereof. In one preferred embodiment, the VH comprises the amino acid sequence as shown in SEQ ID NO: 95 or mutations thereof, and the VL comprises the amino acid sequence as shown in SEQ ID NO: 96 or mutations thereof. In one preferred embodiment, the VH comprises the amino acid sequence as shown in SEQ ID NO: 105 or mutations thereof, and the VL comprises the amino acid sequence as shown in SEQ ID NO: 106 or mutations thereof. In one preferred embodiment, the VH comprises the amino acid sequence as shown in SEQ ID NO: 13 or mutations thereof, and the VL comprises the amino acid sequence as shown in SEQ ID NO: 33 or mutations thereof. In one preferred embodiment, the VH comprises the amino acid sequence as shown in SEQ ID NO: 22 or mutations thereof, and the VL comprises the amino acid sequence as shown in SEQ ID NO: 23 or mutations thereof.

The mutations involve the deletion, substitution, or addition of one or more amino acid residues in the amino acid sequence of VH and/or VL. The amino acid sequence of the mutation has at least 85% sequence identity with the amino acid sequence of VH and/or VL and maintains or improves the binding ability of the antibody to DLL3. The at least 85% sequence identity is preferably at least 90% sequence identity, more preferably at least 95%, 96%, 97%, 98% sequence identity, and most preferably at least 99% sequence identity.

In the present disclosure, the amino acid sequences of the CDRs are all presented according to the Kabat definition rules (the sequences in this application are also presented according to the Kabat definition rules). However, it is well known to those skilled in the art that there are various methods to define the CDRs of an antibody in this art, such as the Kabat definition rules based on sequence variability (see, Kabat et al., Sequences of Proteins of Immunological Interest, 5th Edition, National Institutes of Health, Bethesda, Maryland (1991)) and the Chothia definition rules based on the structural loop region location (see J Mol Biol 273:927-48, 1997). It should be understood by those skilled in the art that, unless otherwise specified, the terms "CDR" and "complementarity-determining region" for a given antibody or its region (such as the variable region) are to be understood as encompassing the complementarity-determining region defined by any of the known methods described in the present disclosure. Although the scope of protection requested in the present disclosure is based on sequences shown according to the Kabat definition rules, amino acid sequences corresponding to other CDR definition rules should also fall within the scope of protection of the present disclosure.

Preferably, the anti-DLL3 antibody is selected from the group consisting of a full-length antibody, Fab, Fab', F(ab')2, Fv, preferably scFv, bispecific antibody, multispecific antibody, heavy chain antibody, or single domain antibody.

In one preferred embodiment, the anti-DLL3 antibody is a full-length antibody, comprising a heavy chain and a light chain; a heavy chain constant region comprised in the heavy chain is preferably derived from human or murine antibody heavy chain constant region; a light chain constant region comprised in the light chain is preferably derived from human or murine antibody light chain constant region. Preferably, the human light chain constant region is a human κ or λ light chain constant region; the human heavy chain constant region is a human IgG1, IgG2, IgG3, or IgG4.

In one preferred embodiment, the heavy chain comprises the amino acid sequence as shown in SEQ ID NO: 17, 27, 37, 47, 67, 77, 87, 97, 107, 43, or 73, or mutations thereof, and/or the light chain comprises the amino acid sequence as shown in SEQ ID NO: 18, 28, 38, 48, 68, 78, 88, 98, 108, 53, or 109, or mutations thereof.

In one preferred embodiment, in the full-length antibody, the heavy chain comprises the amino acid sequence as shown in SEQ ID NO: 17 or mutations thereof, and the light chain comprises the amino acid sequence as shown in SEQ ID NO: 18 or mutations thereof. In one preferred embodiment, the heavy chain comprises the amino acid sequence as shown in SEQ ID NO: 27 or mutations thereof, and the light chain comprises the amino acid sequence as shown in SEQ ID NO: 28 or mutations thereof. In one preferred embodiment, the heavy chain comprises the amino acid sequence as shown in SEQ ID NO: 37 or mutations thereof, and the light chain comprises the amino acid sequence as shown in SEQ ID NO: 38 or mutations thereof. In one preferred embodiment, the heavy chain comprises the amino acid sequence as shown in SEQ ID NO: 47 or mutations thereof, and the light chain comprises the amino acid sequence as shown in SEQ ID NO: 48 or mutations thereof. In one preferred embodiment, the heavy chain comprises the amino acid sequence as shown in SEQ ID NO: 67 or mutations thereof, and the light chain comprises the amino acid sequence as shown in SEQ ID NO: 68 or mutations thereof. In one preferred embodiment, the heavy chain comprises the amino acid sequence as shown in SEQ ID NO: 77 or mutations thereof, and the light chain comprises the amino acid sequence as shown in SEQ ID NO: 78 or mutations thereof. In one preferred embodiment, the heavy chain comprises the amino acid sequence as shown in SEQ ID NO: 87 or mutations thereof, and the light chain comprises the amino acid sequence as shown in SEQ ID NO: 88 or mutations thereof. In one preferred embodiment, the heavy chain comprises the amino acid sequence as shown in SEQ ID NO: 97 or mutations thereof, and the light chain comprises the amino acid sequence as shown in SEQ ID NO: 98 or mutations thereof. In one preferred embodiment, the heavy chain comprises the amino acid sequence as shown in SEQ ID NO: 107 or mutations thereof, and the light chain comprises the amino acid sequence as shown in SEQ ID NO: 108 or mutations thereof. In one preferred embodiment, the heavy chain comprises the amino acid sequence as shown in SEQ ID NO: 43 or mutations thereof, and the light chain comprises the amino acid sequence as shown in SEQ ID NO: 53 or mutations thereof. In one preferred embodiment, the heavy chain comprises the amino acid sequence as shown in SEQ ID NO: 73 or mutations thereof, and the light chain comprises the amino acid sequence as shown in SEQ ID NO: 109 or mutations thereof.

The mutations involve the deletion, substitution, or addition of one or more amino acid residues in the amino acid sequence of the heavy chain and/or the light chain. The amino acid sequence of the mutation has at least 85% sequence identity with the amino acid sequence of the heavy chain and/or the light chain and maintains or improves the binding ability of the antibody to DLL3. The at least 85% sequence identity is preferably at least 90% sequence identity, more preferably at least 95%, 96%, 97%, or 98% sequence identity, and most preferably at least 99% sequence identity.

Another aspect of the present disclosure further provides an anti-DLL3 antibody, which competitively binds to the DLL3 protein with the anti-DLL3 antibody described above.

Another aspect of the present disclosure provides an isolated nucleic acid encoding the anti-DLL3 antibody as described above.

Another aspect of the present disclosure provides a recombinant expression vector comprising the isolated nucleic acid as described above. Preferably, the expression vector comprises a eukaryotic cell expression vector and/or a prokaryotic cell expression vector.

Another aspect of the present disclosure provides a transformant comprising the recombinant expression vector as described above. Preferably, the host cell of the transformant is a prokaryotic and/or a eukaryotic cell, wherein the prokaryotic cell is preferably *E. coli* cell such as TG1 or BL21 cell, and the eukaryotic cell is preferably HEK293 cell or CHO cell.

Another aspect of the present disclosure provides an antibody-drug conjugate (ADC), with the general structure formula: Ab-(L₃-L₂-L₁-D)ₘ;
wherein Ab is an anti-DLL3 antibody;
D is a cytotoxic drug 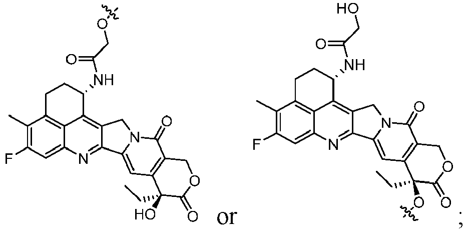
m is 2 to 8;
the structure of L₁ is as shown in formulas I, II, III, or IV, with a-terminal connected to the cytotoxic drug, and e-terminal connected to c-terminal of the L₂;

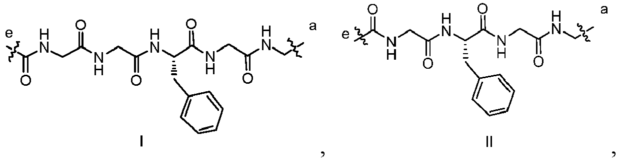

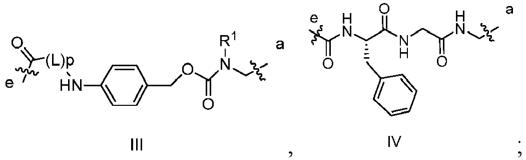

In (L)ₚ, "L" independently represents one or more of phenylalanine residue, alanine residue, glycine residue, glutamic acid residue, aspartic acid residue, cysteine residue, glutamine residue, histidine residue, isoleucine residue, leucine residue, lysine residue, methionine residue, proline residue, serine residue, threonine residue, tryptophan residue, tyrosine residue, and valine residue; "p" is from 2 to 4.

R¹ is C₁-C₆ alkyl substituted by one or moremore -NR¹⁻¹R¹⁻², C₁-C₆ alkyl substituted by one or moremore R¹⁻³S(O)₂-, C₁-C₆ alkyl, C₃-C₁₀ cycloalkyl, C₆-C₁₄ aryl, or 5- to 14-membered heteroaryl; the heteroatom in the 5- to 14-membered heteroaryl is selected from one or more of N, O, and S, and the number of heteroatoms is 1, 2, 3, or 4; the R¹⁻¹, R¹⁻², and R¹⁻³ are each independently C₁-C₆ alkyl; 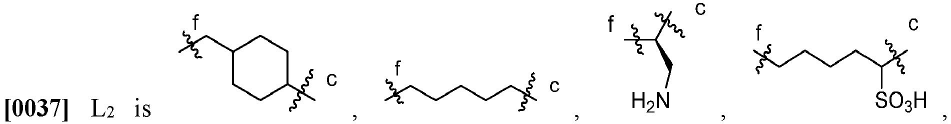 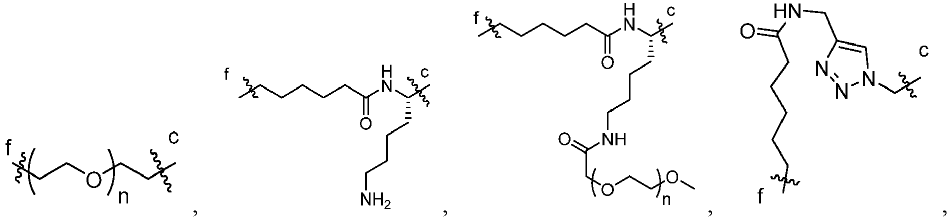 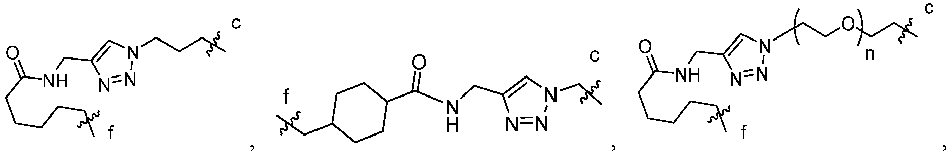 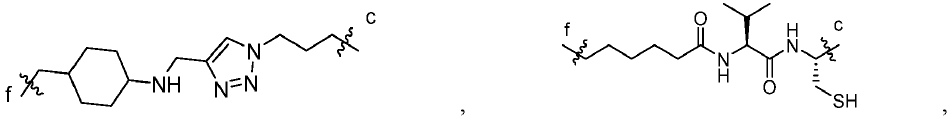 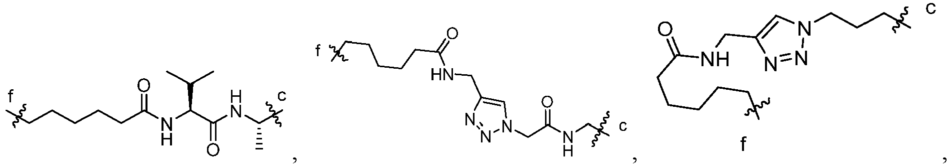 or 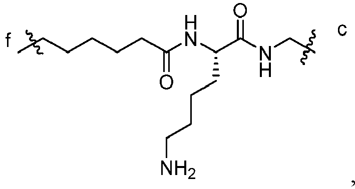 wherein n is independently 1 to 12, such as 8, 9, 10, 11, and 12, and the c-terminal is connected to L₁ through a carbonyl group, and the f-terminal is connected to the d-terminal of L₃;

L₃ is 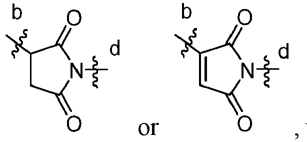 wherein the b-terminal is connected to the Ab, and the d-terminal is connected to the f-terminal of L₂.

In one preferred embodiment, the anti-DLL3 antibody binds to one of the following antigenic epitopes in the DLL3 protein: DSL domain, N-terminal, EGF2 domain, and EGF3-EGF6 domains.

In one preferred embodiment, the anti-DLL3 antibody is preferably the anti-DLL3 antibody as described above.

In one preferred embodiment, the anti-DLL3 antibody binds to the antigenic epitope of the EGF2 domain of the DLL3 protein.

In one preferred embodiment, the anti-DLL3 antibody comprises a VH with an amino acid sequence as shown in SEQ ID NO: 15 and a VL with an amino acid sequence as shown in SEQ ID NO: 16, or comprises a VH with an amino acid sequence as shown in SEQ ID NO: 25 and a VL with an amino acid sequence as shown in SEQ ID NO: 26.

In one preferred embodiment, the anti-DLL3 antibody comprises a light chain with an amino acid sequence as shown in SEQ ID NO: 1 and a heavy chain with an amino acid sequence as shown in SEQ ID NO: 2, or comprises a light chain with an amino acid sequence as shown in SEQ ID NO: 3 and a heavy chain with an amino acid sequence as shown in SEQ ID NO: 4.

In one preferred embodiment, the L is one or more of phenylalanine residue, alanine residue, glycine residue, isoleucine residue, leucine residue, proline residue, and valine residue; preferably one or more of phenylalanine residue, alanine residue, glycine residue, and valine residue.

In one preferred embodiment, the L is valine residue and/or phenylalanine residue; more herein refers to two or three; the p is 2. Preferably, the (L)p is 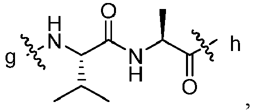 wherein the g-terminal is connected to the c-terminal of L₂ through a carbonyl group.

In one preferred embodiment, the R¹ is C₁-C₆ alkyl substituted by one or more -NR¹⁻¹R¹⁻², C₁-C₆ alkyl substituted by one or more R¹⁻³S(O)₂-, or C₁-C₆ alkyl; the R¹⁻¹, R¹⁻², and R¹⁻³ are each independently C₁-C₄ alkyl.

In one preferred embodiment, when the R¹ is C₁-C₆ alkyl substituted by one or more -NR¹⁻¹R¹⁻², the C₁-C₆ alkyl is C₁-C₄ alkyl, preferably methyl, ethyl, *n*-propyl, isopropyl, *n*-butyl, isobutyl, or tert-butyl, further preferably ethyl; more herein refers to two or three. Preferably, the R¹⁻¹ and R¹⁻² are each independently C₁-C₄ alkyl, preferably methyl, ethyl, *n*-propyl, isopropyl, *n*-butyl, isobutyl, or tert-butyl, further preferably methyl. Preferably, the -NR¹⁻¹R¹⁻² is -N(CH₃)₂. Further preferably, when the R¹ is C₁-C₆ alkyl substituted by one -NR¹⁻¹R¹⁻², the C₁-C₆ alkyl substituted by one -NR¹⁻¹R¹⁻² is

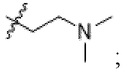

In one preferred embodiment, when the R¹ is C₁-C₆ alkyl substituted by one or more R¹⁻³S(O)₂-, the C₁-C₆ alkyl is C₁-C₄ alkyl, preferably methyl, ethyl, *n*-propyl, isopropyl, *n*-butyl, isobutyl, or tert-butyl, further preferably ethyl; more herein refers to two or three. Preferably, the R¹⁻³ is C₁-C₄ alkyl, preferably methyl, ethyl, *n*-propyl, isopropyl, *n*-butyl, isobutyl, or *tert-*butyl, further preferably methyl. Preferably, when the R¹ is C₁-C₆ alkyl substituted by one R¹⁻³S(O)₂-, the C₁-C₆ alkyl substituted by one R¹⁻³S(O)₂- is

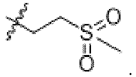

In one preferred embodiment, when the R¹ is C₁-C₆ alkyl, the C₁-C₆ alkyl is preferably C₁-C₄ alkyl, further preferably methyl, ethyl, *n*-propyl, isopropyl, *n*-butyl, isobutyl, or *tert-*butyl, further preferably methyl.

In one preferred embodiment, when the structure of L₁ is as shown in formula I, the L₂ is preferably 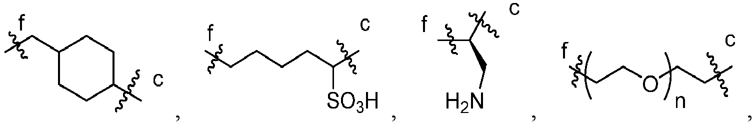 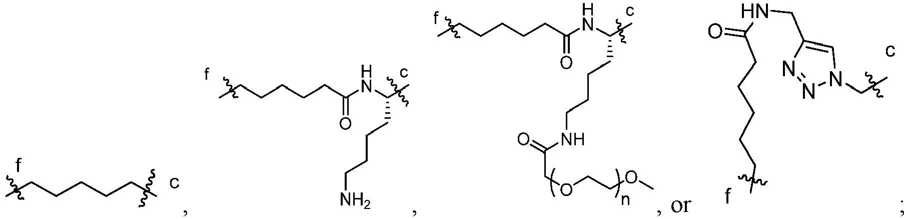 the L₃ is preferably

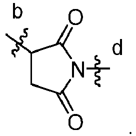

In one preferred embodiment, when the structure of L₁ is as shown in formula II, the L₂ is preferably 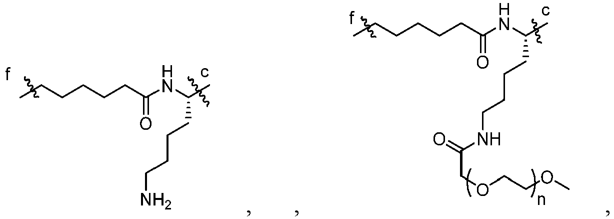 or 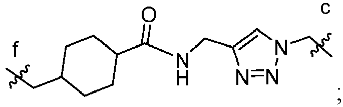 the L₃ is preferably

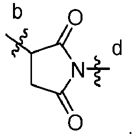

In one preferred embodiment of the present disclosure, when the structure of L₁ is as shown in formula III, the L₂ is preferably 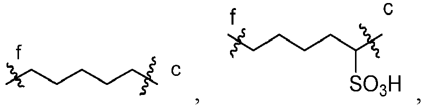 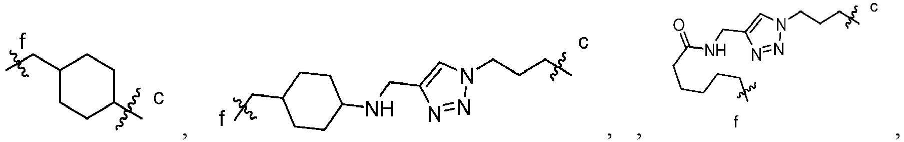 or 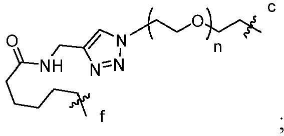 the L₃ is preferably

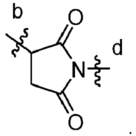

In one preferred embodiment of the present disclosure, when the structure of L₁ is as shown in formula IV, the L₂ is preferably 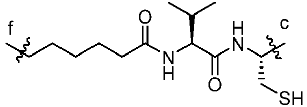 or 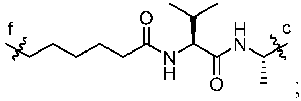 the L₃ is preferably

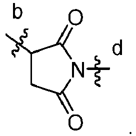

In a preferred embodiment, the structure of L₁ is preferably as shown in formula I or III.

In a preferred embodiment, the formula III is preferably 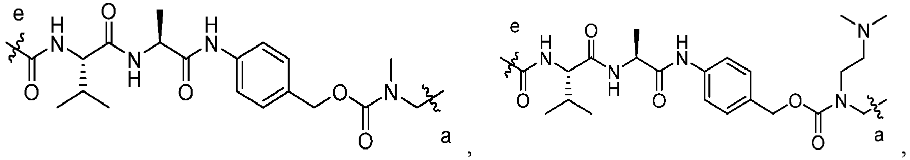 or

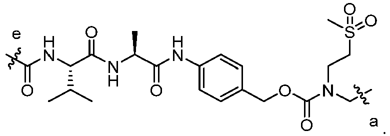

In one preferred embodiment, the formula III is further preferably

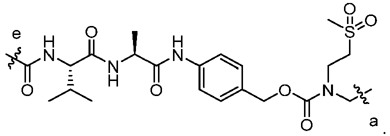

In one preferred embodiment, the b-terminal of L₃ is preferably connected to the sulfhydryl group of the antibody in the form of a thioether bond. Taking 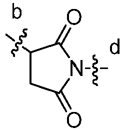 as an example, the connecting form of 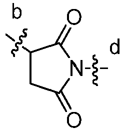 with the cysteine residue in the antibody is

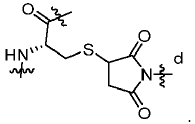

In one preferred embodiment, the m is an integer varying from 2 to 8, such as 2, 3, 4, 5, 6, 7, or 8, or a non-integer, such as 7.68, 7.53, 4.43, 7.12, 6.92, 7.43, 7.23, 6.83, 7.32, 7.56, 7.54, 7.47, 5.82, 6.78, 2.28, 6.32, 7.45, 7.65, 7.64, 7.36, 7.75, 7.80, 7.77, or 7.76.

Preferably, the antibody-drug conjugate is any one of the following compounds: 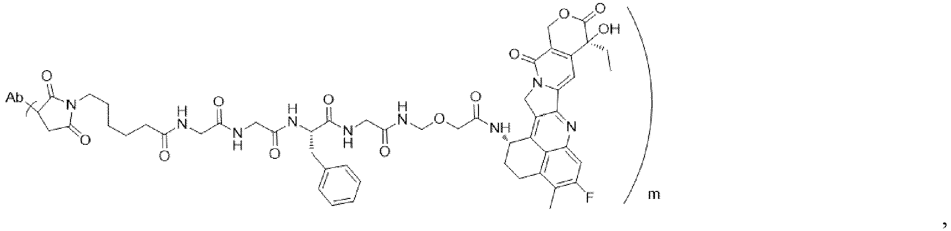 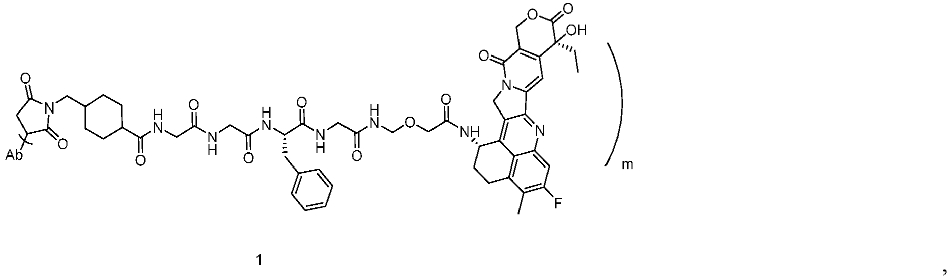 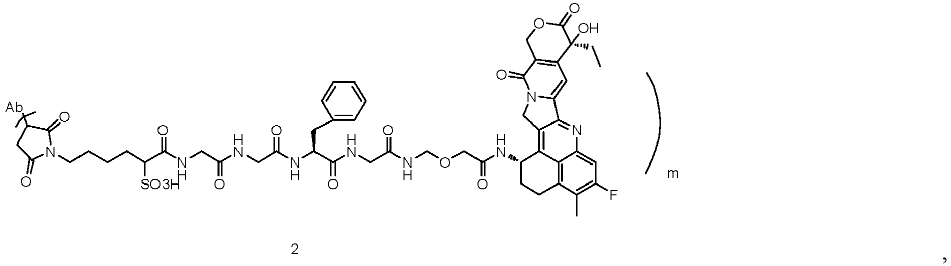 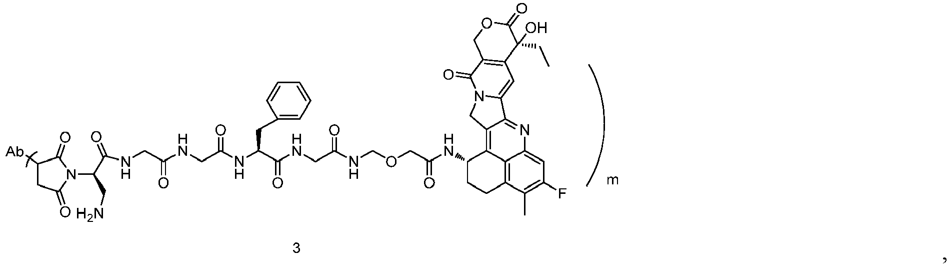 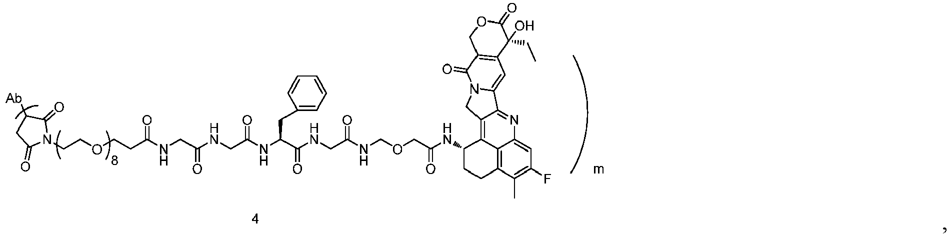 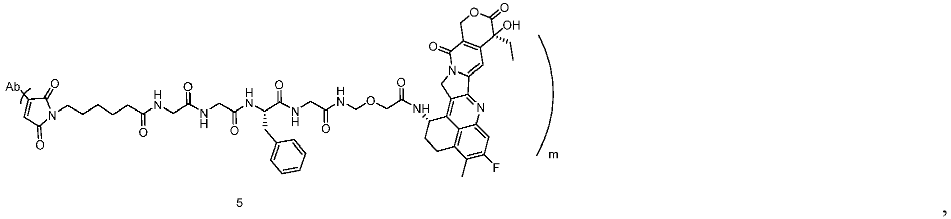 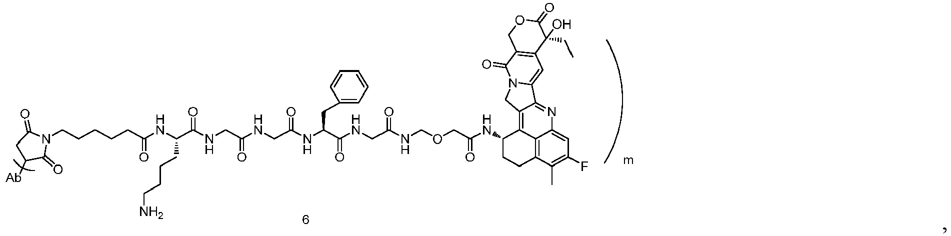 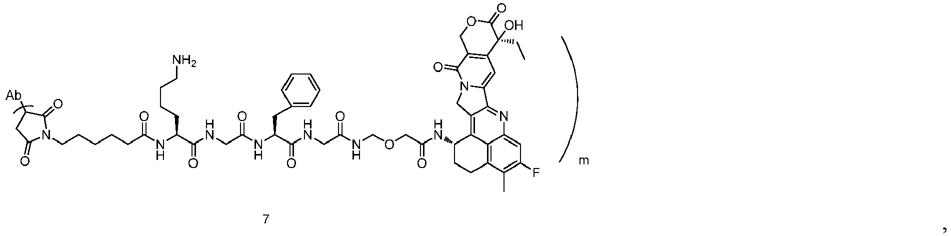 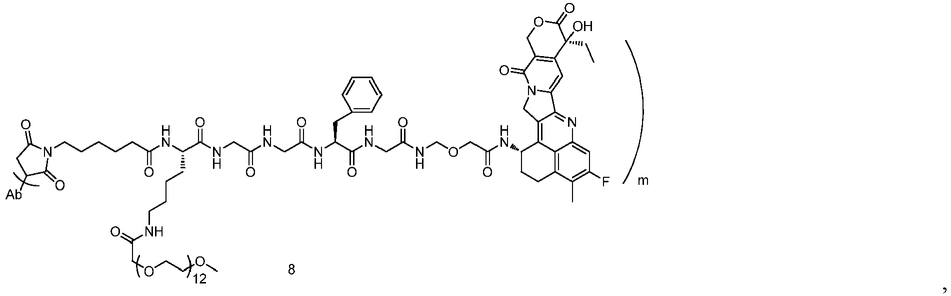 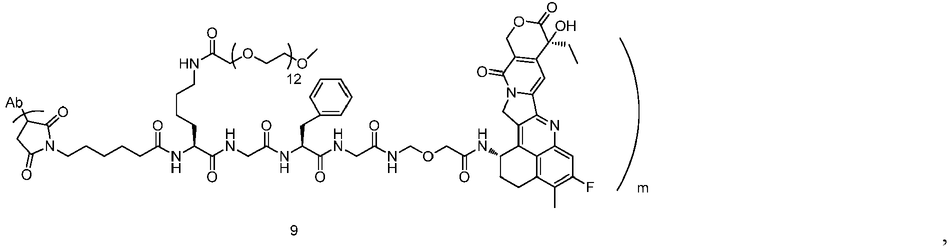 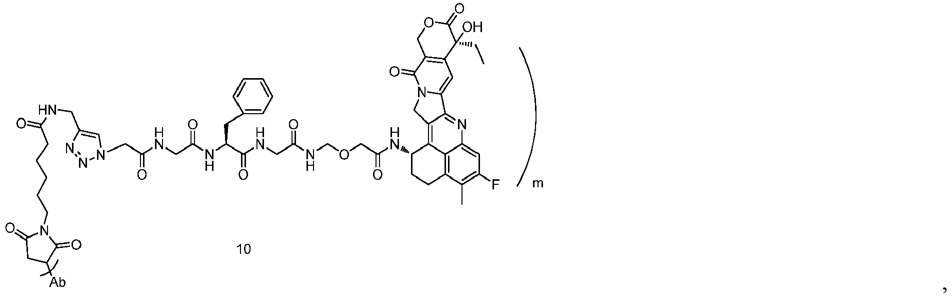 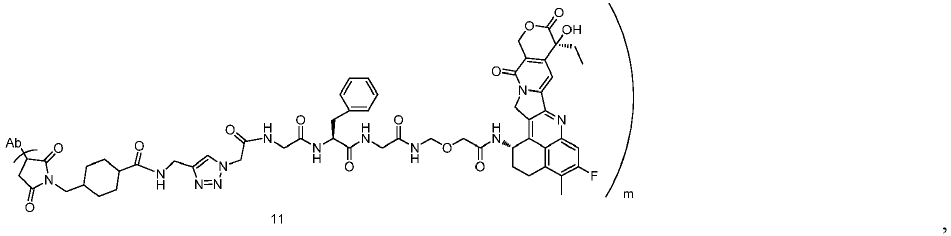 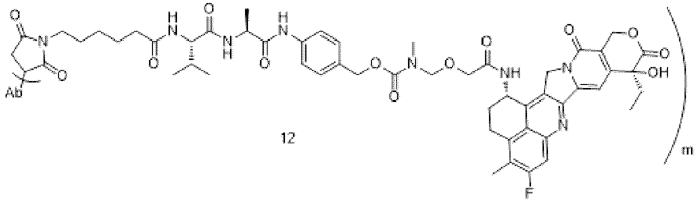 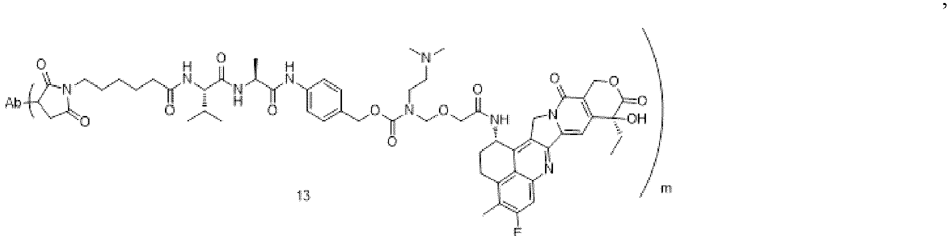 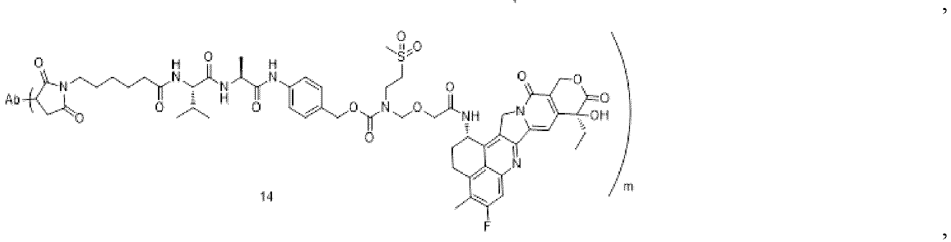 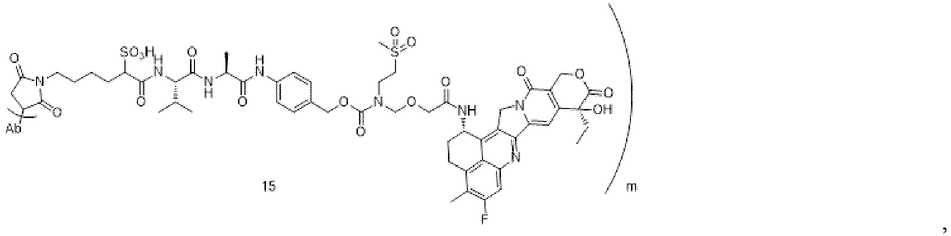 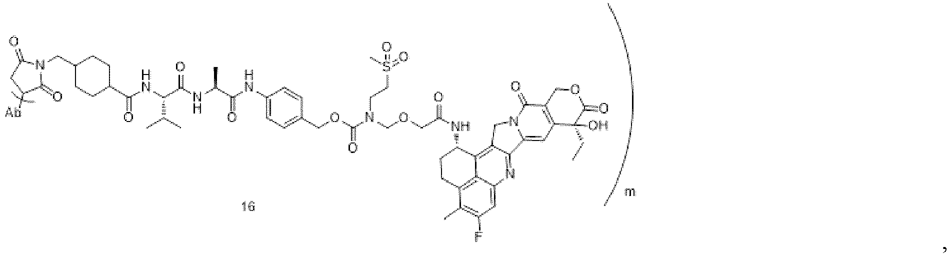 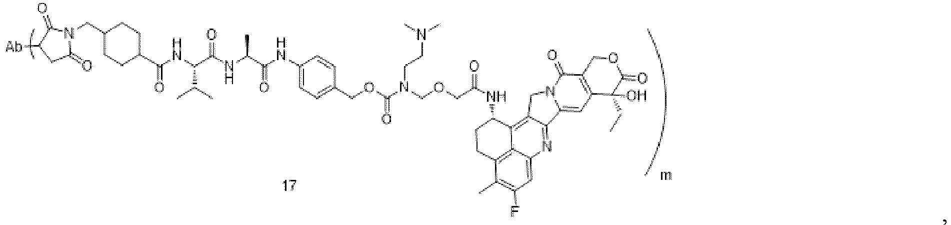 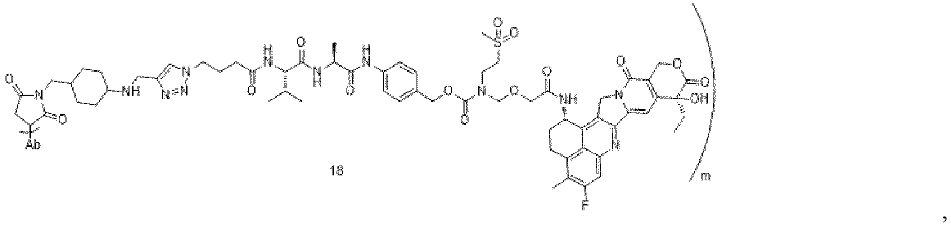 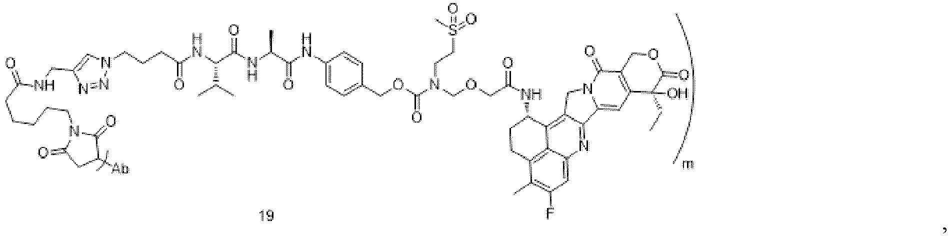 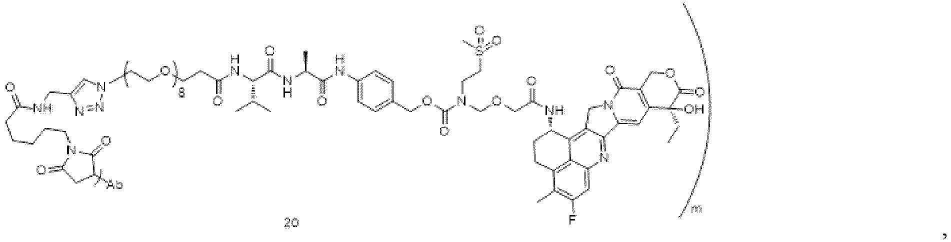 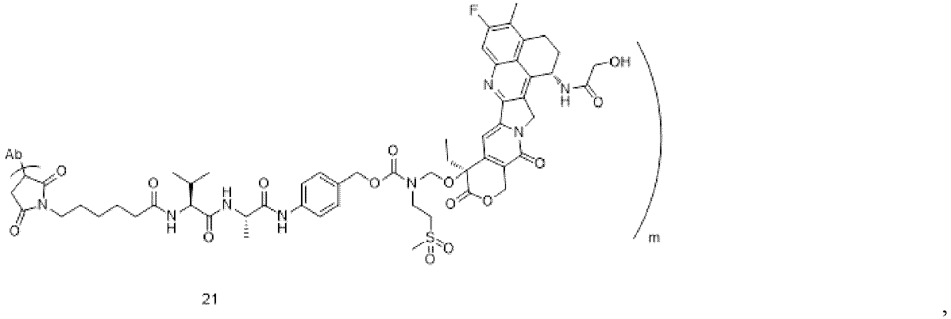 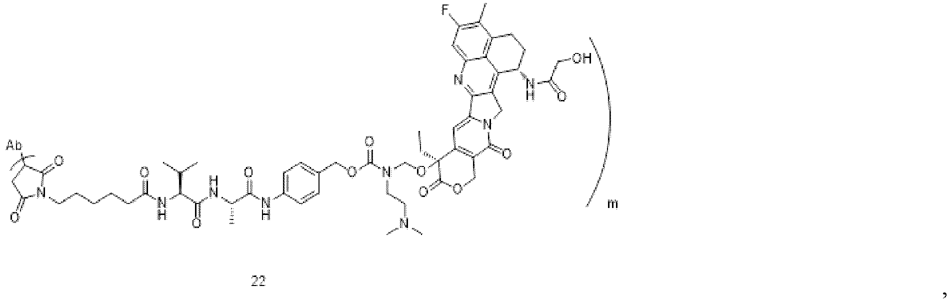 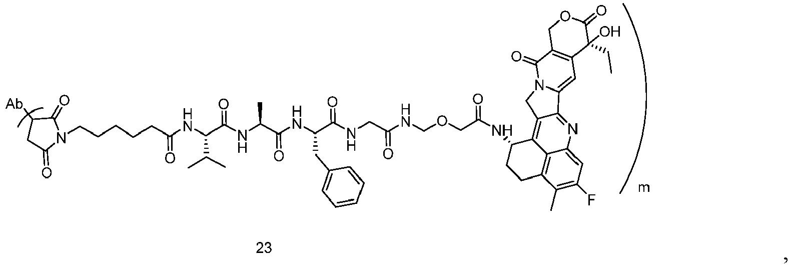 or 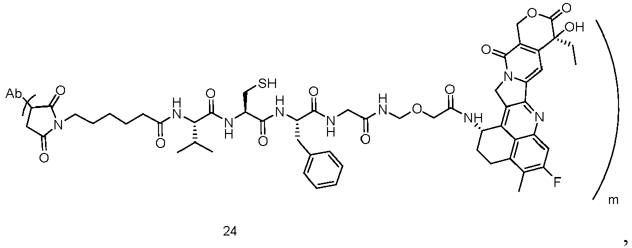 wherein Ab is the anti-DLL3 antibody as described above, and m is from 2 to 8, preferably 7.68, 7.53, 4.43, 7.12, 6.92, 7.43, 7.23, 6.83, 7.32, 7.56, 7.54, 7.47, 5.82, 6.78, 2.28, 6.32, 7.45, 7.65, 7.64, 7.36, 7.75, 7.80, 7.77, or 7.76.

In one preferred embodiment, the antibody-drug conjugate is any one of the following compounds: 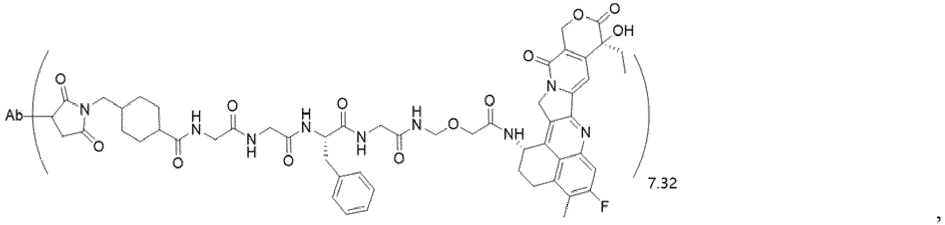 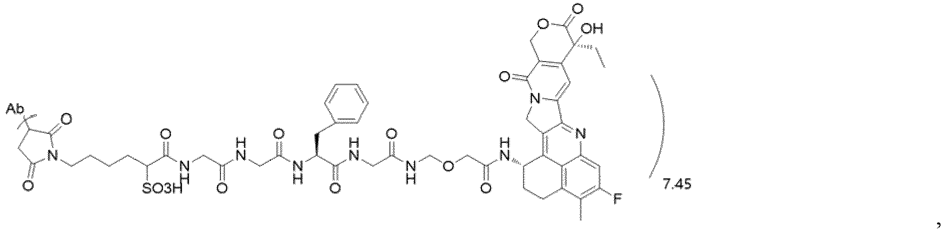 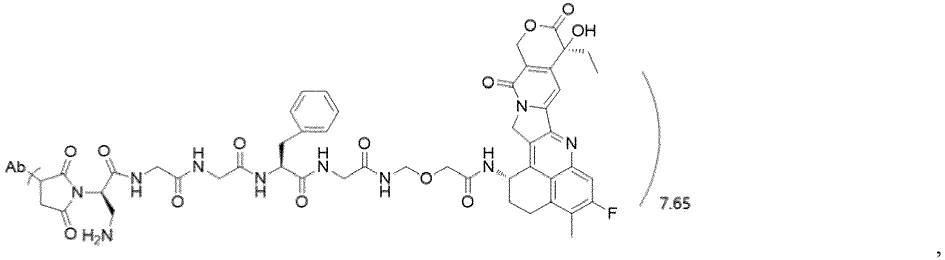 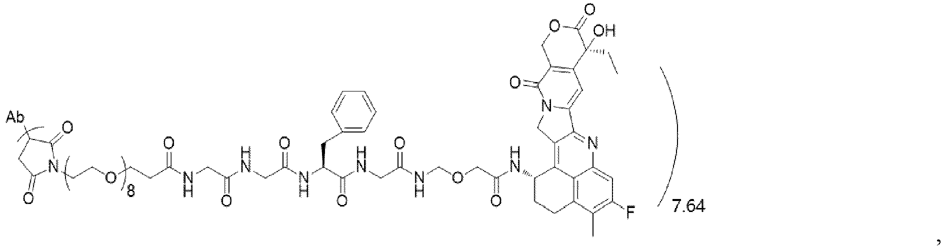 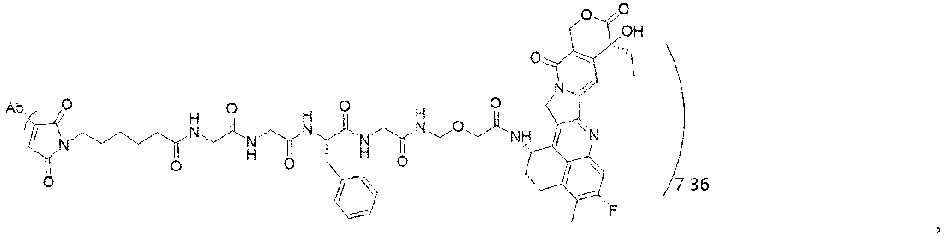 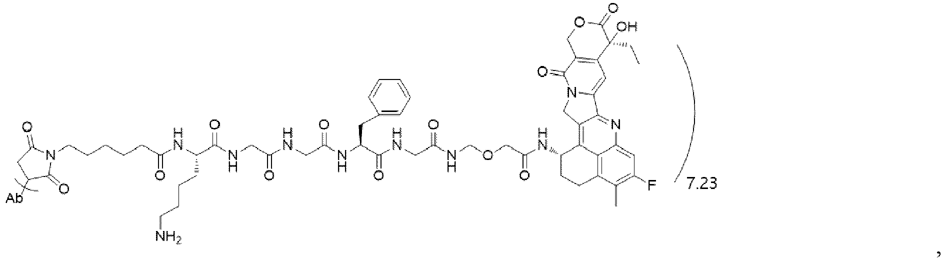 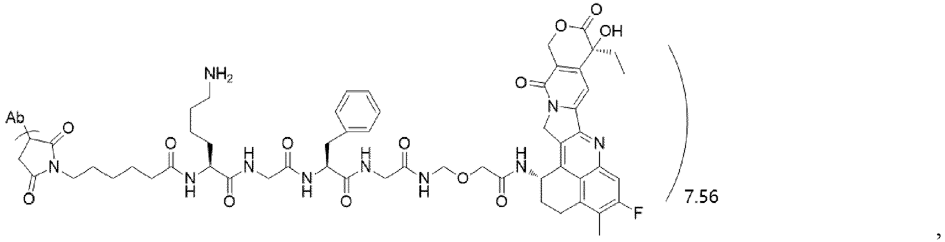 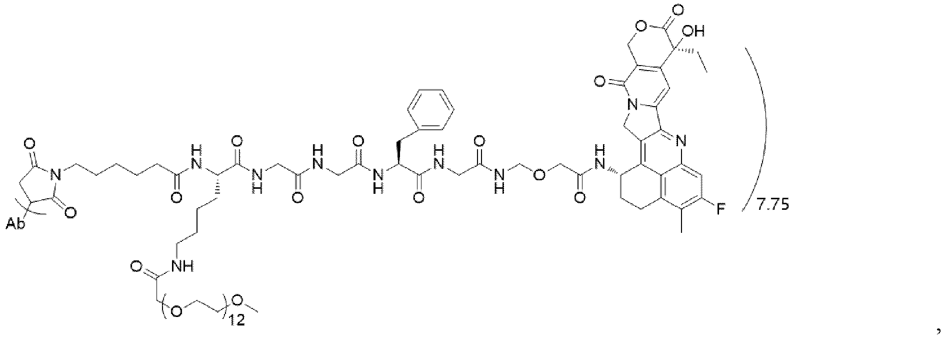 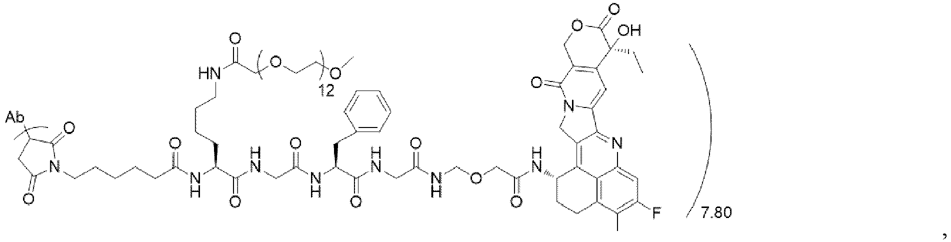 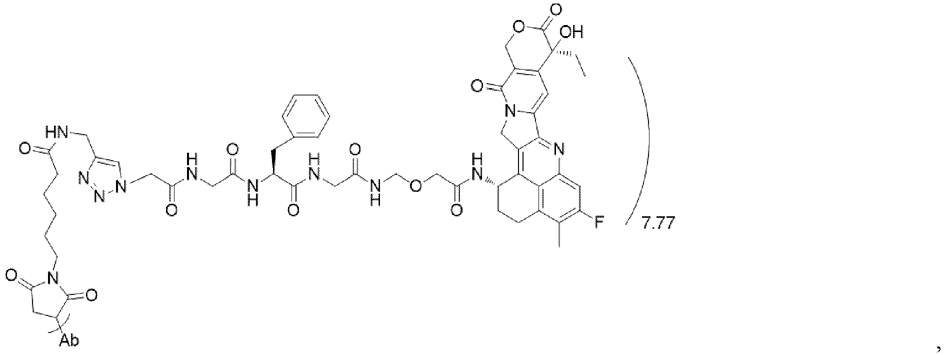 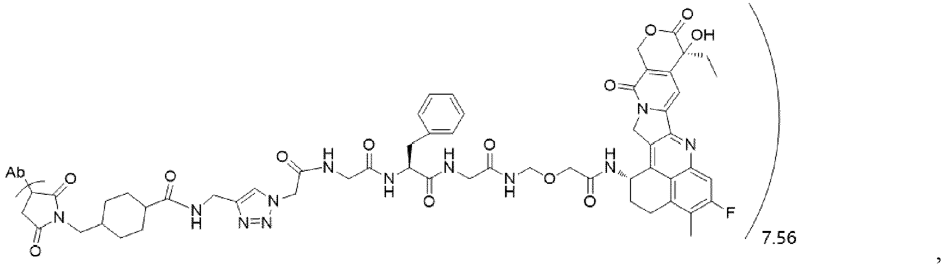 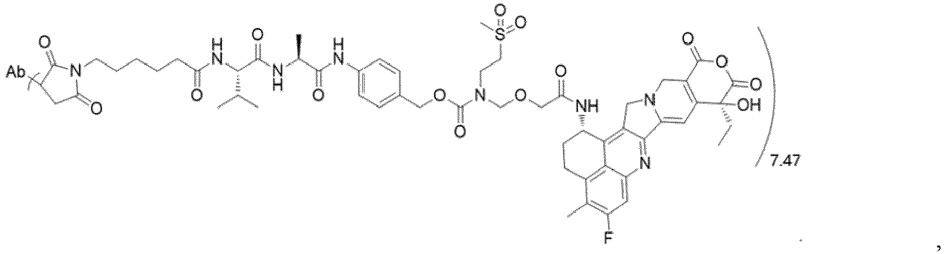 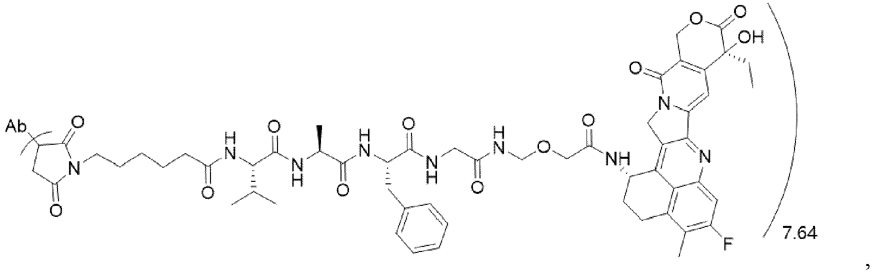 or 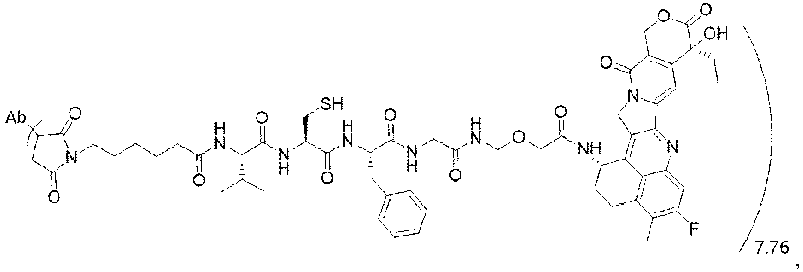 wherein Ab is the anti-DLL3 antibody comprising a VH with an amino acid sequence as shown in SEQ ID NO: 25 and a VL with an amino acid sequence as shown in SEQ ID NO: 26, preferably comprising a heavy chain with an amino acid sequence as shown in SEQ ID NO: 27 and a light chain with an amino acid sequence as shown in SEQ ID NO: 28.

In one preferred embodiment, the antibody-drug conjugate is any one of the following compounds: 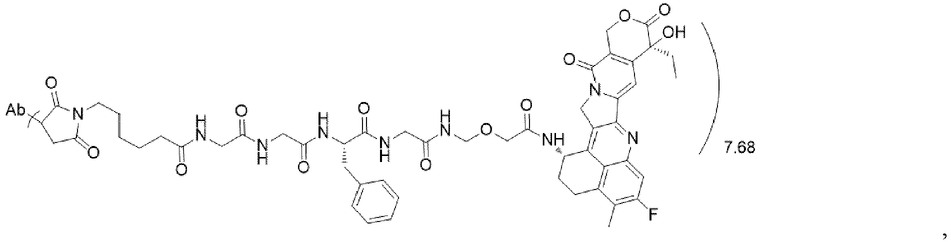 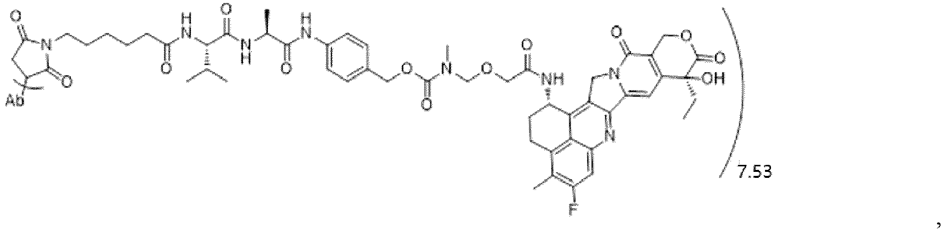 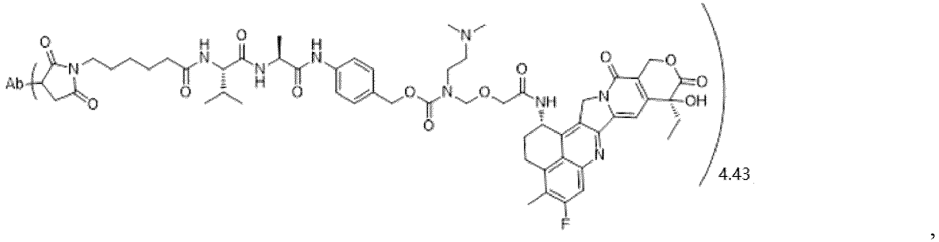 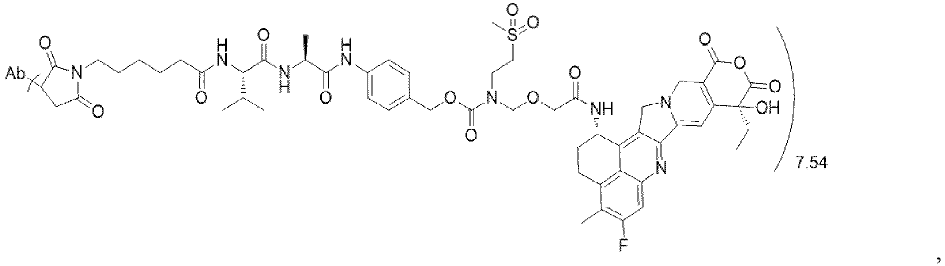 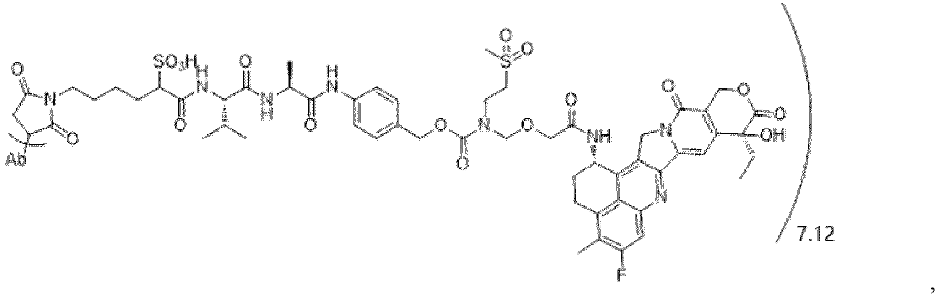 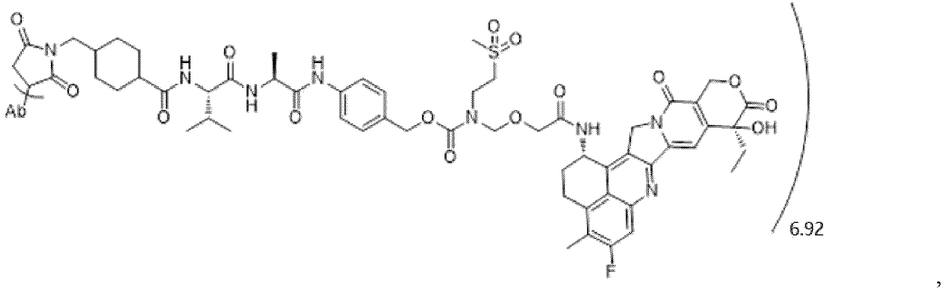 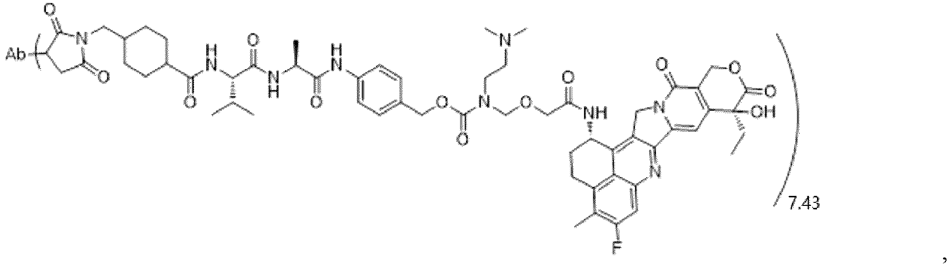 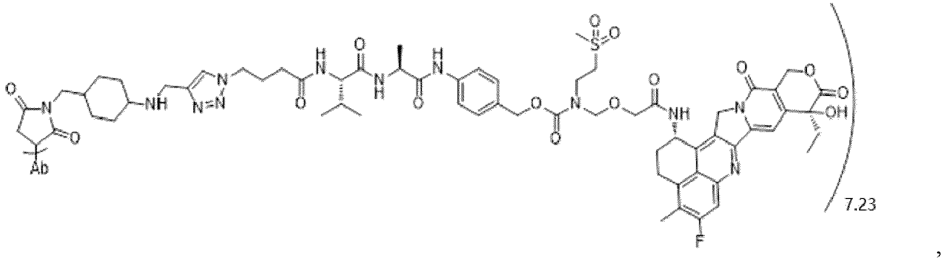 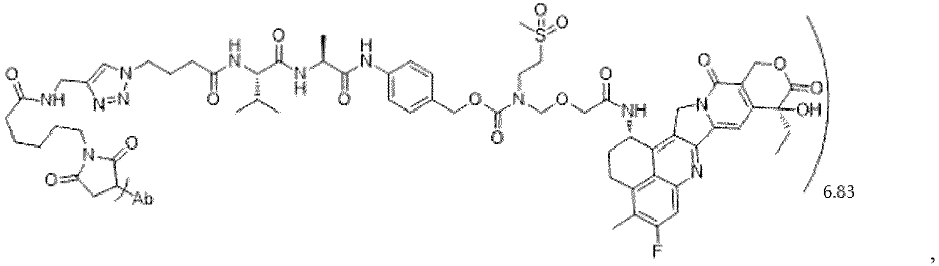 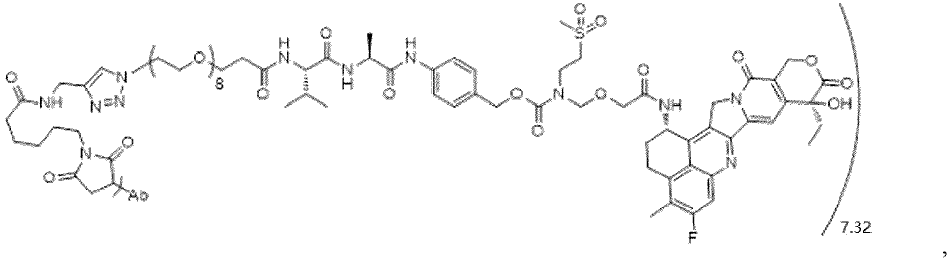 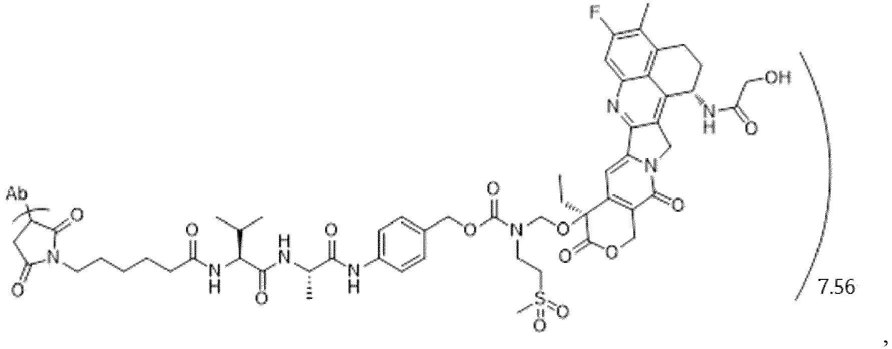 or 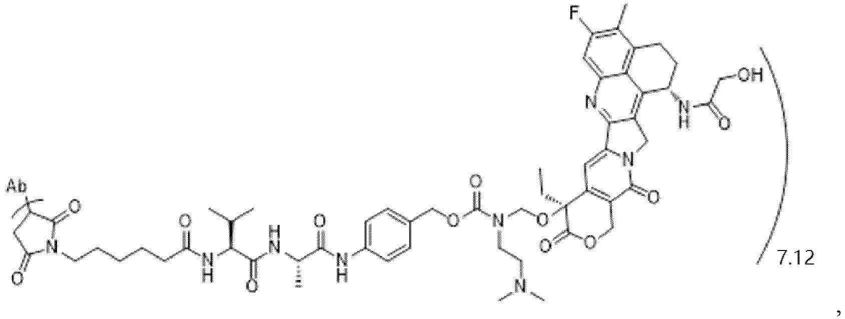 wherein Ab is the anti-DLL3 antibody comprising a VH with an amino acid sequence as shown in SEQ ID NO: 15 and a VL with an amino acid sequence as shown in SEQ ID NO: 16, preferably comprising a heavy chain with an amino acid sequence as shown in SEQ ID NO: 17 and a light chain with an amino acid sequence as shown in SEQ ID NO: 18.

In one preferred embodiment, the antibody-drug conjugate is the following compound: 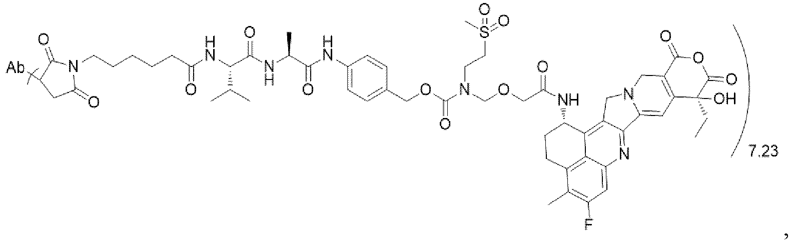 wherein Ab is the anti-DLL3 antibody comprising a heavy chain with an amino acid sequence as shown in SEQ ID NO: 2 and a light chain with an amino acid sequence as shown in SEQ ID NO: 1.

In one preferred embodiment, the antibody-drug conjugate is the following compound: 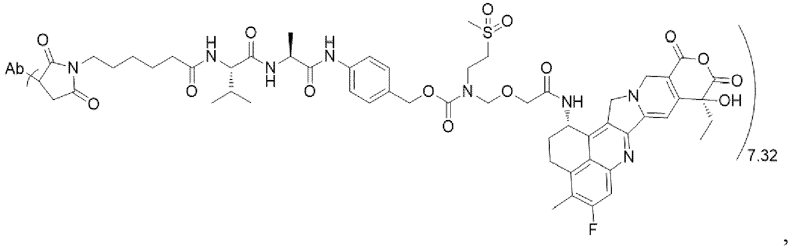 wherein Ab is the anti-DLL3 antibody comprising a heavy chain with an amino acid sequence as shown in SEQ ID NO: 4 and a light chain with an amino acid sequence as shown in SEQ ID NO: 3.

In one preferred embodiment, the antibody-drug conjugate is the following compound: 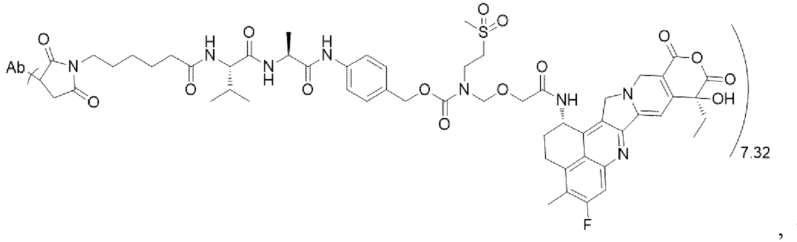 wherein Ab is the anti-DLL3 antibody comprising a VH with an amino acid sequence as shown in SEQ ID NO: 22 and a VL with an amino acid sequence as shown in SEQ ID NO: 23, preferably comprising a heavy chain with an amino acid sequence as shown in SEQ ID NO: 73 and a light chain with an amino acid sequence as shown in SEQ ID NO: 109.

In one preferred embodiment, the antibody-drug conjugate is the following compound: 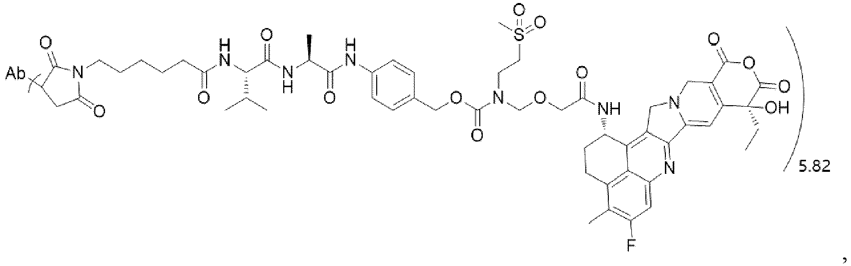 wherein Ab is the anti-DLL3 antibody comprising a VH with an amino acid sequence as shown in SEQ ID NO: 45 and a VL with an amino acid sequence as shown in SEQ ID NO: 46, preferably comprising a heavy chain with an amino acid sequence as shown in SEQ ID NO: 47 and a light chain with an amino acid sequence as shown in SEQ ID NO: 48.

Another aspect of the present disclosure further provides a chimeric antigen receptor comprising the anti-DLL3 antibody as described above.

Another aspect of the present disclosure further provides a genetically modified cell comprising the chimeric antigen receptor as described above. Preferably, the genetically modified cell is a eukaryotic cell, preferably an isolated human cell; more preferably an immune cell such as a T cell, or NK cell.

Another aspect of the present disclosure further provides a preparation method for the anti-DLL3 antibody, the preparation method comprising the following steps: cultivating the transformant as described above, and obtaining the anti-DLL3 antibody from the culture.

Another aspect of the present disclosure further provides a pharmaceutical composition comprising the anti-DLL3 antibody as described above, the antibody-drug conjugate as described above, the chimeric antigen receptor as described above, and/or the genetically modified cell as described above. Preferably, the pharmaceutical composition is in a liquid, gas, solid, or semi-solid dosage form, and/or, the pharmaceutical composition can be administered orally, by injection, nasally, transdermally, or via mucosal routes. More preferably, the pharmaceutical composition further comprises a combination therapeutic agent, and the combination therapeutic agent comprises chemotherapeutic agents, radiation therapy agents, immunosuppressants, and/or cytotoxic drugs.

Another aspect of the present disclosure further provides a use of the anti-DLL3 antibody, the antibody-drug conjugate, the chimeric antigen receptor, the genetically modified cell, and/or the pharmaceutical composition as described above in the preparation of a medicament, a kit, and/or a drug administration device for the treatment and/or prevention of diseases related to abnormal expression of DLL3 ; alternatively, a use of the anti-DLL3 antibody, the antibody-drug conjugate, the chimeric antigen receptor, the genetically modified cell, and/or the pharmaceutical composition as described above in the treatment and/or prevention of diseases related to abnormal expression of DLL3. The diseases related to abnormal expression of DLL3 preferably refer to tumors, the tumor is preferably cancer, and the cancer is preferably endocrine tumors such as neuroendocrine tumors, prostate cancer, pancreatic cancer, colorectal cancer, further preferably small-cell lung cancer.

Another aspect of the present disclosure also provides a kit comprising the anti-DLL3 antibody, the antibody-drug conjugate, the chimeric antigen receptor, the genetically modified cell, and/or the pharmaceutical composition as described above, and optionally, an instruction manual.

Another aspect of the present disclosure also provides a drug administration device comprising: (1) an infusion module for administering the aforementioned pharmaceutical composition to a subject in need thereof, and (2) an optional pharmacodynamic monitoring module.

Another aspect of the present disclosure also provides a method for detecting DLL3, the method comprising the step of using the anti-DLL3 antibody as described above for detection. Preferably, the method is for non-diagnostic and/or non-therapeutic purposes.

Another aspect of the present disclosure also provides a method for diagnosing, preventing, and/or treating diseases related to abnormal expression of DLL3 , the method comprising administering to a subject in need thereof the anti-DLL3 antibody, the antibody-drug conjugate, the genetically modified cells, and/or the pharmaceutical composition as described above. The disease related to abnormal expression of DLL3 is preferably tumor; the tumor is preferably cancer; the cancer is preferably neuroendocrine tumors, more preferably small cell lung cancer.

The present disclosure also provides an antibody group (comprising molecules made up of or comprising antibody fragments or variants), wherein members of the group correspond to one, two, three, four, five, or more different antibodies of the present disclosure [for example, full antibodies, Fab, F(ab)₂ fragments, scFv, etc.].

Another aspect of the present disclosure also provides an antibody-drug conjugate with the general structure formula Ab-(L₅-L₄-L₃-L₂-L₁-D)ₘ;
wherein Ab represents an anti-DLL3 antibody; the anti-DLL3 antibody binds to the DSL domain and/or the N-terminal of the DLL3 protein;
D is a cytotoxic drug;
m is 2 to 8;
the structure of L₁ is as shown in formulas I, II, III, or IV, with a-terminal connected to the cytotoxic drug, and e-terminal connected to c-terminal of the L₂;

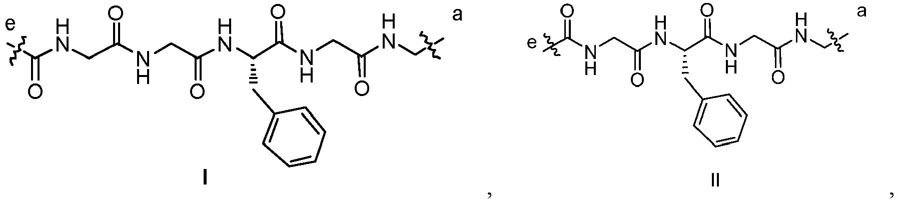

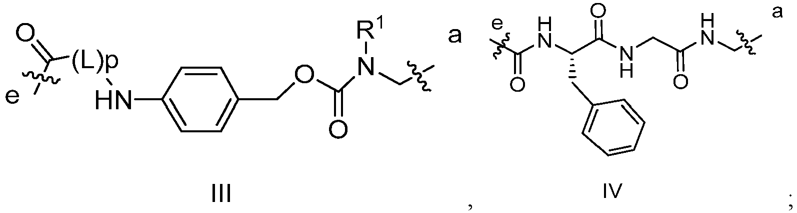

In (L)ₚ, "L" independently represents one or more of phenylalanine residue, alanine residue, glycine residue, glutamic acid residue, aspartic acid residue, cysteine residue, glutamine residue, histidine residue, isoleucine residue, leucine residue, lysine residue, methionine residue, proline residue, serine residue, threonine residue, tryptophan residue, tyrosine residue, and valine residue; "p" is from 2 to 4.

R¹ is C₁-C₆ alkyl substituted by one or more -NR¹⁻¹R¹⁻², C₁-C₆ alkyl substituted by one or more R¹⁻³S(O)₂-, C₁-C₆ alkyl, C₃-C₁₀ cycloalkyl, C₆-C₁₄ aryl, or 5- to 14-membered heteroaryl; the heteroatom in the 5- to 14-membered heteroaryl is selected from one or more of N, O, and S, and the number of heteroatoms is 1, 2, 3, or 4; the R¹⁻¹, R¹⁻², and R¹⁻³ are each independently C₁-C₆ alkyl;
L₂ is 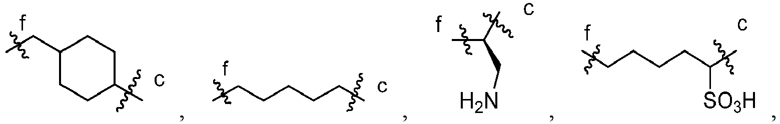 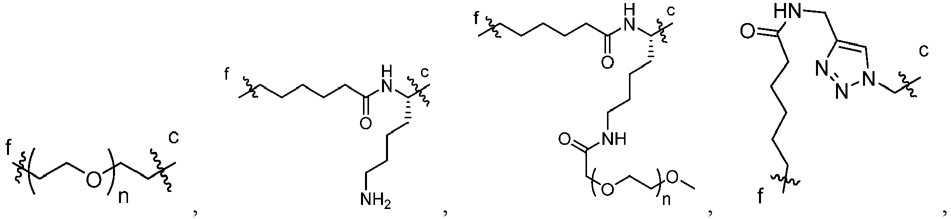 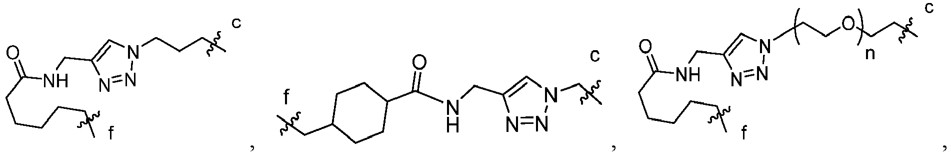 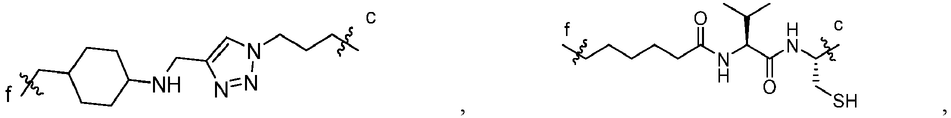 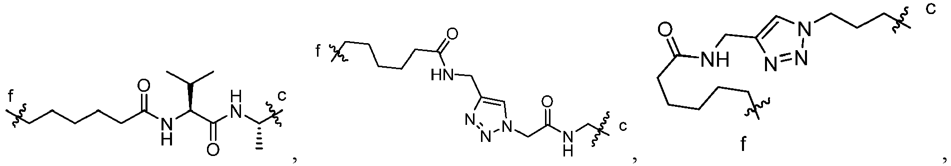 or 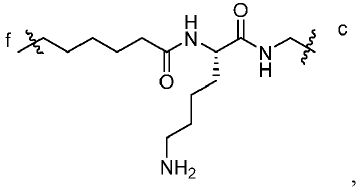 wherein n is independently 1 to 12, and c-terminal is connected to L₁ through a carbonyl group, and f-terminal is connected to d-terminal of the L₃;
L₃ is 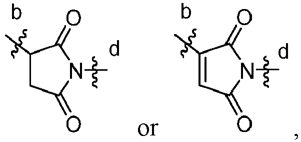 wherein b-terminal is connected to the Ab, and d-terminal is connected to f-terminal of the L₂;
The L₄ is either absent or selected from cleavable linker, non-cleavable linker, hydrophilic linker, pre-charged linker, and linker based on dicarboxylic acids;
The L₅ is either absent or is a compound represented by the following Formula V: 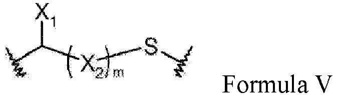
wherein Xi is selected from a hydrogen atom, halogen, hydroxyl, cyano, alkyl, alkoxy, and cycloalkyl;
X₂ is selected from alkyl, cycloalkyl, and heterocyclic groups; m is 0-5; S represents a sulfur atom.

In a preferred embodiment, D is a cytotoxic drug comprising hydroxyl, thiol, or amino groups, such as a microtubule inhibitor and/or a topoisomerase inhibitor.

In one preferred embodiment, the L is preferably one or more of phenylalanine residue, alanine residue, glycine residue, isoleucine residue, leucine residue, proline residue, and valine residue; preferably one or more of phenylalanine residue, alanine residue, glycine residue, and valine residue; further preferably, the L is the valine residue and/or the alanine residue; the "more" means two or three; p is 2.

In one preferred embodiment, the R¹ is C₁-C₆ alkyl substituted by one or more -NR¹⁻¹R¹⁻², C₁-C₆ alkyl substituted by one or more R¹⁻³S(O)₂-, or C₁-C₆ alkyl; the R¹⁻¹, R¹⁻², and R¹⁻³ are each independently C₁-C₄ alkyl.

In one preferred embodiment, when L₁ has a structure as shown in formula I, the L₂ is preferably

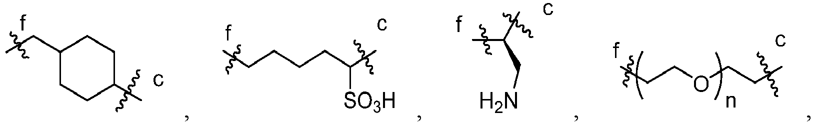

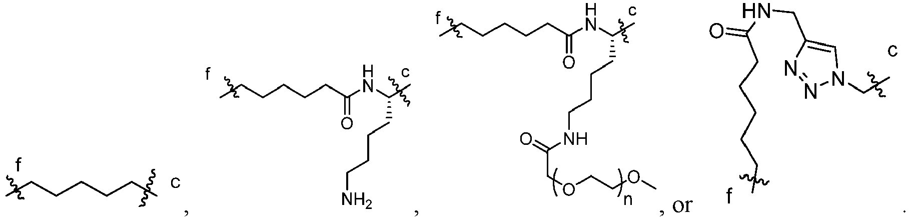

In one preferred embodiment, when L₁ has a structure as shown in formula II, the L₂ is preferably 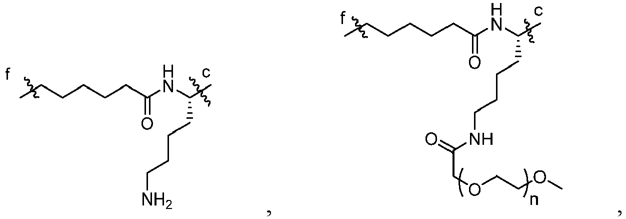 or

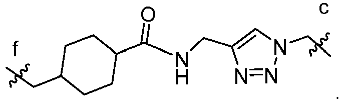

In one preferred embodiment, when L₁ has a structure as shown in formula III, the L₂ is preferably

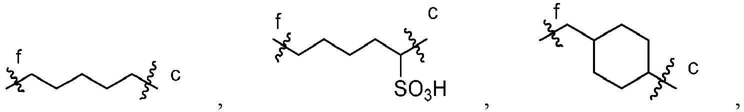

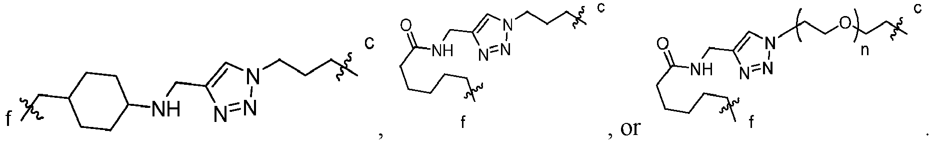

In one preferred embodiment, when L₁ has a structure as shown in formula IV, the L₂ is preferably

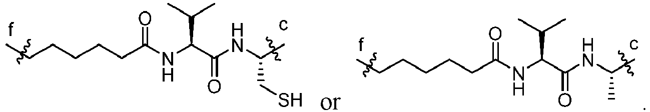

In one preferred embodiment, the n is independently 8, 9, 10, 11, and 12.

In one preferred embodiment, the m is an integer or non-integer from 2 to 8.

In one preferred embodiment, the L₃ is

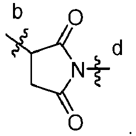

In one preferred embodiment, the L₄ is selected from N-succinimidyl 4-(2-pyridyldithio)pentanoate (SPP), N-succinimidyl 4-iodoacetylaminobenzoate (SIAB), N-succinimidyl 4-(maleimidomethyl)cyclohexane-1-carboxylate (SMCC), 6-maleimidocaproyl (MC), maleimidopropionic acid (MP), valine-citrulline (VC), alanine-phenylalanine (ala-phe), para-aminobenzylcarbamate (PAB), and MC-VC-PAB.

In one preferred embodiment, in the Ls, when Xi is a hydrogen atom, X₂ is an alkyl group, and m is 1, the compound of formula V is S-(3-carbopropyl) thioacetate.

In one preferred embodiment, D is one or more of maytansinoid derivatives, dolastatin 10 derivatives, doxorubicin analogs, or camptothecin analogs.

The maytansinoid derivatives are, for example, DM1, DM3, and DM4.

The dolastatin 10 derivatives are, for example, MMAE and MMAF.

The doxorubicin analogs are, for example, doxorubicin and camsirubicin.

More preferably, D is a camptothecin analog, further preferably a compound of formula Va or Vb: 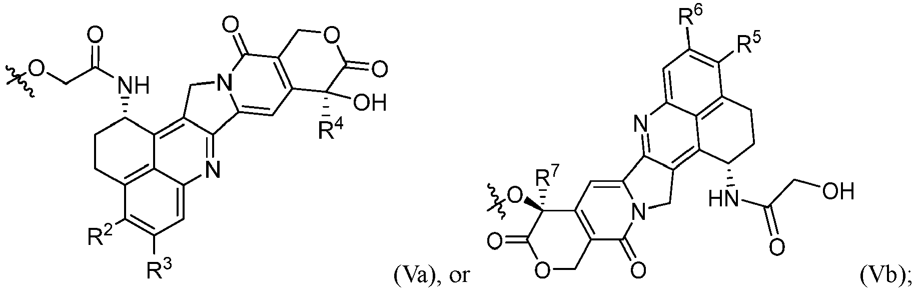
R² and R⁵ are each independently H, C₁-C₆ alkyl, or halogen;
R³ and R⁶ are each independently H, C₁-C₆ alkyl, or halogen;
R⁴ and R⁷ are each independently C₁-C₆ alkyl.

In one preferred embodiment of the present disclosure, the (L)ₚ is 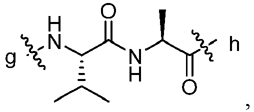 wherein g-terminal is connected to c-terminal of the L₂ through a carbonyl group.

In one preferred embodiment, the formula III is 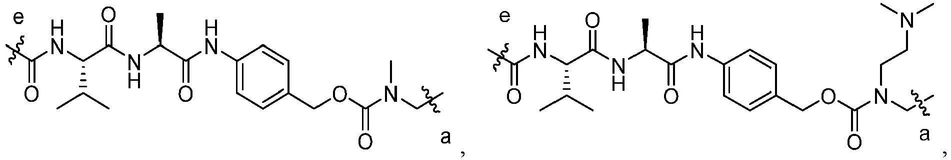 or

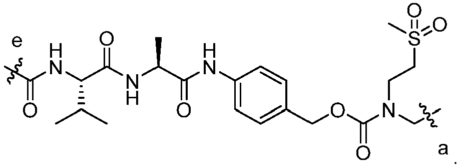

In one preferred embodiment, the antibody-drug conjugate is any one of the following compounds: 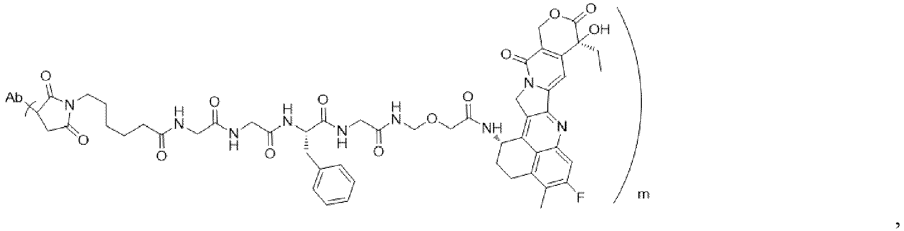 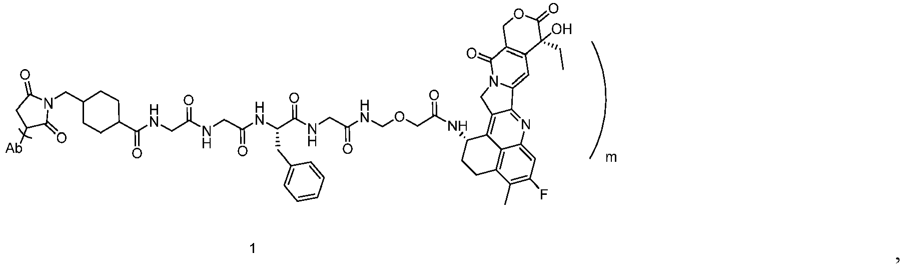 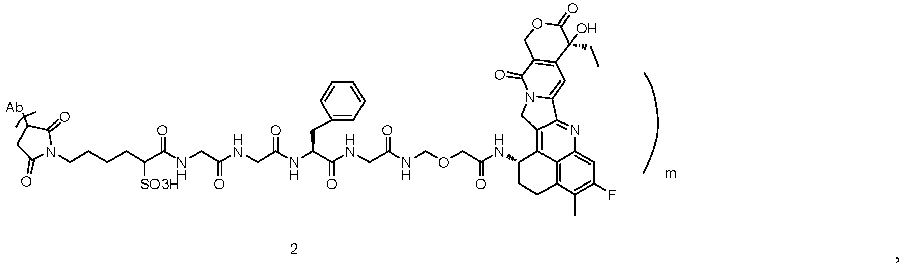 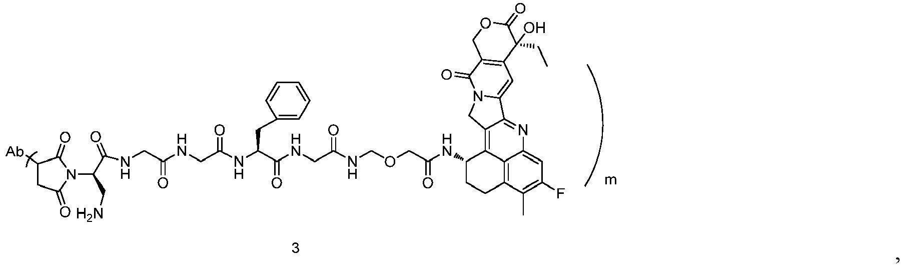 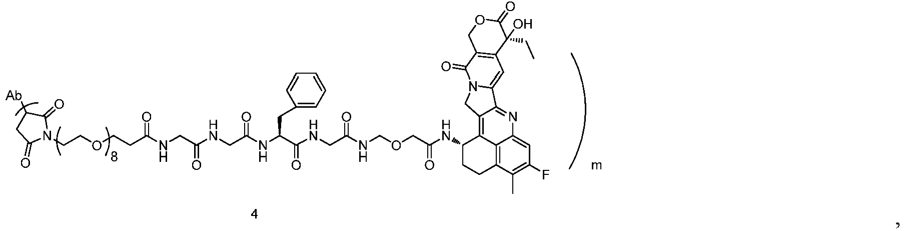 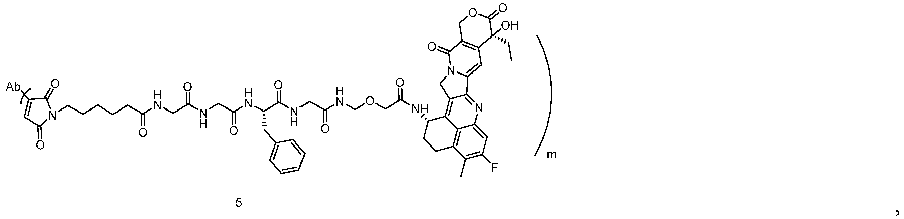 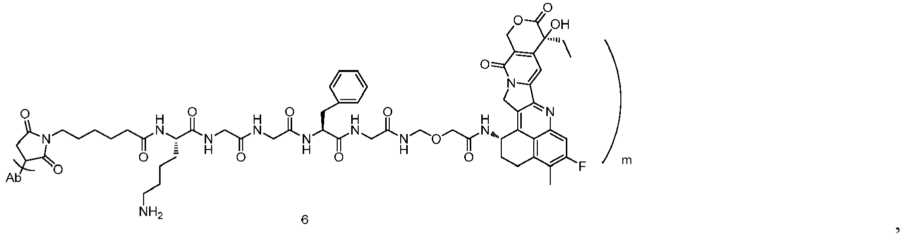 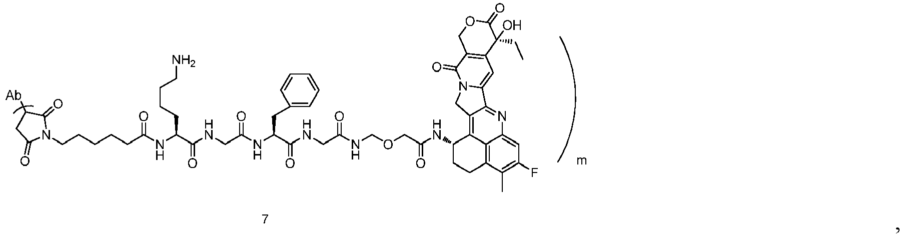 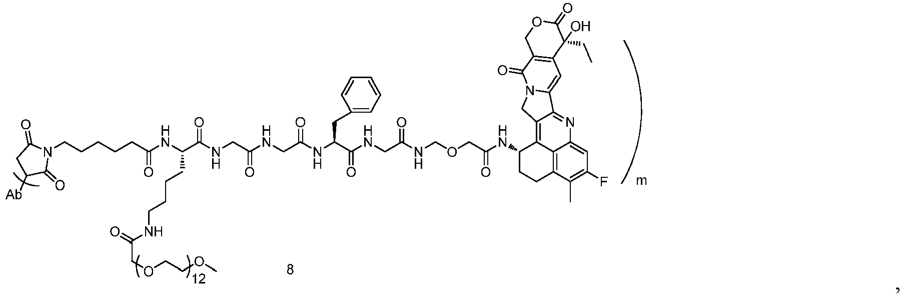 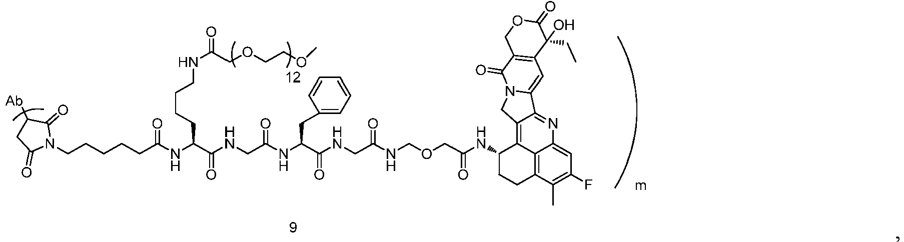 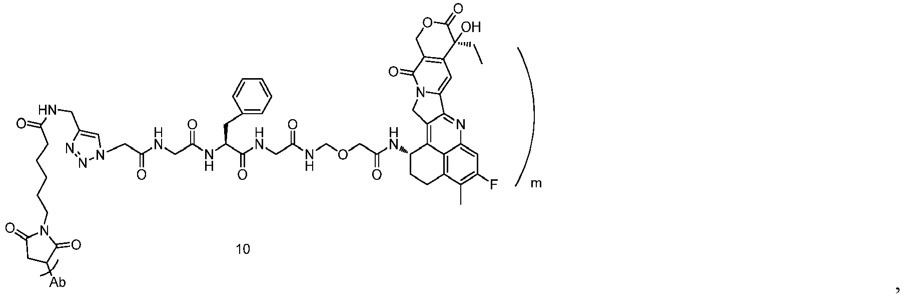 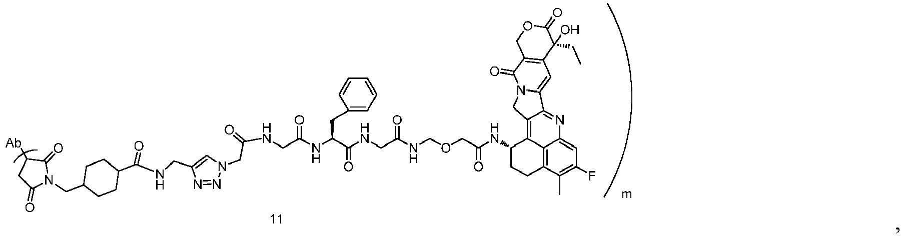 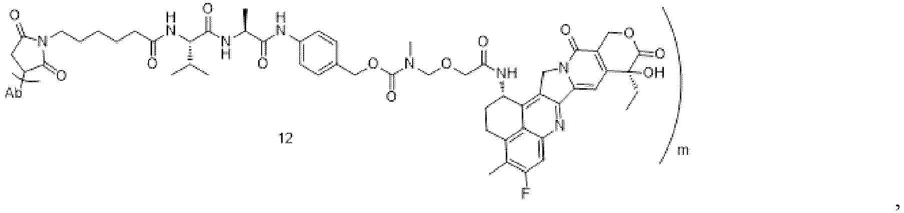 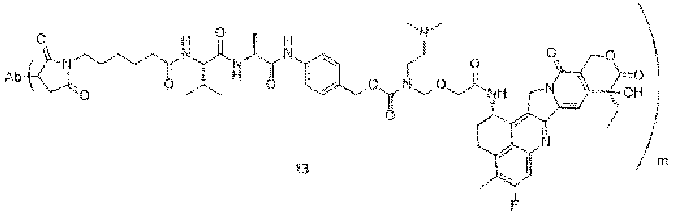 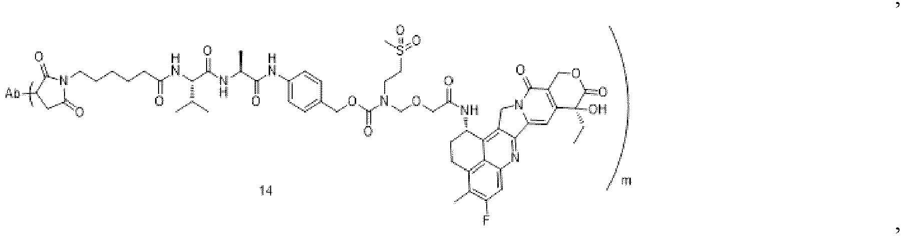 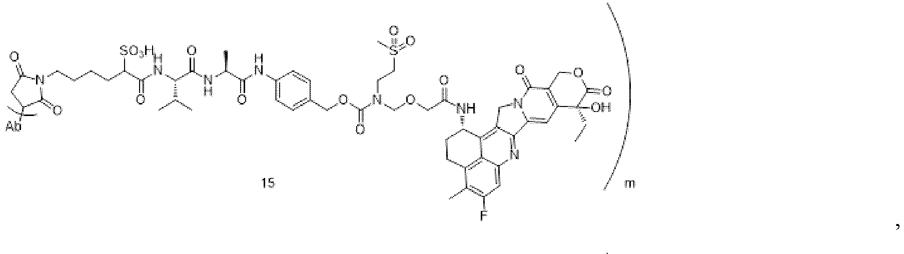 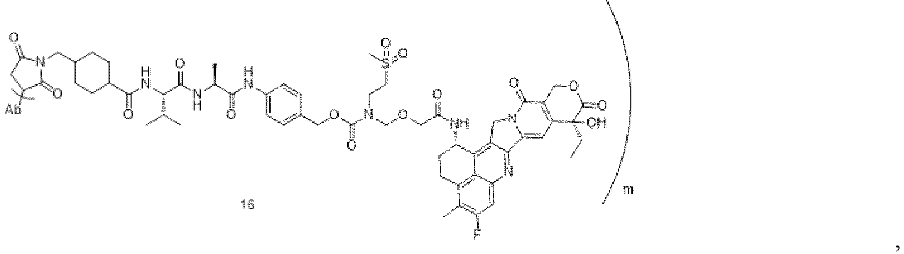 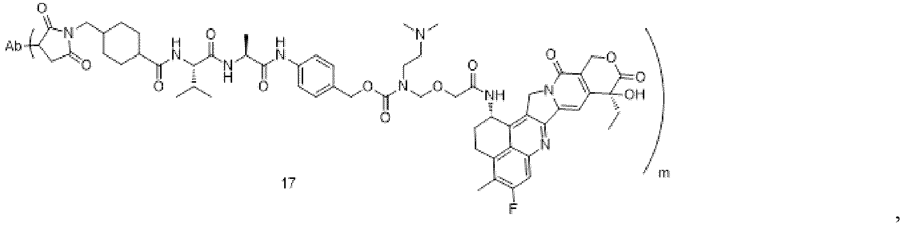 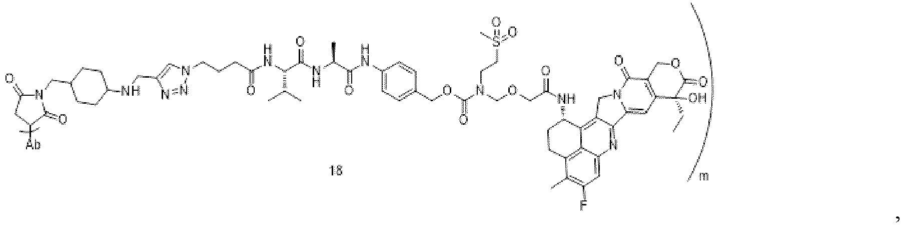 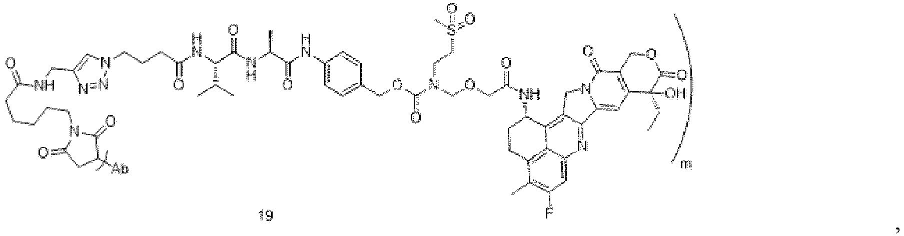 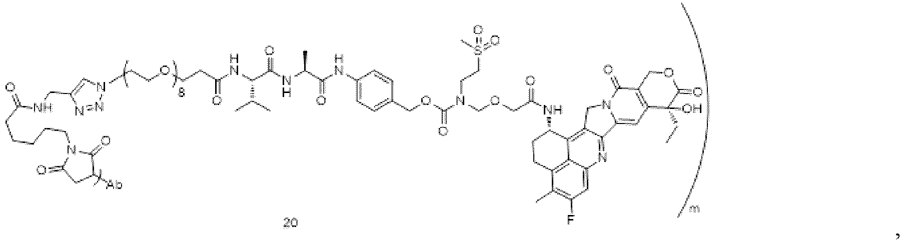 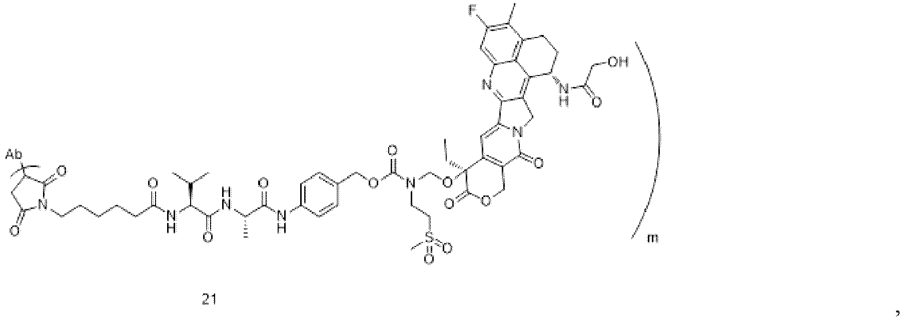 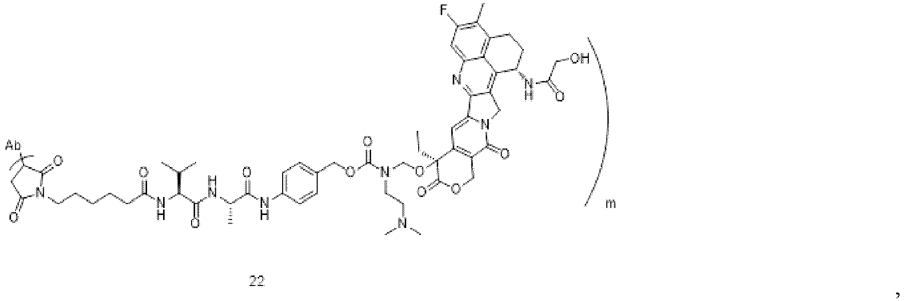 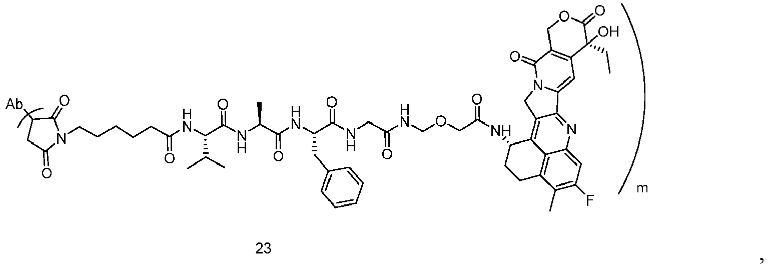 or

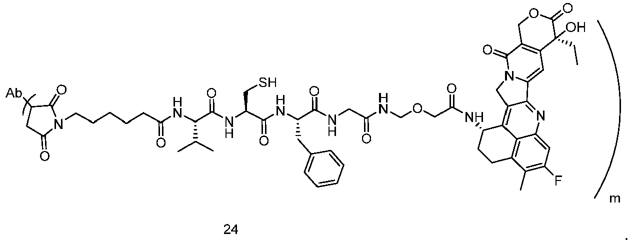

The anti-DLL3 antibody comprises a light chain with an amino acid sequence as shown in SEQ ID NO: 1 and a heavy chain with an amino acid sequence as shown in SEQ ID NO: 2, or comprises a light chain with an amino acid sequence as shown in SEQ ID NO: 3 and a heavy chain with an amino acid sequence as shown in SEQ ID NO: 4;
the m is 7.23 or 7.32.

Preferably, the antibody-drug conjugate is any one of the following compounds: 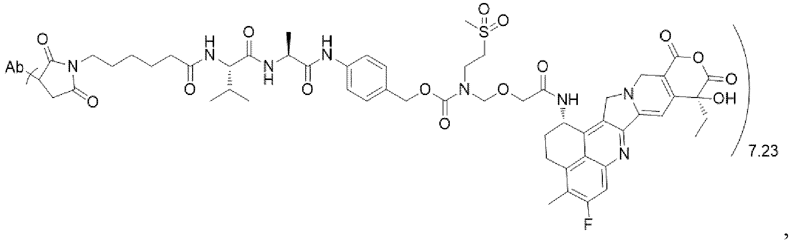 wherein Ab is the anti-DLL3 antibody comprising a heavy chain with an amino acid sequence as shown in SEQ ID NO: 2 and a light chain with an amino acid sequence as shown in SEQ ID NO: 1, or 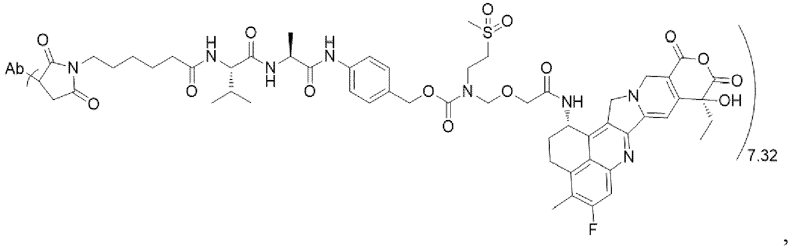 wherein Ab is the anti-DLL3 antibody comprising a heavy chain with an amino acid sequence as shown in SEQ ID NO: 4 and a light chain with an amino acid sequence as shown in SEQ ID NO: 3.

Another aspect of the present disclosure also provides a preparation method for the antibody-drug conjugate as described herein, the preparation method comprising the following steps: adding an excess of linker-drug conjugate dissolved in DMSO into a buffer solution comprising the anti-DLL3 antibody, thereby obtaining the antibody-drug conjugate.

Another aspect of the present disclosure also provides a pharmaceutical composition comprising the antibody-drug conjugate as described herein.

Preferably, the pharmaceutical composition is in a liquid, gas, solid, or semi-solid dosage form, and/or, the pharmaceutical composition can be administered orally, by injection, nasally, transdermally, or via mucosal routes.

More preferably, the pharmaceutical composition further comprises a combination therapeutic agent comprising chemotherapeutic agents, radiation therapy agents, immunosuppressants, and/or cytotoxic drugs.

Another aspect of the present disclosure also provides a use of the antibody-drug conjugate and/or the pharmaceutical composition as described herein in the preparation of a medicament, a kit, and/or a drug administration device for the treatment and/or prevention of diseases related to abnormal expression of DLL3 ;
the disease related to abnormal expression of DLL3 is preferably tumor; the tumor is preferably cancer; the cancer is preferably neuroendocrine tumors, more preferably small cell lung cancer.

Another aspect of the present disclosure also provides a kit comprising the antibody-drug conjugate and/or the pharmaceutical composition as described herein; and optionally, an instruction manual.

Another aspect of the present disclosure also provides a drug administration device, wherein the drug administration device comprises: (1) an infusion module for administering the antibody-drug conjugate and/or the pharmaceutical composition as described herein to a subject in need thereof, and (2) an optional pharmacodynamic monitoring module.

Another aspect of the present disclosure also provides a method for detecting DLL3, wherein the method for detecting DLL3 comprises the step of using the antibody-drug conjugate as described herein for detection. Preferably, the method for detecting DLL3 is for non-diagnostic and/or non-therapeutic purposes.

Another aspect of the present disclosure also provides a method for diagnosing, preventing, and/or treating diseases related to abnormal expression of DLL3 , the method comprising administering to a subject in need thereof the antibody-drug conjugate and/or the pharmaceutical composition as described herein.

In the present disclosure, the conditions and operations of the conjugating reaction can be conventional conditions and operations for the conjugating reaction in the art.

In the present disclosure, m represents the molar ratio of cytotoxic drug molecules to Ab (also known as Drug-Antibody Ratio, DAR), which can be an integer or a decimal. Preferably, it is understood as the average of molar ratio of drug molecules to monoclonal antibody molecules in the antibody-drug conjugate obtained after conjugation of the monoclonal antibody molecules with the cytotoxic drugs. The ratio can generally be determined by methods such as Hydrophobic-Interaction Chromatography (HIC), Polyacrylamide-SDS Gel Electrophoresis (SDS-PAGE), and Liquid Chromatography-Mass Spectrometry (LC-MS).

In the present disclosure, the term "C₁-C₆ alkyl" by itself or in combination with others refers to saturated straight-chain or branched alkyl groups comprising 1 to 6 carbon atoms, particularly 1 to 4 carbon atoms, such as methyl, ethyl, *n*-propyl, isopropyl, *n*-butyl, isobutyl, or tert-butyl. The term "C₁-C₆ alkyl" is preferably methyl or ethyl.

The antibodies of the present disclosure can be prepared using techniques well known in the art, such as hybridoma methods, recombinant DNA techniques, phage display techniques, synthetic techniques, or combinations thereof, or other techniques known in the art.

In the present disclosure, the terms "selectivity" or "specificity" refer to the fact that the disclosed antagonists do not show significant binding to substances other than DLL3, except in those special cases where additional antagonists are incorporated to confer an additional specificity, different from the DLL3-specific binding part (e.g., bispecific or bifunctional molecules, where the molecule is designed to bind or exert two functions, at least one of which involves specific binding to DLL3).

The term "antibody molecule" or "antibody" as described herein refers to immunoglobulin molecules and immunologically active parts of immunoglobulin molecules, that is, molecules comprising antigen-binding sites with immunospecific binding to antigens. Therefore, the term antibody encompasses not only complete antibody molecules but also fragments of the antibodies as well as variants of the antibodies and the antibody fragments (including derivatives). The term antibody molecule as described herein includes, but is not limited to, single-chain Fv (scFv), Fab fragments, Fab' fragments, F(ab')2, disulfide-linked Fv (sdFv), Fv, as well as complete or full-length antibodies. The term "single-chain Fv" or "scFv" refers to a peptide consisting of an antibody's VL (Variable Light) domain linked to the VH (Variable Heavy) domain of the antibody. Antibodies that immunospecifically bind to DLL3 may cross-react with other antigens. Preferably, antibodies that immunospecifically bind to DLL3 do not cross-react with other antigens. Antibodies that immunospecifically bind to DLL3 can be identified, for example, through immunoassays or other methods known to those skilled in the art. A "complete antibody" or "full-length antibody" refers to a protein consisting of two heavy chains (H) and two light chains (L) connected by disulfide bonds. The protein comprises: (1) for the heavy chains, a heavy chain variable region (abbreviated as "VH") and a heavy chain constant region comprising three domains CH₁, CH₂, and CH₃; and (2) for the light chains, a light chain variable region (abbreviated as "VL") and a light chain constant region comprising one domain, CL. The antibodies of the present disclosure include, but are not limited to, monoclonal, multispecific, human or chimeric antibodies, single-chain antibodies, Fab fragments, F(ab') fragments, anti-idiotypic (anti-Id) antibodies (including, for example, anti-Id antibodies against the antibodies of the present disclosure), and epitope-binding fragments of any of the aforementioned antibodies. The immunoglobulin molecules of the present disclosure can be of any type of immunoglobulin (e.g., IgG, IgE, IgM, IgD, IgA, and IgY), class (e.g., IgG1, IgG2, IgG3, IgG4, IgA1, and IgA2), or subclass. In the present disclosure, the antibody can be either a monoclonal antibody or a polyclonal antibody, with the monoclonal antibody preferably being a murine anti-human monoclonal antibody. The antibodies of the present disclosure can be ultra-humanized antibodies or double antibodies.

In the present disclosure, the term "heavy chain antibody" refers to an antibody that comprises only one variable domain of the heavy chain (VHH) and two conventional domains CH2 and CH3, also known as HCAbs.

A "single domain antibody," also known as a "nanobody," refers to the VHH structure cloned from heavy chain antibodies, representing the smallest known unit capable of binding to a target antigen.

In the present disclosure, an amino acid sequence described as having "more than 90%, 95%, 98%, or 99% identity" is obtained by insertion, deletion, or substitution of the amino acid sequences shown in the previous sequence list. The substitution can include, for example, making computer structural simulation analysis of the sequence, analyzing the potential existing post-translational modification (PTM) sites, especially in the Complementarity-Determining Regions (CDRs), including analyzing and substituting the sites of antibody aggregation, susceptible to asparagine deamidation (at sites like NG, NS, NH, etc.), susceptible to aspartic acid isomerization (at DG, DP sites), susceptible to N-glycosylation (at N-{P}S/T sites), and susceptible to oxidation.

"KD" refers to the dissociation constant derived from the ratio of Kd (the dissociation rate of specific binding molecule-target protein interaction) to Ka (the association rate of specific binding molecule-target protein interaction), or Kd/Ka, expressed in molar concentration (M). The KD value can be determined using well-established methods in the art. A preferred method for determining the KD of a binding molecule is through the use of surface plasmon resonance, such as biosensor systems like the Biacore^{™} system (GE Healthcare Life Sciences).

The term "treatment" or its equivalent expression, when used for, for example, cancer, refers to a procedure or process for reducing or eliminating the number of cancer cells in a patient or alleviating the symptoms of cancer. The "treatment" of cancer or another proliferative disorder does not necessarily mean that the cancer cells or other disorders will actually be eliminated, the number of cells or disorders will actually be reduced or the symptoms of cancers or other disorders will actually be alleviated. Generally, the method of treating cancer will be carried out even if it has only a low probability of success, but it is still considered to induce an overall beneficial course of action considering the patient's medical history and estimated survival expectation.

The term "pharmaceutically acceptable carrier" refers to any preparation or carrier medium capable of delivering an effective amount of the active substance of the present disclosure, without interfering with the biological activity of the active substance and causing no toxic side effects to the host or patient. Representative carriers include water, oil, vegetable and mineral, cream base, detergent base, ointment base, and the like. These bases may include suspending agents, thickeners, transdermal enhancers, etc. Their preparations are well known to those skilled in the arts of cosmetics or topical pharmaceutical.

The above preferred conditions can be combined arbitrarily to obtain preferred embodiments of the present disclosure without violating common knowledge in the art.

The reagents and raw materials used in the present disclosure are all commercially available.

The present disclosure's advantageous progressive effects include: the anti-DLL3 antibodies of the present disclosure possess excellent internalization activity, demonstrate superior binding activity with human DLL3 protein, and exhibit strong affinity at the protein level. The antibody-drug conjugates of the present disclosure exhibit excellent drug-forming properties, biological activity, and *in vivo* and *in vitro* anti-tumor activity, enabling the application of cytotoxic drugs in the treatment of patients with tumors characterized by neuroendocrine features, including SCLC.

### BRIEF DESCRIPTION OF THE DRAWINGS

Figure 1 presents the map of pV81 vector.
Figure 2 shows the internalization results of the antibody in Example 4.
Figure 3 shows the dose-response curve of the chimeric antibody's binding activity with hDLL3.
Figure 4 shows the dose-response curves of the chimeric antibody's binding activity with hDLL1 (left side) and hDLL4 (right side).
Figure 5 shows the dose-response curves of the chimeric antibody's binding activity with mouse (left side) and monkey DLL3 (right side).
Figure 6 shows the cytotoxic activity dose-response graph of different ADC candidates treating DLL3 target cells for 6 days.
Figure 7 shows the *in vivo* anti-tumor efficacy of ADC drugs in the NCI-H82 model.

### DETAILED DESCRIPTION OF THE PREFERRED EMBODIMENT

**Table A. Glossary of Abbreviations**

| | |
|---|---|
| DMEM | Dulbecco's Modified Eagle Medium (basic culture medium) |
| HAT | Hypoxanthine-Aminopterin-Thymidine (selective culture medium) |
| PEI | Polyetherimide |
| MOI | Multiplicity of Infection |
| GFP | Green Fluorescent Protein |
| SPF | Specific Pathogen Free (animals) |
| PBST | Phosphate-Buffered Saline with Tween |
| HRP | Horseradish Peroxidase |
| TMB | 3,3',5,5'-Tetramethylbenzidine |
| PBS | Phosphate-Buffered Saline |
| APC | Allophycocyanin |

The present disclosure will be further described below with reference to examples, but the present disclosure is not therefore limited to the scope of the examples. Experimental methods without specific conditions in the following examples are selected according to conventional methods and conditions, or according to the commercial specification.

### Example 1. Preparation of Monoclonal Antibodies FDA027 and FDA031 Specifically Targeting hDLL3

In the present disclosure, monoclonal antibodies FDA027 and FDA031, which exhibit high affinity and specificity towards hDLL3, were selected. FDA027 comprises a light chain with an amino acid sequence as shown in SEQ ID NO: 1 and a heavy chain with an amino acid sequence as shown in SEQ ID NO: 2. FDA031 comprises a light chain with an amino acid sequence as shown in SEQ ID NO: 3 and a heavy chain with an amino acid sequence as shown in SEQ ID NO: 4.

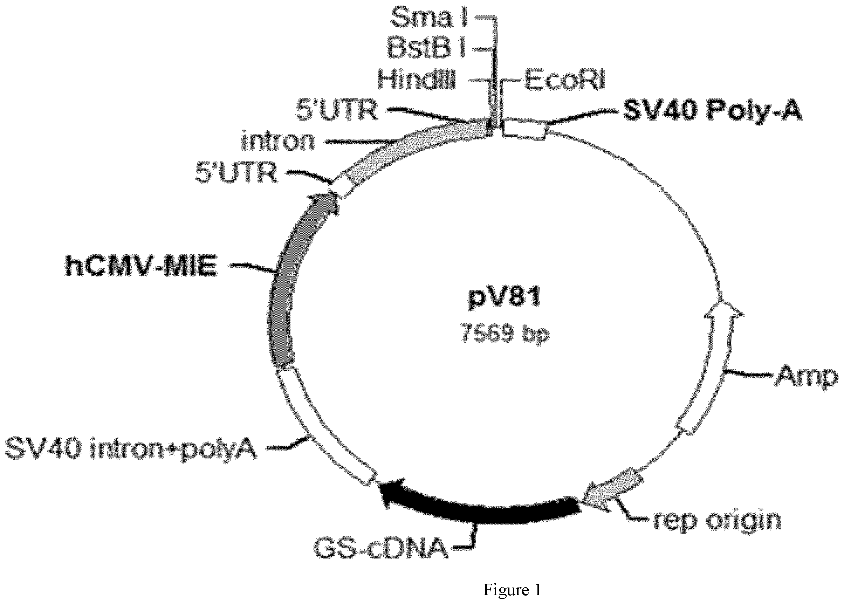

The nucleotide sequences for the light and heavy chains of FDA027 were obtained through whole gene synthesis by Suzhou Genewiz. The sequences were individually cloned into the pV81 vector (as shown in Figure 1) using EcoR I (purchased from NEB, R3104S) and Hind III (purchased from NEB, R3101S) double digestion. These were then transformed into Trans 1-T1 competent cells (purchased from TransGen Biotech, CD501), and clones were selected for sequencing verification. Positive clones were cultured and expanded for plasmid mid-prep extraction, obtaining the eukaryotic expression plasmids for the light chain, FDA027-L/pV81, and the heavy chain, FDA027-H/pV81. The plasmids were mixed at a mass ratio of 1.5:1 and electroporated into suspension-adapted CHO cells (purchased from ATCC). The electroporated cells were inoculated into 96-well plates at 2000-5000 cells/well and cultured for 3 weeks. The expression level was then determined using HTRF (Homogeneous Time-Resolved Fluorescence) according to the instructions of the kit (purchased from Cisbio, 62HFCPEG). The cell pool with the highest expression was expanded into a 125 ml shake flask (with a culture volume of 30 ml) at 37°C, 5.0% CO₂, shaking at 130 rpm. After 3 days, the culture was expanded to a 1000 ml shake flask (with a culture volume of 300 ml) at 37°C, 5.0% CO₂, shaking at 130 rpm. From the fourth day, feeding medium equivalent to 5-8% of the starting culture volume was added every other day. The culture was continued for 10-12 days. The harvest was centrifuged at 9500 r/min for 15 minutes to remove cell debris and the supernatant was collected and filtered through a 0.22 µm filter. The processed sample was purified using a MabSelect affinity column (purchased from GE Healthcare), ultimately yielding the high-purity anti-DLL3 antibody FDA027. FDA031 was prepared using the same operational method as described for FDA027.

### Example 2. Preparation of a Human hDLL3 Overexpression Vector and Stable Transfected Cell Line

A human DLL3 expression plasmid, pCMV3-DLL3-t1 (purchased from Sino Biological Inc., HG20010-UT), was used to transform *Escherichia coli* competent cells, Trans1-T1 (purchased from TransGen Biotech, CD501), for the amplification of the transfection expression plasmid. The transformation process followed the instructions provided with the competent cells. Monoclones were picked from the transformation plates and expanded overnight in culture medium. The culture was then centrifuged at 6000 r/min for 20 minutes, and the bacterial solution was collected for plasmid extraction. A plasmid mid-prep extraction kit (purchased from Macherey-Nagel, product number: DP117) was used, according to the instruction manual to extract plasmid DNA.

Well-growing logarithmic phase HEK293 cells (purchased from the Cell Bank of the Chinese Academy of Sciences) were selected. Fresh culture medium, DMEM with 10% fetal bovine serum, was used to dilute the cells to a density of 5×10⁵ cells/ml. 2 ml of the cell suspension was inoculated per well in a 6-well culture plate and cultured in an incubator (37°C, 5% CO₂). The following day, the transfection reagent Lipofectamine 3000 (purchased from Thermo, L3000-008) was used to transfect the pCMV3-DLL3-t1 plasmid into HEK293 cells, following the instructions provided with the Lipofectamine 3000 transfection kit. 48 hours post-transfection, the cells were inoculated in 96-well culture plates at a density of 1 cell per well and cultured in fresh medium comprising 200 µg/ml G418 (purchased from Thermo, 10687010). The medium, comprising G418, was refreshed every 3 to 4 days to obtain stable monoclonal cells expressing hDLL3. Flow cytometry was used to screen HEK293 cells expressing hDLL3. The detection antibody, goat anti-hDLL3-PE (purchased from R&D, FAB4315P), was diluted with PBS to 10 µg/ml and 100 µl was added to 5×10⁵ cells to be detected, followed by incubation at 4°C for 1 hour. The cells were then resuspended in 1 ml of PBS comprising 2% fetal bovine serum, centrifuged at 1500 r/min for 5 minutes, and the supernatant was discarded, and the operation was repeated twice. The cells were resuspended in 1 ml of PBS and analyzed using a CytoFLEX flow cytometer (purchased from Beckman). Clones with a human DLL3 expression positivity rate greater than 85% were considered positive, and the HEK293-DLL3 stable cell line were established successfully.

HEK293 overexpression cell lines containing different hDLL3 domains were stably transfected using the same method as above, which including cell lines overexpressing hDLL3 without the N-terminal domain (sequence as shown in SEQ ID NO: 5), hDLL3 without the N-terminal and DSL domains (sequence as shown in SEQ ID NO: 6), hDLL3 without the N-terminal, DSL, and EGF1 domains (sequence as shown in SEQ ID NO: 7), and hDLL3 without the N-terminal, DSL, EGF1, and EGF2 domains (sequence as shown in SEQ ID NO: 8).
SEQ ID NO: 5 
GKPIPNPLLGLDSTSGARCEPPAVGTACTRLCRPRSAPSRCGPGLRPCAPLEDECEAPL
VCRAGCSPEHGFCEQPGECRCLEGWTGPLCTVPVSTSSCLSPRGPSSATTGCLVPGPG
PCDGNPCANGGSCSETPRSFECTCPRGFYGLRCEVSGVTCADGPCFNGGLCVGGADP
DSAYICHCPPGFQGSNCEKRVDRCSLQPCRNGGLCLDLGHALRCRCRAGFAGPRCEH
DLDDCAGRACANGGTCVEGGGAHRCSCALGFGGRDCRERADPCAARPCAHGGRC
YAHFSGLVCACAPGYMGARCEFPVHPDGASALPAAPPGLRPGDPQRYLSGGGGSGAG
VIAVIVVVVIAIVAGIVVLVISRKKRMAKYEKAEIKEMGEMHRELNA
SEQ ID NO: 6 
GKPIPNPLLGLDSTSGAPLVCRAGCSPEHGFCEQPGECRCLEGWTGPLCTVPVSTSSC
LSPRGPSSATTGCLVPGPGPCDGNPCANGGSCSETPRSFECTCPRGFYGLRCEVSGVT
 
CADGPCFNGGLCVGGADPDSAYICHCPPGFQGSNCEKRVDRCSLQPCRNGGLCLDL
GHALRCRCRAGFAGPRCEHDLDDCAGRACANGGTCVEGGGAHRCSCALGFGGRDC
RERADPCAARPCAHGGRCYAHFSGLVCACAPGYMGARCEFPVHPDGASALPAAPPG
LRPGDPQRYLSGGGGSGAGVIAVIVVVVIAIVAGIVVLVISRKKRMAKYEKAEIKEMGEMH
RELNA
SEQ ID NO: 7 
GKPIPNPLLGLDSTSGGPGPCDGNPCANGGSCSETPRSFECTCPRGFYGLRCEVSGVT
CADGPCFNGGLCVGGADPDSAYICHCPPGFQGSNCEKRVDRCSLQPCRNGGLCLDL
GHALRCRCRAGFAGPRCEHDLDDCAGRACANGGTCVEGGGAHRCSCALGFGGRDC
RERADPCAARPCAHGGRCYAHFSGLVCACAPGYMGARCEFPVHPDGASALPAAPPG
LRPGDPQRYLSGGGGSGAGVIAVIVVVVIAIVAGIVVLVISRKKRMAKYEKAEIKEMGEMH
RELNA
SEQ ID NO: 8

GKPIPNPLLGLDSTSGSGVTCADGPCFNGGLCVGGADPDSAYICHCPPGFQGSNCEKR
VDRCSLQPCRNGGLCLDLGHALRCRCRAGFAGPRCEHDLDDCAGRACANGGTCVE
GGGAHRCSCALGFGGRDCRERADPCAARPCAHGGRCYAHFSGLVCACAPGYMGAR
CEFPVHPDGASALPAAPPGLRPGDPQRYLSGGGGSGAGVIAVIVVVVIAIVAGIVVLVISRK
KRMAKYEKAEIKEMGEMHRELNA

Note: the underlined is the V5 label, and the italicized part is the intracellular sequence

### Example 3. Preparation of Hybridoma Monoclonal Antibodies and Antibody Screening

### 3-1. Immunization and Serum Titer Testing

Six 6-week-old SPF grade female Balb/C mice (purchased from Shanghai Jihui Laboratory Animal Care Co.,Ltd.) were selected and randomly divided into two groups, A and B. They were immunized with recombinant DLL3 protein (purchased from ACRO, DL3-H5255) at two-week intervals. Freund's complete adjuvant was used for the first immunization and Freund's incomplete adjuvant was used for subsequent immunizations. On days 35 and 49, blood samples were collected from the mice via orbital bleeding, and the immune serum titers of the mice were detected using conventional methods of ELISA and Flow Cytometry (FACS).

ELISA detection method: DLL3 protein was diluted with PBS to a concentration of 1µg/ml. 100µl/well was added to a 96-well ELISA plate and incubated overnight at 4°C. The next day, the supernatant was discarded, and each well was blocked with 200µl of 2% milk in PBS for 2 hours. After discarding the blocking solution, the wells were washed 3 times with 200µl of PBST (0.1% Tween20). The serum to be detected was pre-diluted 100 times as the starting concentration, followed by a 3-fold gradient dilution across 11 concentrations, with 100µl added to each well of the ELISA plate. After incubating at room temperature for 1 hour, the supernatant was discarded and the wells were washed 5 times with 200µl of PBST (0.1% Tween20). Then, 100µl of HRP-labeled goat anti-mouse secondary antibody (Jackson, 1:10000) was added and incubated at room temperature for 1 hour. After discarding the supernatant, the wells were washed 7 times with 200µl of PBST (0.1% Tween20). 100µl of TMB chromogen solution was added to each well for 10 minutes. The reaction was terminated with 2M HCl, and absorbance value at 450nm was read with a Microplate Reader (purchased from Biotek, Elx808).

FACS detection method: 3×10^5 HEK293-DLL3 stable cells or endogenously expressing cells SHP-77 (purchased from ATCC) were added to each well of a 96-well V-bottom plate (purchased from Axygen, wipp02280). The cells were centrifuged at 1500 r/min for 1 minute and the supernatant was discarded. The immunized serum was diluted with PBS at 1:50, followed by 4-fold gradient dilution across 8 concentrations, with 50µL/well added to the plate. The cells were incubated on ice for 30 minutes, then resuspended in 150µL of PBS, centrifuged at 1500 r/min for 1 minute, and the operation was repeated twice. APC-labeled goat anti-mouse IgG (Jackson, catalog number 115-605-164, diluted 1:800 in PBS) was added at 50µL/well and incubated on ice for 30 minutes. The cells were then resuspended in 150µL of PBS, centrifuged at 1500 r/min for 1 minute, and the operation was repeated twice. The samples were detected using a CytoFLEX flow cytometer (purchased from Beckman). After the detection, mice with the highest titer and consistent serum titer over two consecutive immunizations were selected for fusion. Three days before fusion, the mice were given a rush immunization via intraperitoneal injection with DLL3 protein.

### 3-2. Hybridoma Fusion, Screening, and Cloning

Cell suspensions were prepared from the spleens of Balb/C mice that had received rush immunization. The spleen cells were fused with SP2/0 cells (purchased from the Cell Bank of the Chinese Academy of Sciences, TCM42), by electrofusion. After fusion, the hybridoma cells were inoculated at 2×10⁴ cells per well in 96-well plates and selected using HAT medium. After 7 days, the supernatant from each well was detected using the ELISA method as described in Example 3-1. The ELISA-positive cells were further detected using FACS detection method to detect the 293 cell line overexpressing DLL3 and the SHP-77 endogenous cell line. The cell-binding positive clones were subjected to limited dilution and this process was repeated twice. The diluted clones were detected using the ELISA and FACS methods from Example 3-1 to confirm positive clones. The positive clone hybridomas detected after two limiting dilutions were expanded and used for antibody production and purification and antibody expression gene sequencing.

### 3-3. Hybridoma Antibody Production and Purification

To produce milligram quantities of antibodies for functional characterization, selected hybridoma cells were inoculated at a density of 2.5×10^5/mL in cell culture flasks comprising 250mL of serum-free hybridoma medium (purchased from Yuanpei Biotechnology, H630KJ) and incubated at 37°C, shaking at 130 r/min. Once the cell viability dropped to around 30%, the supernatant was collected by centrifugation. Murine monoclonal antibodies were purified using Protein G media and dialyzed into PBS buffer, pH 7.2. The concentration and purity of the antibody were determined using a micro-volume spectrophotometer (purchased from Hangzhou Aosheng Instrument, Nano-300).

### Example 4. Analysis of Antibody Internalization Activity

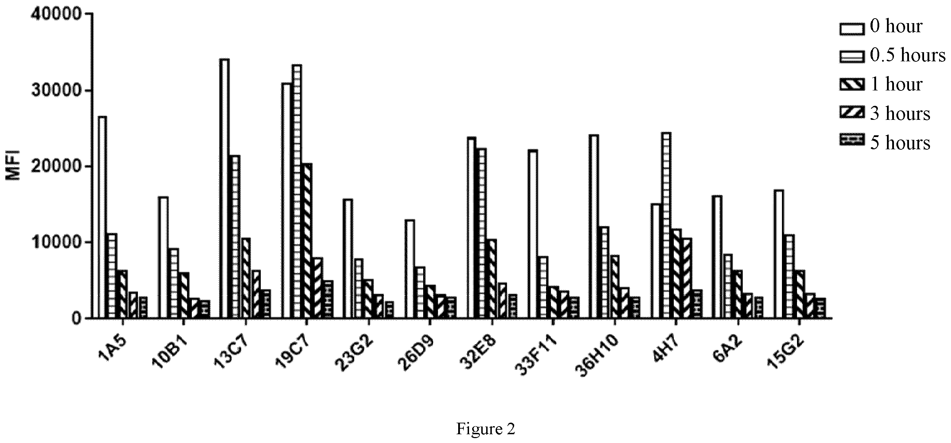

The SHP-77 cells were used to detect the internalization activity of the hybridoma antibodies obtained in Example 3-3 by using the FACS method described in Example 3-1. The detecting method is briefly described as follows: Six aliquots of 5×10⁵ SHP-77 cells each were prepared in a 96-well V-bottom plate and centrifuged at 1500 r/min for 1 minute, after which the supernatant was discarded. 1 µg/ml of the saturated antibody to be detected was added to each well (200µL per well) and incubated on ice for 30 minutes. Then, 150µL of PBS was added to each well, followed by centrifugation at 1500 r/min for 1 minute, and the supernatant was discarded. This step was repeated four times. The cells were resuspended in 200µl of PBS, and one aliquot was incubated at 37°C for various durations: 5 hours, 3 hours, 1 hour, 0.5 hours, and 15 minutes. The remaining aliquot was kept on ice. After incubation, all samples were centrifuged at 4°C, 1500 r/min for 1 minute, and the supernatant was discarded. APC-labeled goat anti-mouse IgG (Jackson, catalog number 109-605-098, diluted 1:800 in PBS) was added to each well (50µL per well) and incubated on ice for 30 minutes. The cells were then washed four times by resuspending in 150µL of PBS, followed by centrifugation at 1500 r/min for 1 minute and discarding the supernatant. Finally, the cells were resuspended in 100 µL of PBS and analyzed using a CytoFLEX flow cytometer (purchased from Beckman). The fluorescence values for antibody internalizationare presented in Table 1. Figure 2 shows the internalization results of the antibodies obtained in 3-3 of Example 3, indicating that these antibodies exhibit excellent internalization activity.

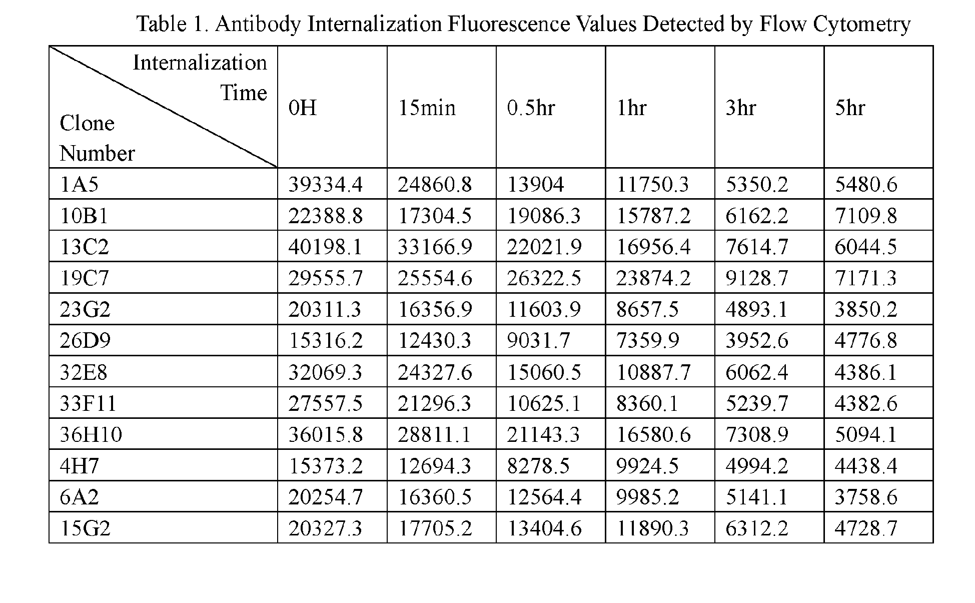

### Example 5. Acquisition of the Expression Gene Sequences of the Hybridoma Antibodies

From each clone, 2×10⁵ hybridoma cells (originating from Example 3-2) were collected for RNA extraction. The cells were centrifuged at 300 g (the unit of rotational speed is used in this example, the same below) for 5 minutes and the supernatant was discarded. 250 µL of Trizol lysis buffer (purchased from TAKARA, T9108) was added to lyse the hybridoma cells. Then, 50 µL of chloroform was added, vortexed until the mixture turned milky, and allowed to stand at room temperature for 5 minutes. After centrifugation at 12000 g at 4°C for 15 minutes, the supernatant was transferred to a new RNase-free 1.5 mL centrifuge tube. 125 µL of isopropanol was added, mixed well, and incubated at room temperature for 10 minutes. 12000g, centrifuge for 10 minutes at 4°C to see the precipitate, the supernatant was discarded, and 250 µL of 75% ethanol was added. The tube was gently inverted and centrifuged at 12000 g at 4°C for 10 minutes, after which the supernatant was discarded, and the precipitate was air-dried at room temperature for 10 minutes. 20 µL of RNase-free water was then added to dissolve the precipitate for 30 minutes. After dissolution, it was mixed with a pipette and the concentration was determined. The RNA of the clones was reverse transcribed into cDNA using the PrimeScript^{™} RT Master Mix Kit (purchased from TAKARA, RR036A) according to the RNA concentration. Antibody light and heavy chain-specific primers were synthesized and used to obtain the antibody light and heavy chain sequences via PCR. The amplified fragments were gel recovered using the Universal DNA Purification Recovery Kit (purchased from TIANGEN Biotech, DP214-03), and then ligated with the pMD18T vector (purchased from TAKARA, 6011) at 4°C for 30 minutes. 5 µL of the ligation product was added to 50 µL of TG-1 competent cells (purchased from Lucigen), gently mixed, and subjected to heat shock at 42°C for 90 seconds. The cells were then incubated on ice for 3 minutes, followed by the addition of 1 mL of 2 YT medium without antibiotics (purchased from Sangon Biotech, A507019-0250), and incubated at 37°C on a shaker at 220 rpm for 1 hour. 100 µL of the culture was then spread on a 2 YT agar plate with Ampicillin resistance (purchased from Sangon Biotech, A600469-0005) and incubated at 37°C for 14-16 hours. Monoclonies were picked for sequencing by Sangon Biotech. The sequencing results were analyzed using Vector NTI, Vbase2 software to obtain the amino acid sequences of the antibodies with good binding and internalization activities. The results are presented in Table 2.

Table 2. Amino Acid Sequences Numbers of each CDR and Variable Region of the Light and Heavy Chain of the Obtained Antibodies (using Kabat numbering)

| | | | | | | | | |
|---|---|---|---|---|---|---|---|---|
| Anti body | Heavy Chain CDR1 (VH CDR1) | Heavy Chain CDR2 (VH CDR2) | Heavy Chain CDR3 (VH CDR3) | Light Chain CDR1 (VL CDR1) | Light Chain CDR2 (VL CDR2) | Light Chain CDR3 (VL CDR3) | Heavy Chain Variable Region (VH) | Light Chain Variable Region (VL) |
| 1A5 | SEQ ID NO: 9 GYTFT ESA | SEQ ID NO: 10 INPNN GGI | SEQ ID NO: 11 GSSYY RDYFD Y | SEQ ID NO: 12 ENVGT YV | GAS | SEQ ID NO: 14 GQSYS YPLT | SEQ ID NO: 15 | SEQ ID NO: 16 |
| 33F1 1 | SEQ ID NO: 19 GYTFT ENT | SEQ ID NO: 20 INPRN GVT | SEQ ID NO: 21 TNTYY RDYFD Y | SEQ ID NO: 12 ENVGT YV | GAS | SEQ ID NO: 24 GQTYT YPLT | SEQ ID NO: 25 | SEQ ID NO: 26 |
| 23G 2 | SEQ ID NO: 29 GYTFT DYA | SEQ ID NO: 30 IVSTDS GKT | SEQ ID NO: 31 ARNPY YDYDG YAMD Y | SEQ ID NO: 32 DHINN W | GAT | SEQ ID NO: 34 QQYW SIPYT | SEQ ID NO: 35 | SEQ ID NO: 36 |
| 32E8 | SEQ ID NO: 39 GFSLST SGMG | SEQ ID NO: 40 IWWD DVK | SEQ ID NO: 41 ARIAE VTGQV GLHYA MDY | SEQ ID NO: 42 SSVNS SS | TTS | SEQ ID NO: 44 HQFHR SPFT | SEQ ID NO: 45 | SEQ ID NO: 46 |
| 13C2 | SEQ ID NO: 49 GFAFSS YD | SEQ ID NO: 50 ISSGGS YT | SEQ ID NO: 51 ASPLL GLRFA Y | SEQ ID NO: 52 TGAVT TINY | GTN | SEQ ID NO: 54 ALYYS NHWV | SEQ ID NO: 55 | SEQ ID NO: 56 |
| 19C7 | SEQ ID NO: 59 GYTFT YYVM | SEQ ID NO: 60 INPYN DGT | SEQ ID NO: 61 ARWLL REYAM DY | SEQ ID NO: 62 DNIHN Y | NAK | SEQ ID NO: 64 QHFWS TPWT | SEQ ID NO: 65 | SEQ ID NO: 66 |
| 26D 9 | SEQ ID NO: 69 GYTFT TYV | SEQ ID NO: 70 INPYN DDT | SEQ ID NO: 71 ARWGL TGTGD YYAM DY | SEQ ID NO: 72 GNVHN Y | NAK | SEQ ID NO: 74 QHFWT TPWT | SEQ ID NO: 75 | SEQ ID NO: 76 |
| 10B1 | SEQ ID NO: 79 FTFSN YT | SEQ ID NO: 80 FSSGG SYT | SEQ ID NO: 81 SRDRR SDGYY HLYAM DY | SEQ ID NO: 82 QDISH Y | YTS | SEQ ID NO: 84 QQGYT LPLT | SEQ ID NO: 85 | SEQ ID NO: 86 |
| 36H 10 | SEQ ID NO: 89 GYTFT DYN | SEQ ID NO: 90 IYPFNG GI | SEQ ID NO: 91 ARLN WEGY | SEQ ID NO: 92 QDINS Y | RAN | SEQ ID NO: 94 LQYAE FPYT | SEQ ID NO: 95 | SEQ ID NO: 96 |
| 4H7 | SEQ ID NO: 99 GFNFK DYY | SEQ ID NO: 100 IDPEN GNA | SEQ ID NO: 101 ASEDA YYPFA Y | SEQ ID NO: 102 QSLLY SSNQK NY | WAS | SEQ ID NO: 104 QQYYS YRT | SEQ ID NO: 105 | SEQ ID NO: 106 |
| 6A2 | SEQ ID NO: 89 GYTFT DYN | SEQ ID NO: 11[?] IYPYN GGT | SEQ ID NO: 111 ARSDP YYTM DY | SEQ ID NO: 112 QSVNN D | FTS | SEQ ID NO: 114 QQDHN SPYT | SEQ ID NO: 13 | SEQ ID NO: 33 |
| 15G 2 | SEQ ID NO: 63 GYSFT DYN | SEQ ID NO: 83 IDPYY GGT | SEQ ID NO: 93 ARGGN NYGDY | SEQ ID NO: 103 ENIYY S | NAN | SEQ ID NO: 113 NANKQ AYDVP WT | SEQ ID NO: 22 | SEQ ID NO: 23 |

### Example 6. Construction, Preparation, and Binding Activity Detection of Chimeric Antibodies

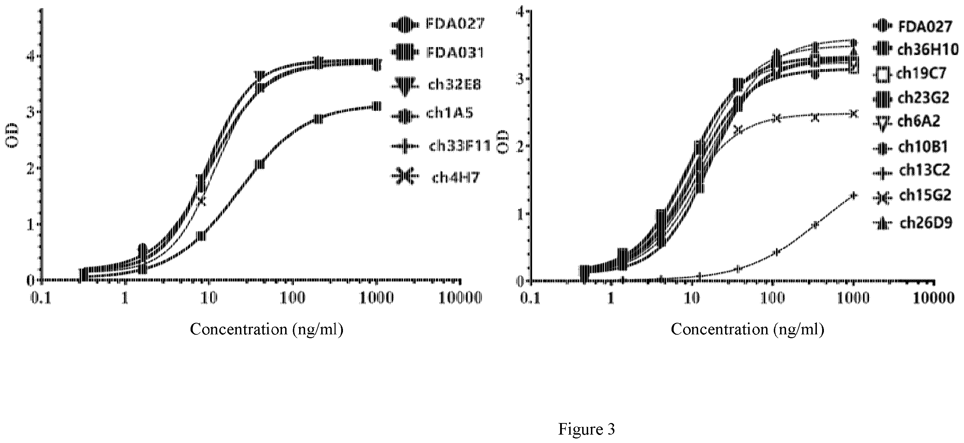

For the hybridoma clones with good binding and internalization activities identified in Example 5, the heavy chain variable region sequences were synthesized and cloned into the pFUSEss-CHIg-hG1 vector (purchased from InvivoGen, catalog number pfusess-hchg1) comprising the amino acid sequence of the human antibody IgG1 heavy chain constant region by homologous recombination. This process resulted in the construction of chimeric antibody heavy chain expression vectors. Similarly, the cloned light chain variable region sequences were homologously recombined into the pFUSE2ss-CLIg-hk vector (purchased from InvivoGen, catalog number pfuse2ss-hclk) comprising the human antibody Kappa light chain constant region CL, obtaining the chimeric antibody light chain expression vectors. The amino acid sequence numbers corresponding to the full length of the light and heavy chains of the constructed chimeric antibodies are shown in Table 3-1. Well-growing logarithmic phase 293F cells were collected and inoculated into 250 mL cell culture flasks in 50 mL of culture medium. PEI was used to cotransfect 25 µg each of the light and heavy chain expression plasmids. The supernatant on the 7th day post-transfection was collected, centrifuged, and filtered through a 0.45 µM filter. The antibodies were purified using Protein A media and dialyzed into PBS buffer, pH 7.2. The concentration of the antibodies was determined using a micro-volume spectrophotometer (purchased from Hangzhou Aosheng Instrument, Nano-300). The binding activity of the obtained chimeric antibodies to human DLL3 protein was detected using the ELISA method (results shown in Figure 3 and Table 3-2), as described in Example 3-1. As indicated in Figure 3, the obtained chimeric antibodies exhibit superior binding activity to human DLL3 protein.

**Table 3-1. Amino Acid Sequence Numbers for the Full Length of the Light and Heavy Chains of the Chimeric Antibodies**

| Chimeric Antibody | Full-Length Heavy Chain | Full-Length Light Chain |
|---|---|---|
| ch1A5 | SEQ ID NO: 17 | SEQ ID NO: 18 |
| ch33F11 | SEQ ID NO: 27 | SEQ ID NO: 28 |
| ch23G2 | SEQ ID NO: 37 | SEQ ID NO: 38 |
| ch32E8 | SEQ ID NO: 47 | SEQ ID NO: 48 |
| ch13C2 | SEQ ID NO: 57 | SEQ ID NO: 58 |
| ch19C7 | SEQ ID NO: 67 | SEQ ID NO: 68 |
| ch26D9 | SEQ ID NO: 77 | SEQ ID NO: 78 |
| ch10B1 | SEQ ID NO: 87 | SEQ ID NO: 88 |
| ch36H10 | SEQ ID NO: 97 | SEQ ID NO: 98 |
| ch4H7 | SEQ ID NO: 107 | SEQ ID NO: 108 |
| ch6A2 | SEQ ID NO: 43 | SEQ ID NO: 53 |
| ch15G2 | SEQ ID NO: 73 | SEQ ID NO: 109 |

**Table 3-2. Binding Activity of Chimeric Antibodies with Human DLL3 Protein**

| Chimeric Antibodies | EC50 (ng/mL) |
|---|---|
| FDA027 | 10.84* |
| FDA031 | 23.35 |
| ch32E8 | 8.828 |
| ch1A5 | 9.213 |
| ch33F11 | 9.546 |
| ch23G2 | 9.406 |
| ch4H7 | 11.48 |
| ch19C7 | 8.658 |
| ch6A2 | 13.13 |
| ch36H10 | 15.88 |
| ch10B1 | 16.77 |
| ch26D9 | 11.78 |
| ch13C2 | 394.8 |
| ch15G2 | 8.526 |

### Example 7. Competition Analysis and Binding Epitope Analysis of Chimeric Antibodies

The chimeric antibodies obtained in Example 6 were labeled with Biotin, and their competition binding activities were detected through the ELISA method. The detection method is briefly described as follows: The concentration of DLL3 protein was diluted with PBS to 1 µg/ml and coated onto a 384-well ELISA plate (purchased from Corning, 3700) overnight. The next day, the supernatant in the plate wells was discarded, and the wells were blocked with 80 µl of 3% milk (dissolved in PBS) for 2 hours. The wells were then washed three times with 80 µl of PBST (0.1% Tween20). Afterwards, the unlabeled chimeric antibodies were pre-diluted to 100 µg/ml and further diluted in a 3-fold gradient across 11 concentration points, with 25µl added to each well of the previously blocked ELISA plate, incubated at room temperature for 1 hour, after which the supernatant was discarded, and the wells were washed five times with 80µl of PBST (0.1% Tween20). Then, 25 µl of the Biotin-labeled chimeric antibodies were added and incubated at room temperature for 1 hour, followed by another round of washing with 80 µl of PBST (0.1% Tween20) five times. Finally, HRP-labeled streptavidin secondary antibody (1:5000, purchased from Kingfisher Biotech, M00091) was added and incubated at room temperature for 1 hour, after which the supernatant was discarded, and the wells were washed seven times with 80µl of PBST (0.1% Tween20). Then, 25µl of TMB chromogen solution was added for 10 minutes, and the reaction was terminated with 2M HCl. The absorbance value at 450nm was read with a Microplate reader (purchased from Biotek, Elx808).

To determine the specific binding regions of the antibodies, HEK293 cell lines overexpressing different hDLL3 structural domains (corresponding sequences: SEQ ID NO: 5, SEQ ID NO: 6, SEQ ID NO: 7, and SEQ ID NO: 8) were analyzed by FACS to determine the binding ability of the chimeric antibodies to these four cell types, identifying the antibody binding regions. In brief: 5×10⁵ cells overexpressing DSL, EGF1, EGF2, and EGF3 were placed in 96-well V-bottom plates. The cells were centrifuged at 1500rpm for 1 minute and the supernatant was discarded. 1µg/ml of the saturated antibody to be detected was added to each well (200µL per well) and incubated on ice for 30 minutes. Then 150 µL of PBS was added to each well, centrifuged at 1500 r/min for 1 min, the supernatant was discarded, and the plate washing was repeated 4 times. APC-labeled goat anti-human IgG (Jackson, diluted 1:800 in PBS) was added to each well (50µL per well) and incubated on ice for 30 minutes. After washing with PBS four times, 100 µL PBS was added to each well to resuspend the cells, which were then analyzed using CytoFLEX (purchased from Beckman). The results showed that antibodies ch1A5, ch33F11, and ch32E8 bind to the EGF2 domain of DLL3; FDA031, ch13C2, and ch15G2 bind to the N-terminal of DLL3; FDA027 binds to the DSL domain of DLL3; and the remaining antibodies bind to the EGF3-EGF6 domains of DLL3. Thus, the binding epitope of ch15G2 is identified as the N-terminal domain; the binding epitope of ch32E8, ch1A5, and ch33F11 is the EGF2 domain; and the binding epitopes of ch36H10, ch6A2, ch19C7, ch26D9, ch23G2, ch10B1, and ch4H7 are the EGF3-EGF6 domains.

### Example 8. Cross-Reactivity of Antibodies with Human DLL1, DLL4 Proteins, and Mouse, Monkey DLL3 Cells

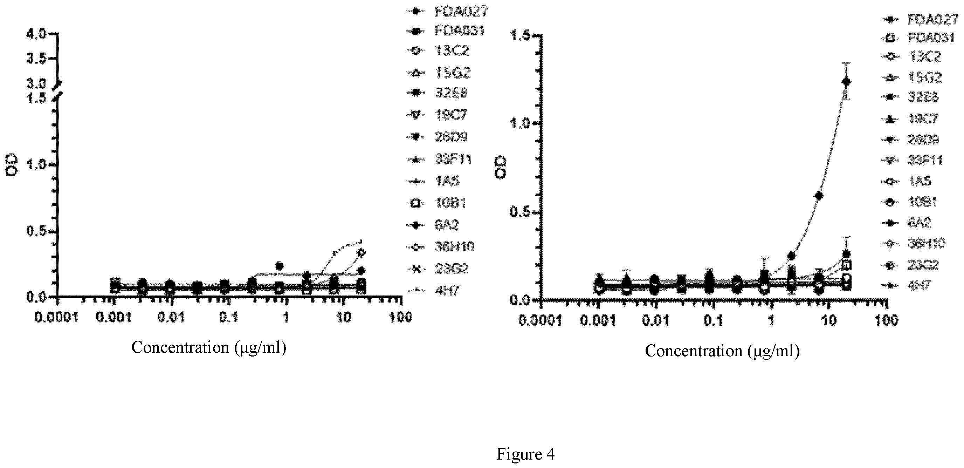

The chimeric antibodies obtained in Example 6 were evaluated for their binding abilities to human DLL1 (purchased from Aero, DL1-H52H8) and DLL4 (purchased from Aero, DL4-H5227) proteins using the ELISA method. The ELISA detection method involved diluting DLL1 and DLL4 proteins with PBS to a final concentration of 1µg/ml and coating them onto 384-well ELISA plates (purchased from Corning, 3700) at 4°C overnight. The next day, the supernatant in the plate wells was discarded, and the wells were blocked with 80 µl of 3% milk (dissolved in PBS) for 2 hours, followed by washing three times with 80µl of PBST (0.1% Tween20). The chimeric antibodies to be detected, FDA027, and FDA031 were then diluted to 20 µg/ml respectively, and further diluted in a 3-fold gradient across 11 concentration points, with 25 µl added to each well of the previously blocked ELISA plate, and incubated at room temperature for 1 hour. After washing with 80 µl of PBST (0.1% Tween20) for five times, 25 µl of HRP-labeled goat anti-human secondary antibody (Jackson, 1:10000) was added to each well and incubated at room temperature for 1 hour, discard the supernatant, and the wells were then washed seven times with 80 µl of PBST (0.1% Tween20). After adding 25 µl of TMB chromogen solution for 10 minutes, the reaction was terminated with 2M HCl, and the absorbance value at 450nm was measured with a Microplate reader (purchased from Biotek, Elx808). The results (shown in Figure 4) indicated that the 12 candidate antibodies showed no significant cross-reactivity with hDLL1 and hDLL4 proteins, with antibodies 36H10 and 4H7 showing weak cross-reactivity with hDLL1 at high concentrations (20 µg/mL), and antibody 6A2 showing some cross-reactivity with hDLL4 at high concentrations (20 µg/mL).

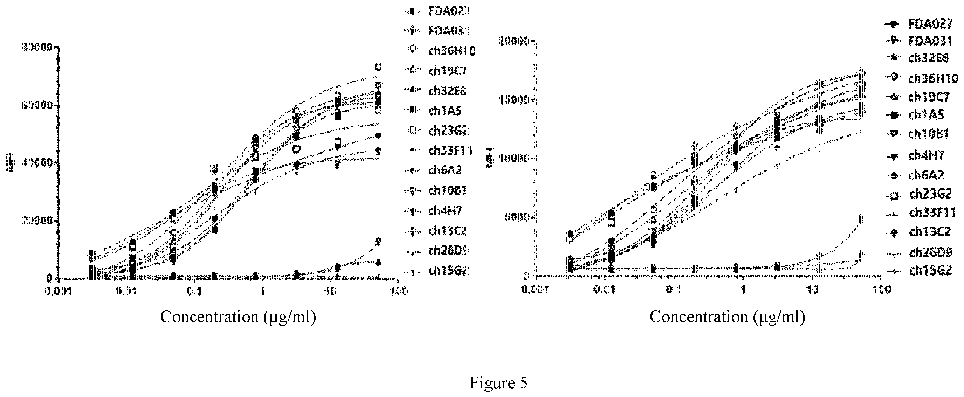

To assess the cross-reactivity of the antibodies with mouse and monkey DLL3, mouse DLL3-expressing plasmid pCMV3-mDLL3 (purchased from Beijing Yiqiao, MG58052-UT) and monkey DLL3-expressing plasmid pCMV3-rheDLL3 (purchased from Beijing Yiqiao, CG90919-UT) were used to construct overexpressing cell lines. The construction of overexpressing cells was as described in Examples 2-3. The binding abilities of the chimeric antibodies to these cells were determined by flow cytometry to assess the cross-reactivity of the antibodies with different species. 5×10⁵ cells of Mouse-DLL3 and Monkey-DLL3 were inoculated in 96-well V-bottom plates (purchased from Axygen, wipp02280), centrifuged at 1500rpm for 1 minute, and the supernatant was discarded. The chimeric antibodies, FDA027, and FDA031 were pre-diluted to 50µg/ml, and further diluted in a 4-fold gradient across 8 concentration points, with 200µL of the antibodies to be detected of different concentrations added to each well and incubated on ice for 30 minutes. Each well then added 150µL PBS, centrifuged at 1500rpm for 1 minute, and the supernatant was discarded. This washing step was repeated four times. APC-labeled goat anti-human IgG (Jackson, diluted 1:800 in PBS) was added to each well (50µL per well) and incubated on ice for 30 minutes. After washing with PBS four times, 100µL PBS was added to each well to resuspend the cells, which were then analyzed using CytoFLEX (Beckman). The results (as shown in Figure 5) indicated that, except for ch32E8, ch13C2, and ch15G2, which did not recognize mouse and monkey DLL3, the remaining 9 antibodies were able to bind to both mouse and monkey DLL3.

### Example 9. Affinity Detection of the Antibodies Binding to Human DLL3 Protein

To assess the affinity of antibodies for human DLL3 protein, this study employed the BLI method to detect the binding kinetics curves of immobilized antibodies with free DLL3. The method was conducted according to the instrument's user manual (supplier: Fortebio, equipment model: Octet 96e). Briefly, AMC sensors were equilibrated for 60s with Loading Buffer/Sample dilution buffer (1×PBS, pH7.4, with 0.1%BSA and 0.02% Tween-20) to obtain Baseline 1. The antibodies to be detected were diluted with Loading Buffer to a concentration of 10ug/ml and bound to the equilibrated sensors, after which the sensors were re-equilibrated with Loading Buffer to obtain Baseline 2. The antibody-loaded sensors were then placed in human DLL3, His tag (supplier: acrobiosystems, catalog number: DL3-H52H4) diluted with Sample Dilution Buffer to concentrations ranging from 100 to 3.13 nM for 90s to obtain the antibody-protein binding curve. Then, the sensors combined with antigen were placed in the Sample Dilution Buffer again and dissociated for 180s, and the dissociation curve was obtained. The k-on and k-off values of the antibody-protein binding were calculated based on the binding and dissociation curves, and the KD values were calculated, with the results shown in Table 4. The results indicated that these chimeric antibodies exhibited strong affinity at the protein level.

**Table 4. Binding Affinity of Antibodies to Human DLL3 Protein**

| Antibody Names | KD (M) | kon (1/Ms) | Koff (1/s) |
|---|---|---|---|
| ch1A5 | 5.97E-11 | 1.24E+06 | 7.40E-05 |
| ch33F11 | 5.21E-11 | 1.21E+06 | 6.31E-05 |
| ch23G2 | 1.58E-11 | 1.76E+06 | 2.79E-05 |
| ch13C2 | 5.37E-09 | 2.23E+05 | 1.20E-03 |
| ch32E8 | 9.00E-11 | 1.49E+06 | 1.34E-04 |
| ch19C7 | 7.39E-10 | 1.30E+06 | 9.62E-04 |
| ch26D9 | 3.81E-11 | 1.73E+06 | 6.58E-05 |
| ch10B1 | 2.04E-10 | 1.37E+06 | 2.79E-04 |
| ch36H10 | 9.91E-11 | 1.69E+06 | 1.68E-04 |
| ch4H7 | 1.04E-10 | 1.07E+06 | 1.11E-04 |
| ch6A2 | 4.66E-10 | 1.47E+06 | 6.84E-04 |
| ch15G2 | 7.02E-11 | 1.72E+06 | 1.21E-04 |

### Example 10. Preparation of Linker-Drug Conjugates

The linker-drug conjugates used in the present disclosure, LE00 and LE01-LE24, are outlined in Table 5. LE00 (GGFG-Dxd) was synthesized following the method reported in WO2015146132A, and LE01-LE24 were synthesized following the methods reported in WO2020259258A1.

**Table 5. Structure of Linker-Drug Conjugates**

| **Linker-Drug Conjugates** | **Referenced Synthesis Methods** |
|---|---|
| 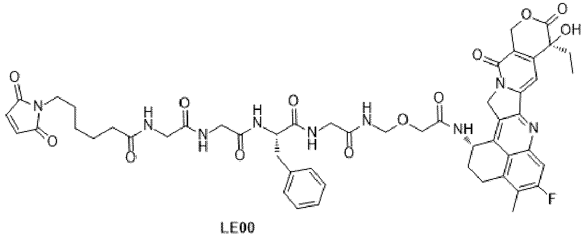 | WO2015146132A 1 Example 7 |
| 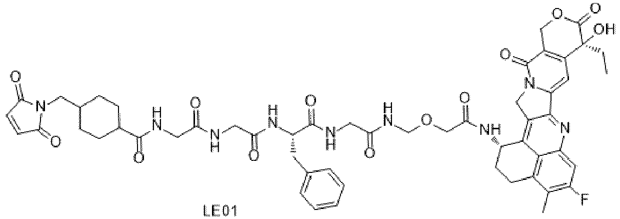 | WO2020259258A 1 Example 1 |
| 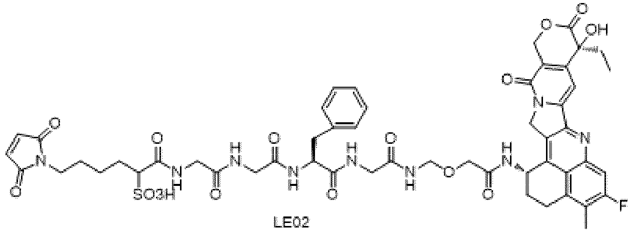 | WO2020259258A 1 Example 2 |
| 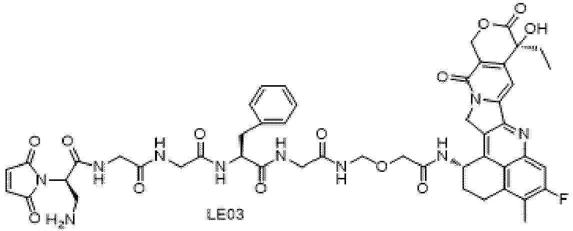 | WO2020259258A 1 Example 2 |
| 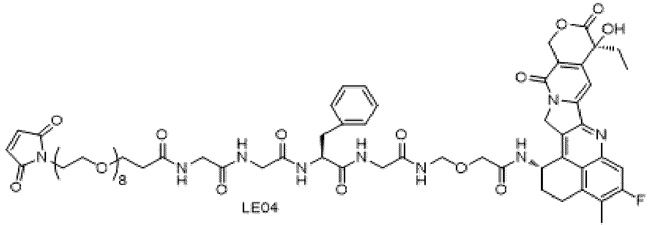 | WO2020259258A 1 Example 2 |
| 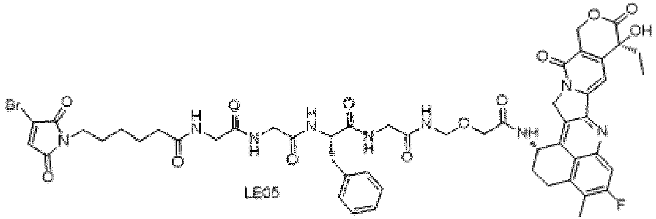 | WO2020259258A 1 Example 2 |
| 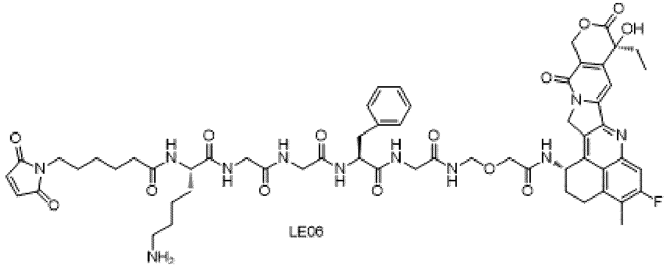 | WO2020259258A 1 Example 2 |
| 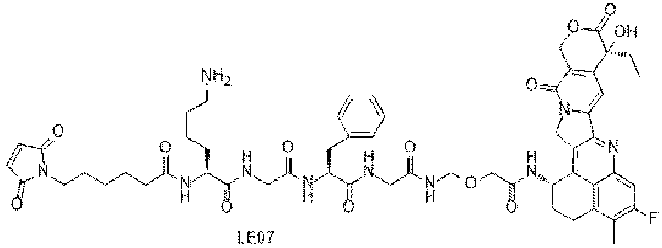 | WO2020259258A 1 Example 2 |
| 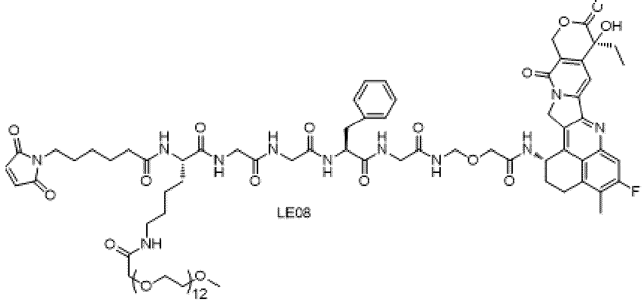 | WO2020259258A 1 Example 2 |
| 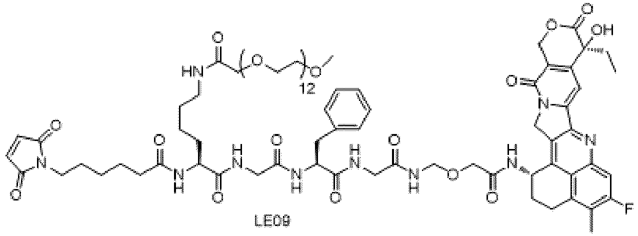 | WO2020259258A 1 Example 4 |
| 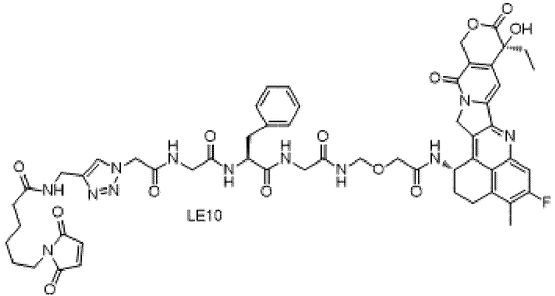 | WO2020259258A 1 Example 4 |
| 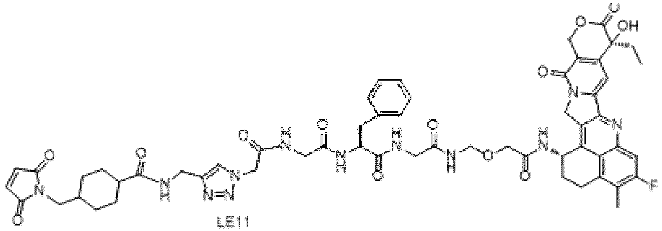 | WO2020259258A 1 Example 4 |
| 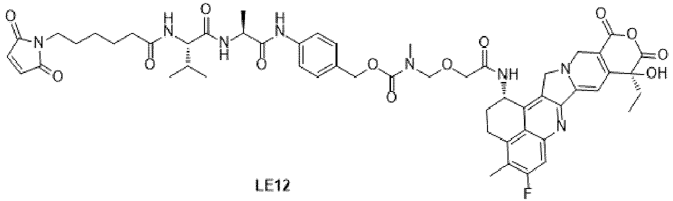 | WO2020259258A 1 Example 6 |
| 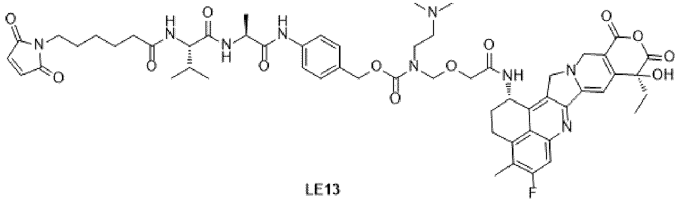 | WO2020259258A 1 Example 7 |
| 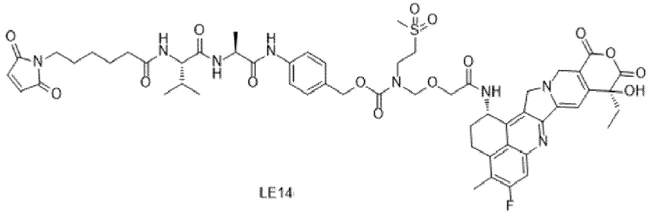 | WO2020259258A 1 Example 7 |
| 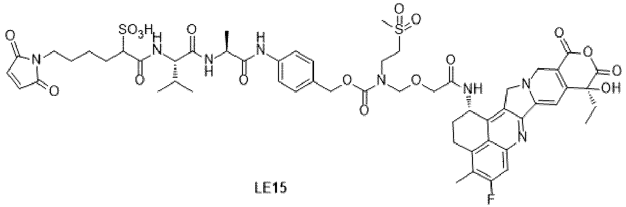 | WO2020259258A 1 Example 7 |
| 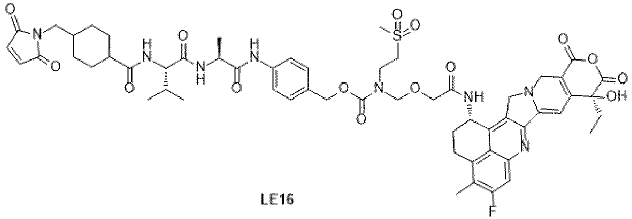 | WO2020259258A 1 Example 7 |
| 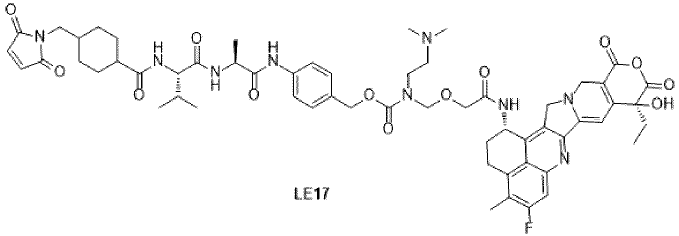 | WO2020259258A 1 Example 7 |
| 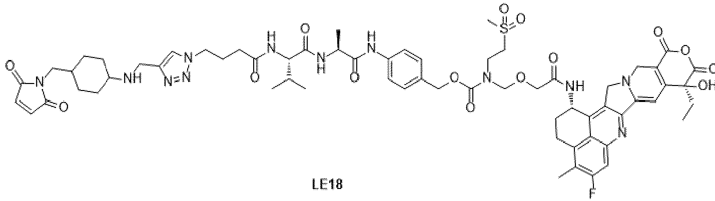 | WO2020259258A 1 Example 7 |
| 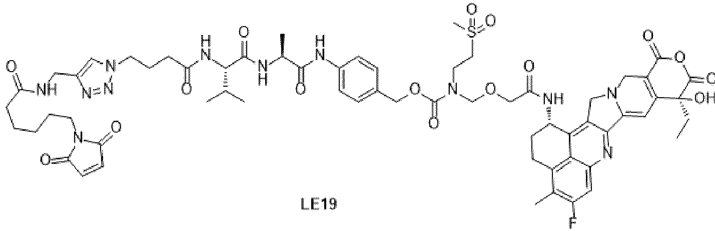 | WO2020259258A 1 Example 7 |
| 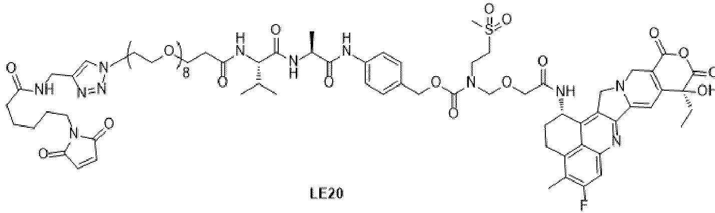 | WO2020259258A 1 Example 7 |
| 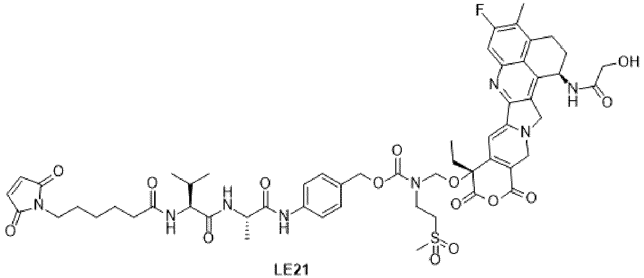 | WO2020259258A 1 Example 8 |
| 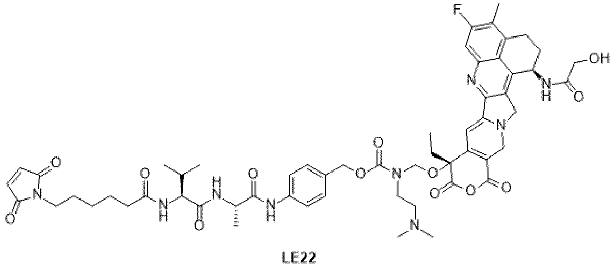 | WO2020259258A 1 Example 8 |
| 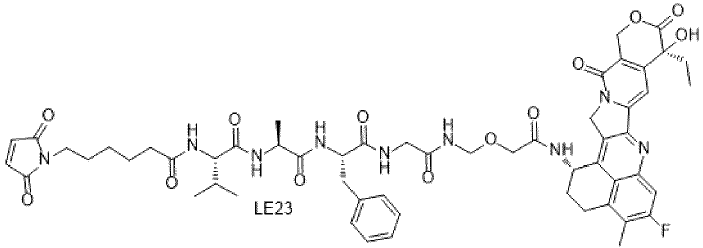 | WO2020259258A 1 Example 5 |
| 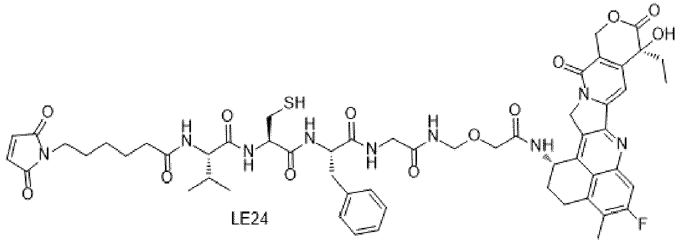 | WO2020259258A 1 Example 5 |

### Example 11. Preparation of ADCs by Conjugation of Antibodies with Linker-Drug Conjugates

Different anti-DLL3 antibodies obtained from Examples 1 and 6 were exchanged into a 50 mM PB/1.0 mM EDTA buffer (pH 7.0) using G25 desalting columns. Then, 12 equivalents of TECP were added and the mixture was stirred at 37°C for 2 hours to fully open the inter-chain disulfide bonds of the antibodies. The pH of the reduced antibody solution was subsequently adjusted to 6.0 using phosphoric acid, and the water bath temperature was lowered to 25°C to prepare for the conjugation reaction. The linker-drug conjugates prepared as per Example 10 were dissolved in DMSO, and 12 equivalents of the linker-drug conjugate were added dropwise to the reduced anti-DLL3 antibody solution respectively. Each sample was prepared according to the maximum Drug-to-Antibody Ratio (DAR)((i.e. excessive coupling), observing the formation of any precipitates during the conjugation reactions. DMSO was added to reach a final concentration of 10% (v/v), and the mixture was stirred at 25°C for 0.5 hours. After the reaction, the samples were filtered through a 0.22 µm membrane. Unconjugated small molecules were removed by purification using a tangential flow ultrafiltration system, with the buffer being 50 mM PB/1.0 mM EDTA solution (pH 6.0). After the purification, a final concentration of 6% sucrose was added, and the samples were stored at -20°C. The UV method was used to measure absorbance values at 280 nm and 370 nm, from which DAR values were calculated. The results are shown in Table 6. The results indicate that the antibodies ch1A5, ch33F11, ch32E8, ch13C2, FDA027, FDA031, ch15G2, and ch4H7 were successfully conjugated with LE14. ch33F11 was successfully conjugated with LE01-LE11, LE23, and LE24; ch1A5 was successfully conjugated withLE00, LE12, LE13, LE15, LE16, LE17, LE18, LE19, LE20, LE21 and LE22. The DAR values and free Dxd contents of the resulting ADCs met the expected requirements. However, antibodies ch19C7, ch23G2, ch6A2, and ch26D9 showed noticeable flocculent precipitation during centrifugal concentration when conjugated with LE14, resulting in a lower yield and indicating that their drug conjugation was not feasible. The ADC samples from ch36H10 and ch10B1 had DAR values of 1.89 and 1.90, respectively, both had high impurity contents. Particularly, ch36H10 had a high residual of small molecules (49.35%), indicating a failed conjugation.

**Table 6. Druggability Evaluation of ADC Samples Prepared by Conjugating Different Antibodies with LE14**

| ADC Name | Concentrati on (g/L) | Volum e (mL) | Total Amou nt (mg) | Polymer Proporti on (%) | DA R | Impurity Percenta ge | Free Dxd Conte nt (%) | Recove ry Rate |
|---|---|---|---|---|---|---|---|---|
| ch1A5-LE00 | 6.42 | 4.1 | 26.32 | 4.5 | 7.68 | 2.53 | 0.45 | 86.23% |
| ch33F1 1-LE01 | 4.78 | 4.2 | 20.07 | 3.2 | 7.32 | 3.23 | 0.32 | 88.23% |
| ch33F1 1-LE02 | 5.43 | 3.8 | 20.63 | 2.8 | 7.45 | 1.24 | 0.46 | 87.12% |
| ch33F1 1-LE03 | 6.43 | 5.6 | 36.00 | 4.2 | 7.65 | 0.78 | 0.78 | 84.53% |
| ch33F1 1-LE04 | 4.56 | 3.4 | 15.50 | 3.4 | 7.64 | 0.32 | 0.12 | 83.67% |
| ch33F1 1-LE05 | 5.12 | 4.2 | 21.5 | 4.3 | 7.36 | 4.32 | 0.35 | 89.43% |
| ch33F1 1-LE06 | 4.68 | 4.3 | 20.12 | 2.9 | 7.23 | 5.43 | 0.23 | 88.5% |
| ch33F1 1-LE07 | 5.32 | 5.2 | 27.66 | 2.7 | 7.56 | 0.89 | 1.32 | 86.53% |
| ch33F1 1-LE08 | 5.89 | 4.6 | 27.09 | 3.5 | 7.75 | 1.45 | 0.87 | 88.04% |
| ch33F1 1-LE09 | 6.76 | 5.3 | 33.83 | 4.6 | 7.80 | 2.34 | 1.45 | 84.65% |
| ch33F1 1-LE10 | 6.23 | 3.9 | 24.30 | 0.8 | 7.77 | 3.24 | 2.32 | 83.4% |
| ch33F1 1-LE11 | 5.68 | 4.1 | 23.29 | 2.3 | 7.56 | 3.56 | 1.43 | 87.6% |
| ch1A5-LE12 | 5.63 | 3.8 | 21.4 | 5.2 | 7.53 | 3.52 | 1.23 | 88.0% |
| ch1A5-LE13 | 4.58 | 4.3 | 19.69 | 3.82 | 4.43 | 2.32 | 1.32 | 85.3% |
| ch1A5-LE15 | 6.52 | 3.6 | 23.47 | 0.7 | 7.12 | 3.5 | 0.32 | 88.5% |
| ch1A5-LE16 | 5.83 | 2.7 | 15.74 | 5.23 | 6.92 | 4.38 | 0.65 | 82.3% |
| ch1A5-LE17 | 4.86 | 2.6 | 12.64 | 1.62 | 7.43 | 5.62 | 1.57 | 88% |
| ch1A5-LE18 | 5.38 | 3.5 | 18.83 | 2.35 | 7.23 | 1.36 | 0.57 | 89.3% |
| ch1A5-LE19 | 6.21 | 4.2 | 26.08 | 1.83 | 6.83 | 5.62 | 1.23 | 82.3% |
| ch1A5-LE20 | 4.32 | 3.3 | 14.26 | 3.62 | 7.32 | 4.51 | 0.78 | 88.2% |
| ch1A5-LE21 | 4.25 | 3.5 | 14.88 | 2.45 | 7.56 | 2.38 | 0.56 | 83.1% |
| ch1A5-LE22 | 5.86 | 4.1 | 24.02 | 1.53 | 7.12 | 6.21 | 0.83 | 76.3% |
| ch33F1 1-LE23 | 5.23 | 4.3 | 22.49 | 1.23 | 7.64 | 3.87 | 0.12 | 87.8% |
| ch33F1 1-LE24 | 4.76 | 3.8 | 18.09 | 1.34 | 7.76 | 6.32 | 0.24 | 86.5% |
| ch1A5-LE14 | 6.89 | 3.5 | 24.12 | 4.2 | 7.54 | 2.47 | 0.57 | 85.2% |
| ch33F1 1-LE14 | 5.90 | 4.2 | 24.78 | 13.5 | 7.47 | 4.33 | 0.26 | 77.7% |
| ch32E8-LE14 | 6.59 | 4.0 | 26.36 | 3.2 | 5.82 | 26.50 | 2.27 | 81.8% |
| ch4H7-LE14 | 5.46 | 2.5 | 13.65 | 3.0 | 6.78 | 14.23 | 1.10 | 73.8% |
| ch19C7-LE14 | 2.14 | 3.1 | 6.63 | 1.5 | 2.28 | 19.19 | 1.72 | 30.6% |
| ch23G2 -LE14 | 5.13 | 3.8 | 19.49 | 0.7 | 6.32 | 12.41 | 1.08 | 70.7% |
| ch6A2-LE14 | 1.44 | 4.0 | 5.76 | 0.6 | 1.41 | 63.69 | 18.26 | 58.1% |
| ch36H1 0-LE14 | 6.43 | 4.0 | 25.72 | 2.9 | 1.89 | 72.01 | 49.35 | 79.3% |
| ch10B1-LE14 | 5.96 | 4.0 | 23.84 | 11.2 | 1.90 | 50.53 | 1.38 | 74.6% |
| FDA02 7-LE14 | 5.83 | 4.2 | 24.49 | 0.8 | 7.23 | 23.1 | 0.56 | 85.3% |
| FDA03 1-LE14 | 6.23 | 4.1 | 25.54 | 1.2 | 7.32 | 12.3 | 0.32 | 83.1% |
| ch13C2-LE14 | 6.23 | 3.8 | 23.67 | 2.3 | 7.12 | 5.87 | 1.35 | 79.1% |
| ch26D9 -LE14 | 4.32 | 2.8 | 12.1 | 5.6 | 6.32 | 32.1 | 7.83 | 68% |
| Ch15G2 -LE14 | 5.12 | 2.9 | 14.85 | 3.2 | 7.32 | 12.3 | 5.63 | 72% |

### Example 12. In Vitro Cytotoxic Activity Evaluation of ADC Samples

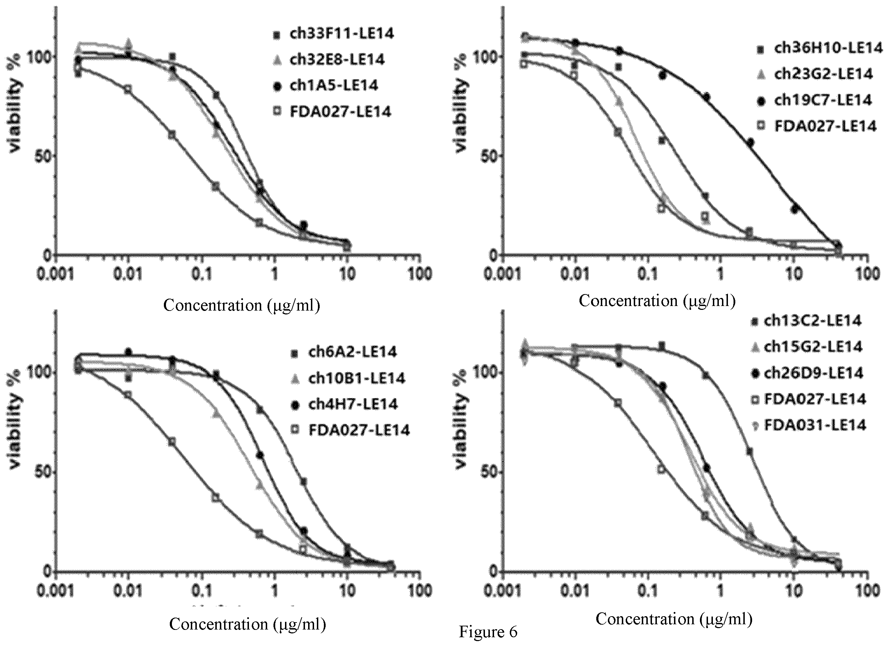

HEK293 cells stably transfected with DLL3 with high expression were selected as cell lines for *in vitro* activity detection, and the dose-response of different antibody drug conjugates on cell killing was observed. 2000 cells per well were inoculated in a 96-well cell culture plate and cultured for 20-24 hours. The antibody drug conjugates prepared according to the method described in Example 11 were diluted to a series of 9 concentration gradient test solutions: 80, 20, 5, 1.25, 0.3125, 0.0781, 0.0195, 0.00488, and 0.000488 µg/ml. 100µl per well of these diluted test solutions were added to the wells comprising the inoculated cells. The plates were then incubated in a 37°C, 5% CO₂ incubator for 144 hours. Afterwards, CellTiter-Glo^{®} Luminescent Cell Viability Assay Reagent (50µl/well) was added, and the plates were shaken at 500 rpm at room temperature for 10 minutes to ensure mixing. The data was read using a SpectraMaxL Microplate reader (OD570nm, with a 2-second interval between readings). The viability of the untreated control cells was considered as 100% for data processing, and the IC₅₀ values were calculated. The results are presented in Figure 6 and Table 7.

**Table 7. Summary of IC₅₀ Values and Maximum Killing Rates of ADCs of the Candidate Antibodies in Killing DLL3/HEK293 Cells in vitro**

| **Sample Name** | **IC₅₀ (µg/mL)** | **Maximum Killing Rate (%)** |
|---|---|---|
| ch1A5-LE00 | 0.321 | 96.3 |
| ch33F11-LE01 | 0.432 | 95.4 |
| ch33F11-LE02 | 0.564 | 97.3 |
| ch33F11-LE03 | 0.375 | 95.2 |
| ch33F11-LE04 | 0.453 | 94.8 |
| ch33F11-LE05 | 0.532 | 96.2 |
| ch33F11-LE06 | 0.383 | 95.7 |
| ch33F11-LE07 | 0.432 | 95.2 |
| ch33F11-LE08 | 0.634 | 96.8 |
| ch33F11-LE09 | 0.563 | 94.3 |
| ch33F11-LE10 | 0.276 | 96.7 |
| ch33F11-LE11 | 0.364 | 93.1 |
| ch1A5-LE12 | 0.412 | 94.8 |
| ch1A5-LE13 | 0.337 | 96.3 |
| ch1A5-LE15 | 0.432 | 95.2 |
| ch1A5-LE16 | 0.386 | 96.5 |
| ch1A5-LE17 | 0.254 | 93.5 |
| ch1A5-LE18 | 0.227 | 95.6 |
| ch1A5-LE19 | 0.521 | 97.2 |
| ch1A5-LE20 | 0.632 | 95.4 |
| ch1A5-LE21 | 0.534 | 92.7 |
| ch1A5-LE22 | 0.432 | 97.1 |
| ch33F11-LE23 | 0.369 | 93.6 |
| ch33F11-LE24 | 0.463 | 96.5 |
| ch36H10-LE14 | 0.695 | 96.9 |
| ch32E8-LE14 | 0.225 | 96.8 |
| ch1A5-LE14 | 0.277 | 96.6 |
| ch19C7-LE14 | 8.69 | 88.4 |
| ch23G2-LE14 | 0.303 | 96.8 |
| ch33F11-LE14 | 0.377 | 98.3 |
| ch6A2-LE14 | 0.992 | 96.2 |
| ch10B1-LE14 | 0.46 | 97.6 |
| ch4H7-LE14 | 0.718 | 96.9 |
| ch13C2-LE14 | 2.72 | 97.9 |
| ch15G2-LE14 | 0.385 | 90.5 |
| ch26D9-LE14 | 0.563 | 95.6 |
| FDA031-LE14 | 0.389 | 95.2 |
| FDA027-LE14 | 0.087¹ | 95.1² |

¹ and ² indicate a mean value.

The results from Table 7 indicate that, with the exception of ch19C7-LE14 and ch13C2-LE14, which showed relatively weaker *in vitro* cytotoxic activity, all ADCs disclosed in the present invention demonstrated significant *in vitro* cell-killing effects on DLL3-positive cells. Among these, FDA027-LE14 exhibited the strongest *in vitro* cytotoxic activity, and also had the most optimal IC₅₀ value in terms of *in vitro* activity. Furthermore, the IC₉₀ values of the various ADCs disclosed in the present disclosure did not show significant differences when compared to FDA027-LE14.

### Example 13. In Vivo Antitumor Activity of ADC Drugs in NCI-H82 Model

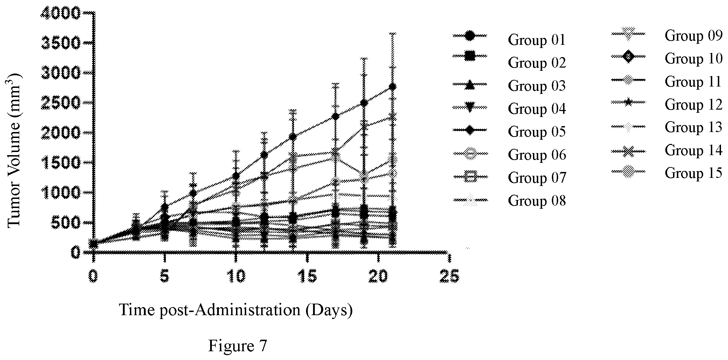

Human small cell lung cancer cells (NCI-H82), suspended in 200µl Matrigel, were subcutaneously injected into the right dorsal side of female Balb/c nude mice (6-8 weeks old, purchased from Shanghai Lingchang Biotechnology Co., Ltd.). When the average tumor volume reached approximately 140mm³, the mice were randomly divided into 15 groups based on tumor size and body weight, with 8 animals in each group of 1-13, which are: vehicle control group; ch1A5-LE14, ch33F11-LE14, ch23G2LE14, ch32E8-LE14, FDA031-LE14, and FDA027-LE14 conjugates at two dosage levels (2.5mg/kg and 5.0mg/kg), administered intravenously once a week for a total of three doses. Groups 14-15, each with 6 animals, served as control groups receiving Lurbinectedin (0.18mg/kg) and Topotecan Hydrochloride (1.8mg/kg), respectively. Group Lurbinectedin was administered intravenously once a week for a total of three doses, while Group Topotecan Hydrochloride was administered intraperitoneally twice a week (continuously on the 1st and 2nd days) for a total of three doses. The body weight and tumor volume of the experimental animals were measured thrice weekly, and their survival status was monitored throughout the experiment. The results, as depicted in Figure 7 and Table 8, showed that on day 21 post-treatment, the average tumor volume in the mice of vehicle control group (Group 01) was 2773mm³. And the average tumor volumes in the FDA027-LE14 treatment groups (Group 12 at 2.5mg/kg and Group 13 at 5mg/kg) were 486mm³ and 305mm³, respectively. The average tumor volume of the ch1A5-LE14 treatment group (Group 02) with a test drug of 2.5 mg/kg on day 21 post-treatment was 607mm³, while the average tumor volume of the ch1A5-LE14 treatment group (Group 03) with a test drug of 5 mg/kg on day 21 post-treatment was 245mm³. The average tumor volume of the ch33F11-LE14 treatment group (Group 04) with a test drug of 2.5 mg/kg on day 21 post-treatment was 715 mm³, while the average tumor volume of the ch33F11-LE14 treatment group (Group 05) with a test drug of 5 mg/kg on day 21 post-treatment was 293mm³. The average tumor volume of the ch23G2-LE14 treatment group (Group 06) with a test drug of 2.5 mg/kg on day 21 post-treatment was 1322 mm³, while the average tumor volume of the ch23G2-LE14 treatment group (Group 07) with a test drug of 5 mg/kg on day 21 post-treatment was 441mm³. The average tumor volume of the ch32E8-LE14 treatment group (Group 08) with a test drug of 2.5 mg/kg on day 21 post-treatment was 945 mm³, while the average tumor volume of the ch32E8-LE14 treatment group (Group 09) with a test drug of 5 mg/kg on day 21 post-treatment was 433mm³. For FDA031-LE14 at 2.5 mg/kg (Group 10), the average tumor volume was 564 mm³, and at 5 mg/kg (Group 11), it was 245 mm³ on Day 21 post-treatment. The control group treated with 0.18 mg/kg Lurbinectedin (sourced from CE Pharm Pharmaceutical Co., Ltd., Lot No. 000039-87-CE013BA-a1) had an average tumor volume of 2268 mm³ on Day 21 post-treatment, and the control group treated with 1.8 mg/kg Topotecan Hydrochloride (from Sain Chemical Technology (Shanghai) Co., Ltd., Lot No. R3URR4E) had an average tumor volume of 1553 mm³ on Day 21 post-treatment. The experimental results indicate that the aforementioned treatment groups, particularly ch1A5-LE14, demonstrated significant *in vivo* antitumor activity. Besides the control group treated with Topotecan Hydrochloride, all experimental mice survived without weight loss, indicating good safety profiles of the test drugs.

**Table 8: In vivo Antitumor Efficacy of ADC Drugs in the NCI-H82 Model**

| **Group** | **Observation Days** | | | | | | | | | |
|---|---|---|---|---|---|---|---|---|---|---|
| | **0** | **3** | **5** | **7** | **10** | **12** | **14** | **17** | **19** | **21** |
| Average Tumor Volume/mm³ | | | | | | | | | | |
| **01** | 143 | 375 | 762 | 993 | 1280 | 1633 | 1932 | 227 7 | 2498 | 277 3 |
| **02** | 143 | 351 | 472 | 503 | 500 | 523 | 540 | 650 | 622 | 607 |
| **03** | 143 | 373 | 395 | 338 | 235 | 228 | 238 | 285 | 266 | 245 |
| **04** | 143 | 409 | 505 | 500 | 530 | 580 | 610 | 724 | 738 | 715 |
| **05** | 143 | 379 | 401 | 376 | 287 | 294 | 264 | 350 | 320 | 293 |
| **06** | 143 | 354 | 506 | 628 | 768 | 784 | 869 | 118 1 | 1220 | 132 2 |
| **07** | 143 | 408 | 411 | 408 | 390 | 398 | 358 | 485 | 450 | 441 |
| **08** | 143 | 398 | 479 | 646 | 752 | 814 | 875 | 979 | 949 | 945 |
| **09** | 143 | 393 | 448 | 433 | 343 | 345 | 334 | 382 | 392 | 433 |
| **10** | 143 | 391 | 506 | 561 | 570 | 521 | 496 | 628 | 594 | 564 |
| **11** | 143 | 393 | 434 | 433 | 361 | 396 | 382 | 311 | 279 | 245 |
| **12** | 143 | 333 | 368 | 393 | 425 | 405 | 386 | 466 | 518 | 486 |
| **13** | 143 | 361 | 448 | 472 | 478 | 496 | 454 | 375 | 304 | 305 |

| **Group** | **Observation Days** | | | | | | | | | |
|---|---|---|---|---|---|---|---|---|---|---|
| | **0** | **3** | **5** | **7** | **9** | **11** | **14** | **16** | **18** | **21** |
| **14** | 144 | 319 | / | 794 | 1049 | 1285 | 1607 | 167 9 | 2100 | 226 8 |
| **15** | 143 | 328 | / | 777 | 1140 | 1276 | 1403 | 158 3 | 1300 | 155 3 |

| **Group** | **Observation Days** | | | | | | | | | |
|---|---|---|---|---|---|---|---|---|---|---|
| | **0** | **3** | **5** | **7** | **10** | **12** | **14** | **17** | **19** | **21** |
| Standard Deviation | | | | | | | | | | |
| **01** | 12 | 52 | 92 | 117 | 92 | 131 | 140 | 193 | 262 | 313 |
| **02** | 12 | 38 | 50 | 58 | 99 | 95 | 115 | 173 | 162 | 148 |
| **03** | 143 | 373 | 395 | 338 | 235 | 228 | 238 | 285 | 266 | 245 |
| **04** | 14 | 44 | 41 | 67 | 71 | 84 | 85 | 112 | 119 | 113 |
| **05** | 13 | 48 | 48 | 40 | 37 | 46 | 34 | 57 | 52 | 55 |
| **06** | 10 | 49 | 80 | 121 | 143 | 163 | 184 | 251 | 263 | 286 |
| **07** | 15 | 66 | 59 | 81 | 106 | 107 | 95 | 116 | 111 | 103 |
| **08** | 11 | 51 | 70 | 122 | 170 | 182 | 222 | 238 | 242 | 251 |
| **09** | 12 | 53 | 53 | 46 | 48 | 45 | 36 | 41 | 42 | 52 |
| **10** | 15 | 54 | 76 | 85 | 110 | 99 | 101 | 139 | 129 | 115 |
| **11** | 16 | 46 | 57 | 102 | 145 | 172 | 167 | 65 | 38 | 32 |
| **12** | 16 | 46 | 43 | 57 | 71 | 76 | 69 | 83 | 81 | 76 |
| **13** | 10 | 42 | 88 | 127 | 169 | 182 | 167 | 93 | 52 | 35 |

| **Group** | **Observation Days** | | | | | | | | | |
|---|---|---|---|---|---|---|---|---|---|---|
| | **0** | **3** | **5** | **7** | **9** | **11** | **14** | **16** | **18** | **21** |
| **14** | 13 | 35 | / | 128 | 147 | 224 | 315 | 313 | 355 | 369 |
| **15** | 8 | 42 | / | 153 | 227 | 252 | 367 | 590 | 518 | 588 |

### Example 14. In Vivo Antitumor Activity of ADC in NCI-H209 Model

Female Balb/c nude mice, aged 6-8 weeks, were subcutaneously injected on the right dorsal side with 1×10⁷ human small cell lung cancer cells (NCI-H209) suspended in 200 µL of Matrigel. When the average tumor volume reached approximately 150 mm³, the mice were randomly divided into seven groups based on tumor size and body weight, with six animals per group. The groups included a vehicle control group, and three dosage groups for both of the conjugates ch1A5-LE14 and FDA027-LE14, specifically at 1.5 mg/kg, 2.5 mg/kg, and 5.0 mg/kg, administered as a single dose. The body weight and tumor volume of the experimental animals were measured thrice weekly, and their survival status was monitored throughout the experiment. The results are presented in Table 9. The vehicle control group (Group 1) had an average tumor volume of 2006 mm³ at the end of treatment. The 1.5 mg/kg FDA027-LE14 treatment group (Group 5), the 2.5 mg/kg FDA027-LE14 group (Group 6), and the 5 mg/kg FDA027-LE14 group (Group 7) all exhibited an average tumor volume of 0 mm³ on Day 21 post-treatment. The 1.5 mg/kg ch1A5-LE14 treatment group (Group 2), the 2.5 mg/kg ch1A5-LE14 group (Group 3), and the 5 mg/kg ch1A5-LE14 group (Group 4) showed an average tumor volume of 0 mm³ on Day 21 post-treatment. The experimental results indicated that both FDA027-LE14 and ch1A5-LE14 demonstrated significant *in vivo* antitumor activity, and all experimental mice survived without weight loss, indicating good safety profiles of the test drugs.

**Table 9: In Vivo Antitumor Activity of ADC Drugs in the NCI-H209 Model.**

| Group | Observation Days | | | | | | | | | |
|---|---|---|---|---|---|---|---|---|---|---|
| | 0 | 3 | 5 | 7 | 9 | 12 | 14 | 16 | 19 | 21 |
| | Average Tumor Volume/mm³ | | | | | | | | | |
| 1 | 158 | 348 | 535 | 809 | 1040 | 1207 | 1367 | 1506 | 1745 | 2006 |
| 2 | 158 | 117 | 39 | 7 | 0 | 0 | 0 | 0 | 0 | 0 |
| 3 | 158 | 109 | 29 | 8 | 0 | 0 | 0 | 0 | 0 | 0 |
| 4 | 157 | 93 | 26 | 0 | 0 | 0 | 0 | 0 | 0 | 0 |
| 5 | 157 | 105 | 30 | 7 | 0 | 0 | 0 | 0 | 0 | 0 |
| 6 | 157 | 103 | 28 | 0 | 0 | 0 | 0 | 0 | 0 | 0 |
| 7 | 158 | 93 | 25 | 0 | 0 | 0 | 0 | 0 | 0 | 0 |

| | Standard Deviation | | | | | | | | | |
|---|---|---|---|---|---|---|---|---|---|---|
| 1 | 6 | 7 | 41 | 77 | 90 | 96 | 106 | 110 | 145 | 139 |
| 2 | 6 | 14 | 6 | 5 | 0 | 0 | 0 | 0 | 0 | 0 |
| 3 | 7 | 13 | 2 | 4 | 0 | 0 | 0 | 0 | 0 | 0 |
| 4 | 5 | 9 | 3 | 0 | 0 | 0 | 0 | 0 | 0 | 0 |
| 5 | 8 | 8 | 2 | 5 | 0 | 0 | 0 | 0 | 0 | 0 |
| 6 | 4 | 9 | 2 | 0 | 0 | 0 | 0 | 0 | 0 | 0 |
| 7 | 6 | 14 | 2 | 0 | 0 | 0 | 0 | 0 | 0 | 0 |

### Example 15. Toxicity Evaluation of ADC Drugs in Mice

Mice of the KM strain were selected for toxicity evaluation. The mice were randomly divided into four groups, each consisting of ten animals, with an equal number of males and females. Each group received a single intravenous injection of 600 µL of the ch1A5-LE14 conjugate at doses of 200 mg/kg, 400 mg/kg, 500 mg/kg, and 640 mg/kg. Toxicity responses to the ch1A5-LE14 conjugate were observed over a period of 14 days post-administration. At 200 mg/kg, one animal exhibited a temporary decrease in body weight on Day 3, which then recovered and increased; other animals showed a growth trend throughout the experiment. Clinical observations showed no abnormalities, except for the 200 mg/kg group, and the mice of other groups displayed transient fur fluffing on the day of administration and recovered quickly. By the end of the 14-day observation period, all animals survived without dying and death, and the results showed that the conjugates of the present invention was well tolerated in mice, and the maximum tolerated dose was up to 640 mg/kg. In another study evaluating the toxicity of the ch1A5-LE14 conjugate in KM mice, the intraperitoneal injection doses were 410 mg/kg, 512 mg/kg, 640 mg/kg, 800 mg/kg, and 1000 mg/kg, respectively. Symptoms observed included fur fluffing, arched backs, reduced activity, huddling, diarrhea, soft stools, and perianal filth. In the 1000 mg/kg group, five out of ten mice died, and in the 800 mg/kg group, one mouse underwent euthanasia after experiencing a body weight decrease exceeding 30% on three consecutive occasions during the 14-day observation period, in accordance with animal welfare guidelines. No deaths were observed in the other groups, further demonstrating the excellent tolerance of the conjugate in mice.

### Example 16. Toxicity Evaluation of ADC Drugs in Rats

SD rats were selected for intravenous injection of the ch1A5-LE14 conjugate to assess tolerance in rats. The rats were randomly divided into three groups, each consisting of eight animals, with an equal number of males and females. One group received 100 mg/kg once a week for three weeks, another group received a single dose of 300 mg/kg, and a blank control group was established. The volume of administration was adjusted to 10 µL/g of rat body weight, and observations were made for 12 days following the last administration. At the end of the 12-day observation period post-last administration, no dying or death incidents occurred in any group. All animals in the high-dose 300 mg/kg group exhibited fur fluffing post-administration, while no abnormalities were observed in the 100 mg/kg group. The body weight in the 300 mg/kg group decreased post-administration but showed a recovery trend from Day 7 onwards. All animals were euthanized for autopsy at the end of the observation period, and no abnormal findings were observed in the organs. The results of the toxicity study in rats further demonstrate that the conjugate of the present disclosure exhibits a very good tolerance level in rats.

Although specific embodiments of the present disclosure have been described, it should be understood by those skilled in the art that these are provided for illustrative purposes only. Various changes or modifications can be made without departing from the principles and essence of the present disclosure. Therefore, the scope of the present disclosure is defined by the attached claims.

## Claims

1. An anti-DLL3 antibody comprising a heavy chain variable region (VH) and a light chain variable region (VL); wherein
the VH comprises complementarity-determining regions (CDRs) as follows: VH CDR1 as shown in amino acid sequences of SEQ ID NO: 9, 19, 29, 39, 59, 69, 79, 89, 99, or 63, VH CDR2 as shown in amino acid sequences of SEQ ID NO: 10, 20, 30, 40, 60, 70, 80, 90, 100, 110, or 83, and VH CDR3 as shown in amino acid sequences of SEQ ID NO: 11, 21, 31, 41, 61, 71, 81, 91, 101, 111, or 93;
the VL comprises CDRs as follows: VL CDR1 as shown in amino acid sequences of SEQ ID NO: 12, 32, 42, 62, 72, 82, 92, 102, 112, or 103, VL CDR2 as shown in GAS, GAT, TTS, NAK, YTS, RAN, WAS, FTS, or NAN, and VL CDR3 as shown in amino acid sequences of SEQ ID NO: 14, 24, 34, 44, 64, 74, 84, 94, 104, 114, or 113.

2. The anti-DLL3 antibody according to claim 1, wherein amino acid sequences of VH CDR1, VH CDR2, and VH CDR3 comprised in the VH are respectively as shown in SEQ ID NO: 9, 10, and 11; or amino acid sequences of VH CDR1, VH CDR2, and VH CDR3 comprised in the VH are respectively as shown in SEQ ID NO: 19, 20, and 21; or amino acid sequences of VH CDR1, VH CDR2, and VH CDR3 comprised in the VH are respectively as shown in SEQ ID NO: 29, 30, and 31; or amino acid sequences of VH CDR1, VH CDR2, and VH CDR3 comprised in the VH are respectively as shown in SEQ ID NO: 39, 40, and 41; or amino acid sequences of VH CDR1, VH CDR2, and VH CDR3 comprised in the VH are respectively as shown in SEQ ID NO: 59, 60, and 61; or amino acid sequences of VH CDR1, VH CDR2, and VH CDR3 comprised in the VH are respectively as shown in SEQ ID NO: 69, 70, and 71; or amino acid sequences of VH CDR1, VH CDR2, and VH CDR3 comprised in the VH are respectively as shown in SEQ ID NO: 79, 80, and 81; or amino acid sequences of VH CDR1, VH CDR2, and VH CDR3 comprised in the VH are respectively as shown in SEQ ID NO: 89, 90, and 91; or amino acid sequences of VH CDR1, VH CDR2, and VH CDR3 comprised in the VH are respectively as shown in SEQ ID NO: 99, 100, and 101; or amino acid sequences of VH CDR1, VH CDR2, and VH CDR3 comprised in the VH are respectively as shown in SEQ ID NO: 89, 110, and 111; or amino acid sequences of VH CDR1, VH CDR2, and VH CDR3 comprised in the VH are respectively as shown in SEQ ID NO: 63, 83, and 93;
amino acid sequences of VL CDR1, VL CDR2, and VL CDR3 comprised in the VL are respectively as shown in SEQ ID NO: 12, GAS, and SEQ ID NO: 14; or amino acid sequences of VL CDR1, VL CDR2, and VL CDR3 comprised in the VL are respectively as shown in SEQ ID NO: 12, GAS, and SEQ ID NO: 24; or amino acid sequences of VL CDR1, VL CDR2, and VL CDR3 comprised in the VL are respectively as shown in SEQ ID NO: 32, GAT, and SEQ ID NO: 34; or amino acid sequences of VL CDR1, VL CDR2, and VL CDR3 comprised in the VL are respectively as shown in SEQ ID NO: 42, TTS, and SEQ ID NO: 44; or amino acid sequences of VL CDR1, VL CDR2, and VL CDR3 comprised in the VL are respectively as shown in SEQ ID NO: 62, NAK, and SEQ ID NO: 64; or amino acid sequences of VL CDR1, VL CDR2, and VL CDR3 comprised in the VL are respectively as shown in SEQ ID NO: 72, NAK, and SEQ ID NO: 74; or amino acid sequences of VL CDR1, VL CDR2, and VL CDR3 comprised in the VL are respectively as shown in SEQ ID NO: 82, YTS, and SEQ ID NO: 84; or amino acid sequences of VL CDR1, VL CDR2, and VL CDR3 comprised in the VL are respectively as shown in SEQ ID NO: 92, RAN, and SEQ ID NO: 94; or amino acid sequences of VL CDR1, VL CDR2, and VL CDR3 comprised in the VL are respectively as shown in SEQ ID NO: 102, WAS, and SEQ ID NO: 104; or amino acid sequences of VL CDR1, VL CDR2, and VL CDR3 comprised in the VL are respectively as shown in SEQ ID NO: 112, FTS, and SEQ ID NO: 114; or amino acid sequences of VL CDR1, VL CDR2, and VL CDR3 comprised in the VL are respectively as shown in SEQ ID NO: 103, NAN, and 113.

3. The anti-DLL3 antibody according to claim 1 or 2, wherein amino acid sequences of VH CDR1, VH CDR2, and VH CDR3 comprised in the VH are respectively as shown in SEQ ID NO: 9, 10, and 11; and amino acid sequences of VL CDR1, VL CDR2, and VL CDR3 comprised in the VL are respectively as shown in SEQ ID NO: 12, GAS, and SEQ ID NO: 14; or
amino acid sequences of VH CDR1, VH CDR2, and VH CDR3 comprised in the VH are respectively as shown in SEQ ID NO: 19, 20, and 21; and amino acid sequences of VL CDR1, VL CDR2, and VL CDR3 comprised in the VL are respectively as shown in SEQ ID NO: 12, GAS, and SEQ ID NO: 24; or
amino acid sequences of VH CDR1, VH CDR2, and VH CDR3 comprised in the VH are respectively as shown in SEQ ID NO: 29, 30, and 31; and amino acid sequences of VL CDR1, VL CDR2, and VL CDR3 comprised in the VL are respectively as shown in SEQ ID NO: 32, GAT, and SEQ ID NO: 34; or
amino acid sequences of VH CDR1, VH CDR2, and VH CDR3 comprised in the VH are respectively as shown in SEQ ID NO: 39, 40, and 41; and amino acid sequences of VL CDR1, VL CDR2, and VL CDR3 comprised in the VL are respectively as shown in SEQ ID NO: 42, TTS, and SEQ ID NO: 44; or
amino acid sequences of VH CDR1, VH CDR2, and VH CDR3 comprised in the VH are respectively as shown in SEQ ID NO: 59, 60, and 61; and amino acid sequences of VL CDR1, VL CDR2, and VL CDR3 comprised in the VL are respectively as shown in SEQ ID NO: 62, NAK, and SEQ ID NO: 64; or
amino acid sequences of VH CDR1, VH CDR2, and VH CDR3 comprised in the VH are respectively as shown in SEQ ID NO: 69, 70, and 71; and amino acid sequences of VL CDR1, VL CDR2, and VL CDR3 comprised in the VL are respectively as shown in SEQ ID NO: 72, NAK, and SEQ ID NO: 74; or
amino acid sequences of VH CDR1, VH CDR2, and VH CDR3 comprised in the VH are respectively as shown in SEQ ID NO: 79, 80, and 81; and amino acid sequences of VL CDR1, VL CDR2, and VL CDR3 comprised in the VL are respectively as shown in SEQ ID NO: 82, YTS, and SEQ ID NO: 84; or
amino acid sequences of VH CDR1, VH CDR2, and VH CDR3 comprised in the VH are respectively as shown in SEQ ID NO: 89, 90, and 91; and amino acid sequences of VL CDR1, VL CDR2, and VL CDR3 comprised in the VL are respectively as shown in SEQ ID NO: 92, RAN, and SEQ ID NO: 94; or
amino acid sequences of VH CDR1, VH CDR2, and VH CDR3 comprised in the VH are respectively as shown in SEQ ID NO: 99, 100, and 101; and amino acid sequences of VL CDR1, VL CDR2, and VL CDR3 comprised in the VL are respectively as shown in SEQ ID NO: 102, WAS, and SEQ ID NO: 104; or
amino acid sequences of VH CDR1, VH CDR2, and VH CDR3 comprised in the VH are respectively as shown in SEQ ID NO: 89, 110, and 111; and amino acid sequences of VL CDR1, VL CDR2, and VL CDR3 comprised in the VL are respectively as shown in SEQ ID NO: 112, FTS, and SEQ ID NO: 114; or
amino acid sequences of VH CDR1, VH CDR2, and VH CDR3 comprised in the VH are respectively as shown in SEQ ID NO: 63, 83, and 93; and amino acid sequences of VL CDR1, VL CDR2, and VL CDR3 comprised in the VL are respectively as shown in SEQ ID NO: 103, NAN, and 113.

4. The anti-DLL3 antibody according to any one of claims 1 to 3, wherein the heavy chain variable region (VH) further comprises a heavy chain variable region framework region (VH FWR), and/or the light chain variable region (VL) further comprises a light chain variable region framework region (VL FWR); wherein the VH FWR is the heavy chain variable region framework region of a human or murine antibody, and the VL FWR is the light chain variable region framework region of a human or murine antibody;
preferably:
the VH comprises the amino acid sequence as shown in SEQ ID NO: 15, 25, 35, 45, 65, 75, 85, 95, 105, 13, or 22, and/or the VL comprises the amino acid sequence as shown in SEQ ID NO: 16, 26, 36, 46, 66, 76, 86, 96, 106, 33, or 23;
more preferably:
the VH comprises the amino acid sequence as shown in SEQ ID NO: 15, and the VL comprises the amino acid sequence as shown in SEQ ID NO: 16; or
the VH comprises the amino acid sequence as shown in SEQ ID NO: 25, and the VL comprises the amino acid sequence as shown in SEQ ID NO: 26; or
the VH comprises the amino acid sequence as shown in SEQ ID NO: 35, and the VL comprises the amino acid sequence as shown in SEQ ID NO: 36; or
the VH comprises the amino acid sequence as shown in SEQ ID NO: 45, and the VL comprises the amino acid sequence as shown in SEQ ID NO: 46; or
the VH comprises the amino acid sequence as shown in SEQ ID NO: 65, and the VL comprises the amino acid sequence as shown in SEQ ID NO: 66; or
the VH comprises the amino acid sequence as shown in SEQ ID NO: 75, and the VL comprises the amino acid sequence as shown in SEQ ID NO: 76; or
the VH comprises the amino acid sequence as shown in SEQ ID NO: 85, and the VL comprises the amino acid sequence as shown in SEQ ID NO: 86; or
the VH comprises the amino acid sequence as shown in SEQ ID NO: 95, and the VL comprises the amino acid sequence as shown in SEQ ID NO: 96; or
the VH comprises the amino acid sequence as shown in SEQ ID NO: 105, and the VL comprises the amino acid sequence as shown in SEQ ID NO: 106; or
the VH comprises the amino acid sequence as shown in SEQ ID NO: 13, and the VL comprises the amino acid sequence as shown in SEQ ID NO: 33; or
the VH comprises the amino acid sequence as shown in SEQ ID NO: 22, and the VL comprises the amino acid sequence as shown in SEQ ID NO: 23.

5. The anti-DLL3 antibody according to any one of claims 1 to 4, wherein the anti-DLL3 antibody is a full-length antibody, Fab, Fab', F(ab')2, Fv, preferably scFv, a heavy chain antibody, or a single-domain antibody, and/or the anti-DLL3 antibody is a monoclonal antibody, a bispecific antibody, or a multispecific antibody.

6. The anti-DLL3 antibody according to claim 5, wherein the anti-DLL3 antibody is a full-length antibody, and the full-length antibody comprises a heavy chain and a light chain; wherein the heavy chain comprises the amino acid sequence as shown in SEQ ID NO: 17, 27, 37, 47, 67, 77, 87, 97, 107, 43, or 73, and/or the light chain comprises the amino acid sequence as shown in SEQ ID NO: 18, 28, 38, 48, 68, 78, 88, 98, 108, 53, or 109;
preferably:
the heavy chain comprises the amino acid sequence as shown in SEQ ID NO: 17, and the light chain comprises the amino acid sequence as shown in SEQ ID NO: 18; or
the heavy chain comprises the amino acid sequence as shown in SEQ ID NO: 27, and the light chain comprises the amino acid sequence as shown in SEQ ID NO: 28; or
the heavy chain comprises the amino acid sequence as shown in SEQ ID NO: 37, and the light chain comprises the amino acid sequence as shown in SEQ ID NO: 38; or
the heavy chain comprises the amino acid sequence as shown in SEQ ID NO: 47, and the light chain comprises the amino acid sequence as shown in SEQ ID NO: 48; or
the heavy chain comprises the amino acid sequence as shown in SEQ ID NO: 67, and the light chain comprises the amino acid sequence as shown in SEQ ID NO: 68; or
the heavy chain comprises the amino acid sequence as shown in SEQ ID NO: 77, and the light chain comprises the amino acid sequence as shown in SEQ ID NO: 78; or
the heavy chain comprises the amino acid sequence as shown in SEQ ID NO: 87, and the light chain comprises the amino acid sequence as shown in SEQ ID NO: 88; or
the heavy chain comprises the amino acid sequence as shown in SEQ ID NO: 97, and the light chain comprises the amino acid sequence as shown in SEQ ID NO: 98; or
the heavy chain comprises the amino acid sequence as shown in SEQ ID NO: 107, and the light chain comprises the amino acid sequence as shown in SEQ ID NO: 108; or
the heavy chain comprises the amino acid sequence as shown in SEQ ID NO: 43, and the light chain comprises the amino acid sequence as shown in SEQ ID NO: 53; or
the heavy chain comprises the amino acid sequence as shown in SEQ ID NO: 73, and the light chain comprises the amino acid sequence as shown in SEQ ID NO: 109.

7. An isolated nucleic acid encoding the anti-DLL3 antibody according to any one of claims 1 to 6.

8. A recombinant expression vector comprising the isolated nucleic acid according to claim 7;
preferably, the expression vector comprises a eukaryotic cell expression vector and/or a prokaryotic cell expression vector.

9. A transformant comprising the recombinant expression vector according to claim 8; preferably, the host cell of the transformant is a prokaryotic and/or a eukaryotic cell, wherein the prokaryotic cell is preferably *E. coli* cell such as TG1 or BL21 cell, and the eukaryotic cell is preferably HEK293 cell or CHO cell.

10. An antibody-drug conjugate with a general formula: Ab-(L₃-L₂-L₁-D)ₘ;
wherein Ab is an anti-DLL3 antibody;
D is a cytotoxic drug 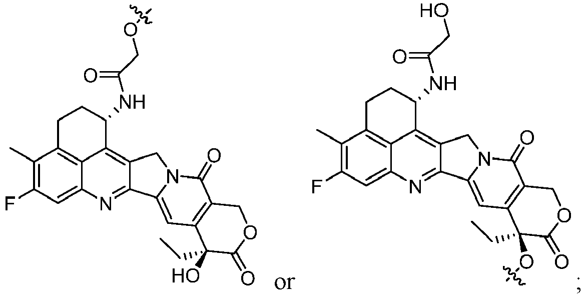
m is 2 to 8;
the structure of L1 is as shown in formulas I, II, III, or IV, with a-terminal connected to the cytotoxic drug, and e-terminal connected to c-terminal of the L₂; 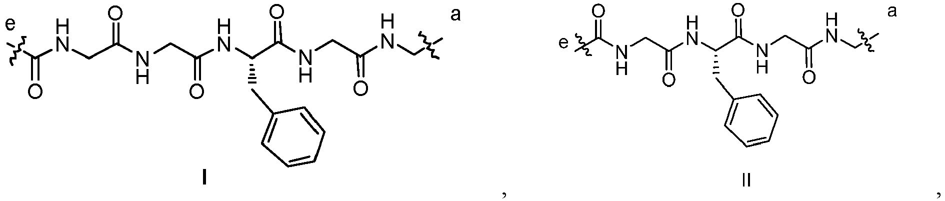 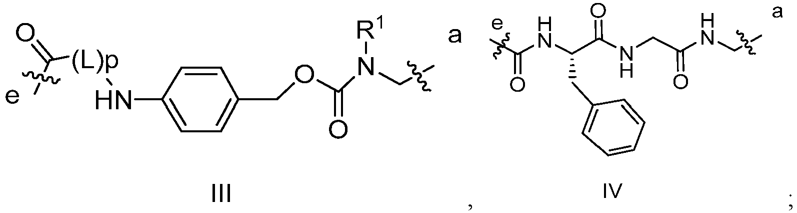
in (L)ₚ, L independently represents one or more of phenylalanine residue, alanine residue, glycine residue, glutamic acid residue, aspartic acid residue, cysteine residue, glutamine residue, histidine residue, isoleucine residue, leucine residue, lysine residue, methionine residue, proline residue, serine residue, threonine residue, tryptophan residue, tyrosine residue, and valine residue; "p" is from 2 to 4.
R¹ is C₁-C₆ alkyl substituted by one or more -NR¹⁻¹R¹⁻², C₁-C₆ alkyl substituted by one or more R¹⁻³S(O)₂-, C₁-C₆ alkyl, C₃-C₁₀ cycloalkyl, C₆-C₁₄ aryl, or 5- to 14-membered heteroaryl; the heteroatom in the 5- to 14-membered heteroaryl is selected from one or more of N, O, and S, and the number of the heteroatom is 1, 2, 3, or 4; the R¹⁻¹, R¹⁻², and R¹⁻³ are each independently C₁-C₆ alkyl;
L₂ is 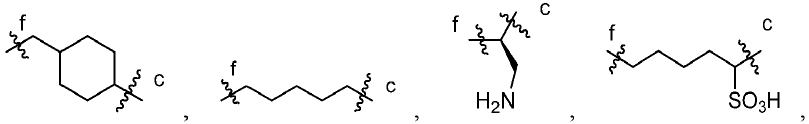 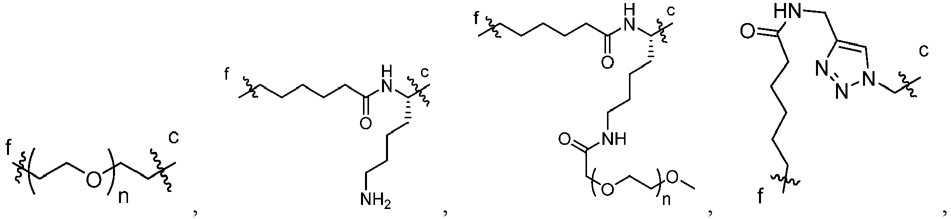 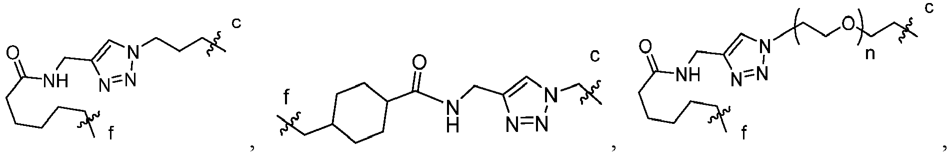 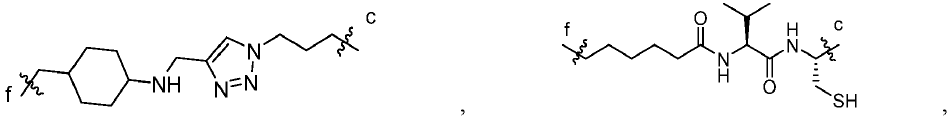 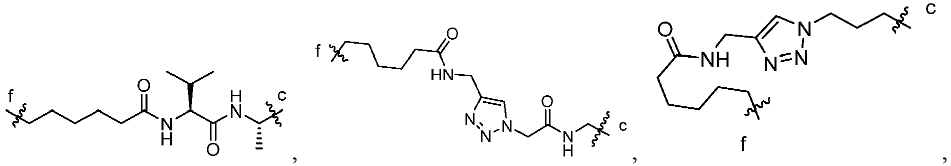 or 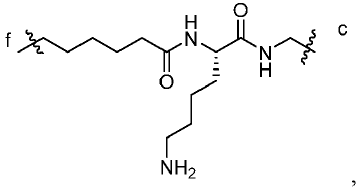 wherein n is independently 1 to 12, and c-terminal is connected to L₁ through a carbonyl group, and f-terminal is connected to d-terminal of L₃;
L3 is 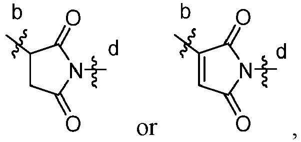 wherein b-terminal is connected to the Ab, and d-terminal is connected to f-terminal of L₂.

11. The antibody-drug conjugate according to claim 10, wherein
the anti-DLL3 antibody binds to one or more of following antigen-binding epitopes of DLL3 protein: DSL domain, N-terminal, EGF2 domain, and EGF3-EGF6 domain;
and/or L is one or more of phenylalanine residue, alanine residue, glycine residue, isoleucine residue, leucine residue, proline residue, and valine residue; preferably one or more of phenylalanine residue, alanine residue, glycine residue, and valine residue; further preferably, the L is valine residue and/or alanine residue; the "more" means two or three; p is 2;
and/or the R¹ is C₁-C₆ alkyl substituted by one or more -NR¹⁻¹R¹⁻², C₁-C₆ alkyl substituted by one or more R¹⁻³S(O)₂-, or C₁-C₆ alkyl; the R¹⁻¹, R¹⁻², and R¹⁻³ are each independently C₁-C₄;
and/or when the structure of L₁ is as shown in formula I, the L₂ is preferably 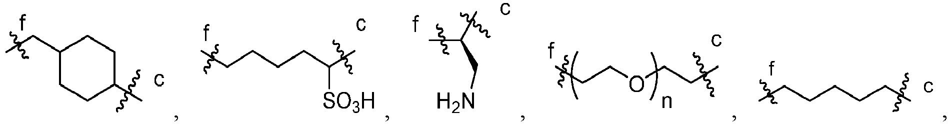 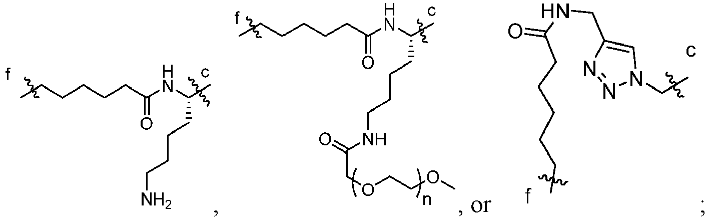
and/or when the structure of L₁ is as shown in formula II, the L₂ is preferably 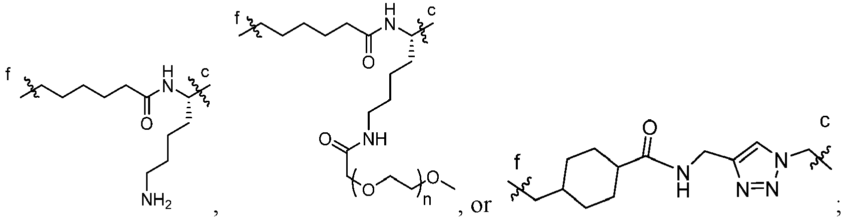
and/or when the structure of L₁ is as shown in formula III, the L₂ is preferably 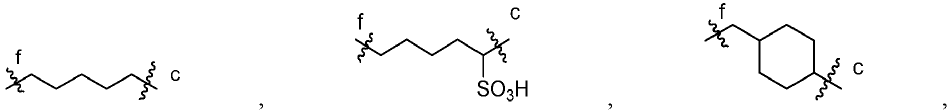 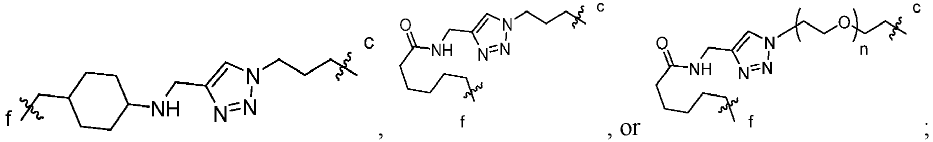
and/or when the structure of L₁ is as shown in formula IV, the L₂ is preferably 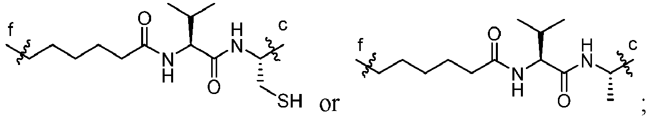
and/or the n is independently 8, 9, 10, 11, and 12;
and/or the m is an integer or non-integer from 2 to 8;
and/or the L₃ is preferably

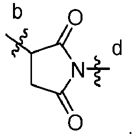

12. The antibody-drug conjugate according to claim 10 or 11, wherein
the anti-DLL3 antibody binds to the antigen-binding epitope in the EGF2 domain of DLL3 protein;
and/or the (L)p is 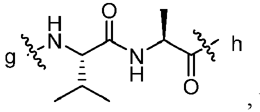 wherein g-terminal is connected to c-terminal of L₂ through a carbonyl group;
and/or the formula III is preferably 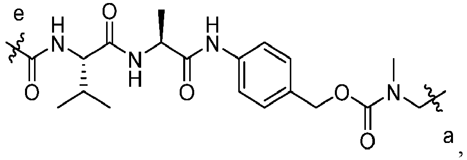 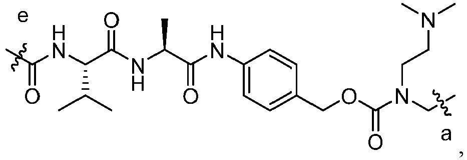 or

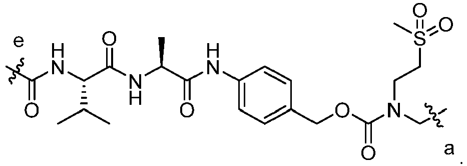

13. The antibody-drug conjugate according to claim 10, wherein the antibody-drug conjugate is any one of the following compounds: 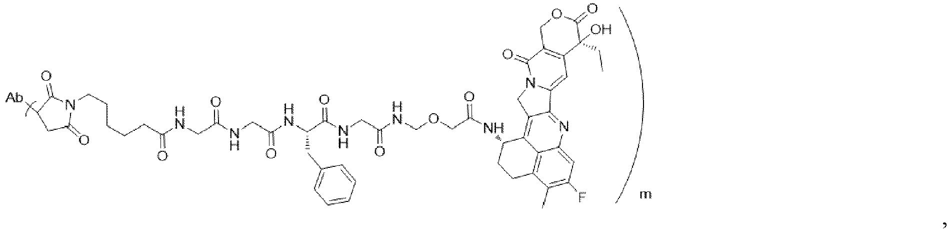 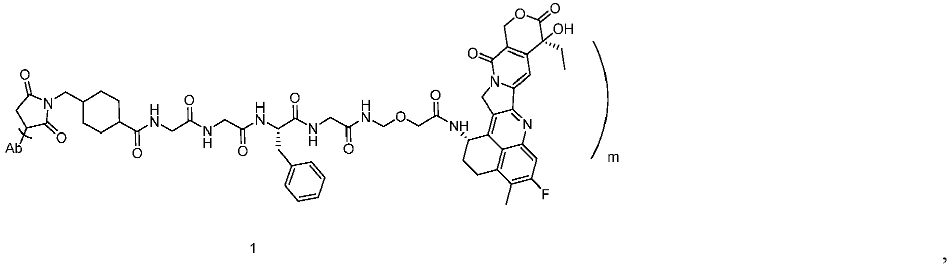 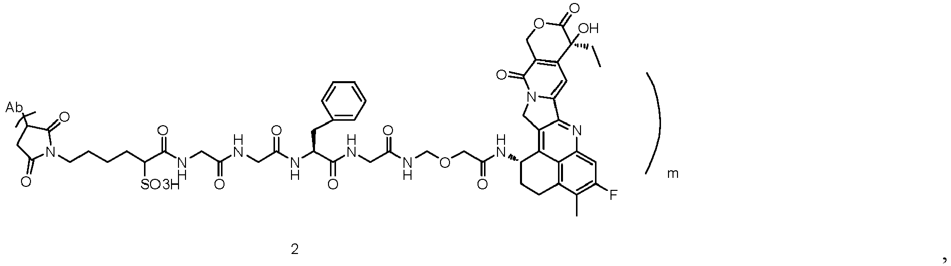 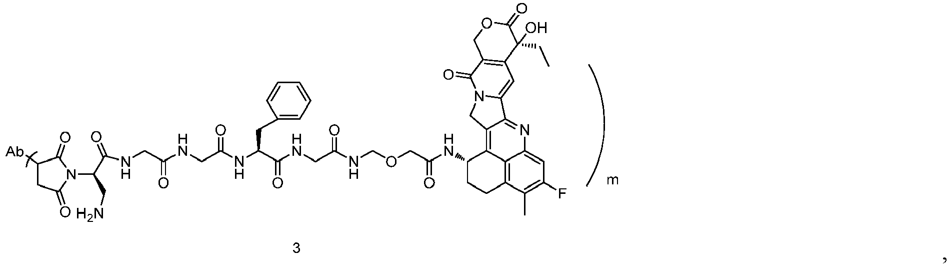 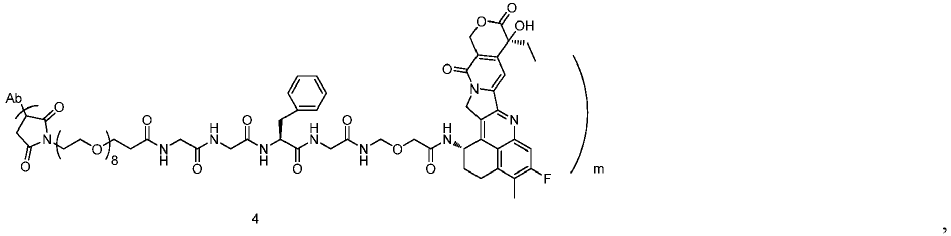 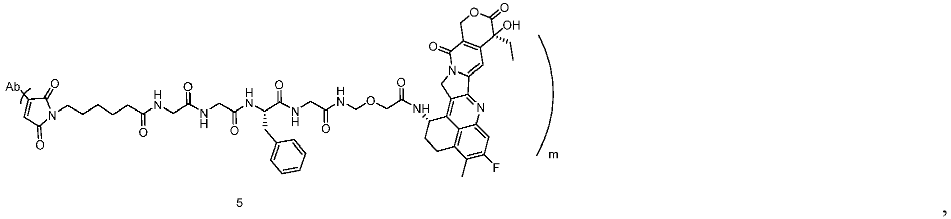 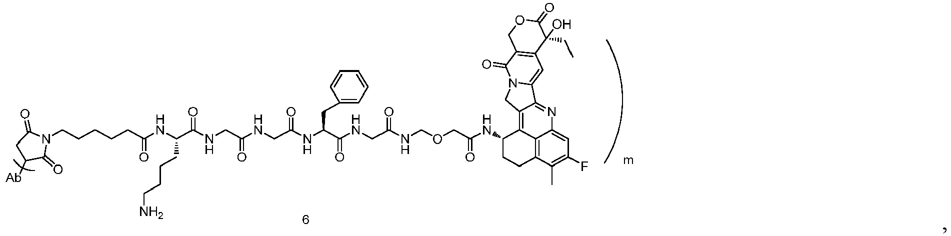 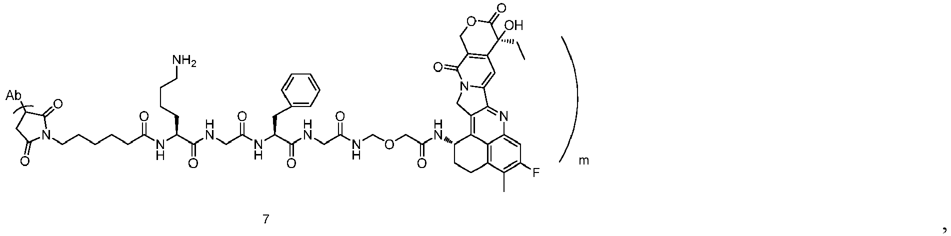 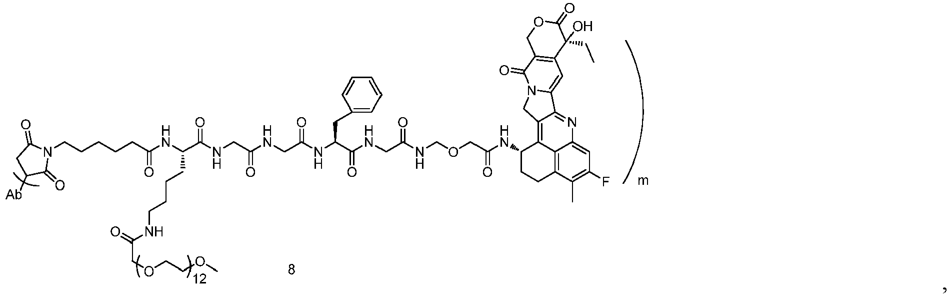 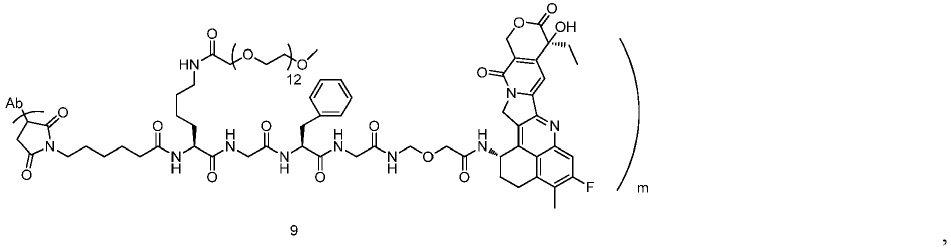 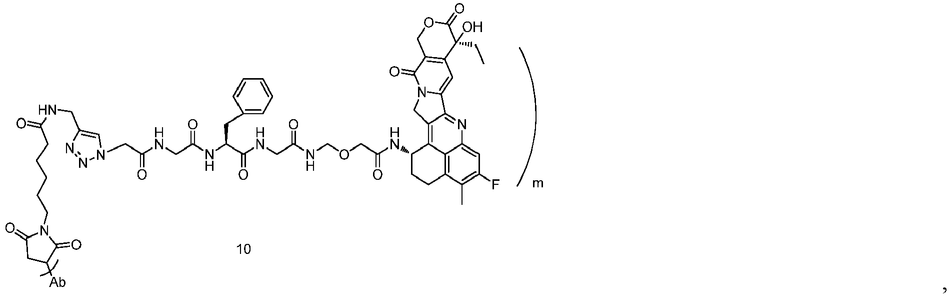 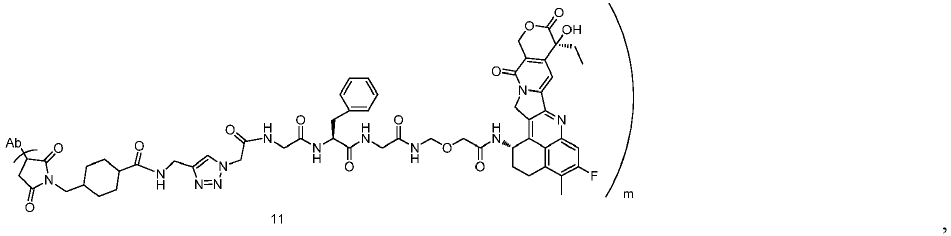 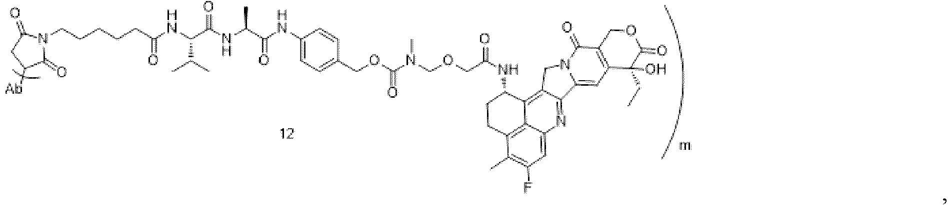 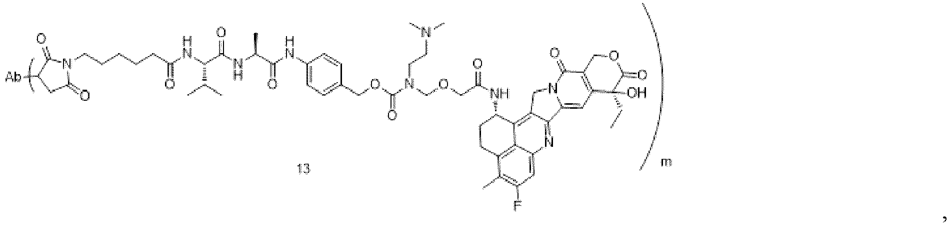 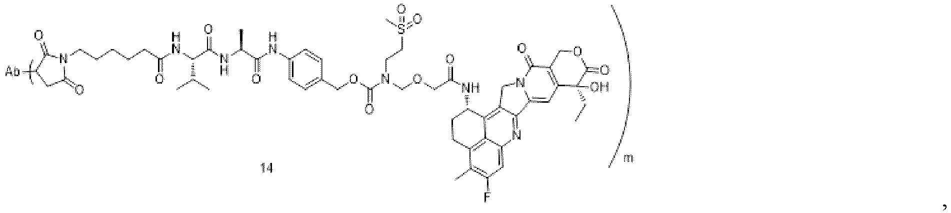 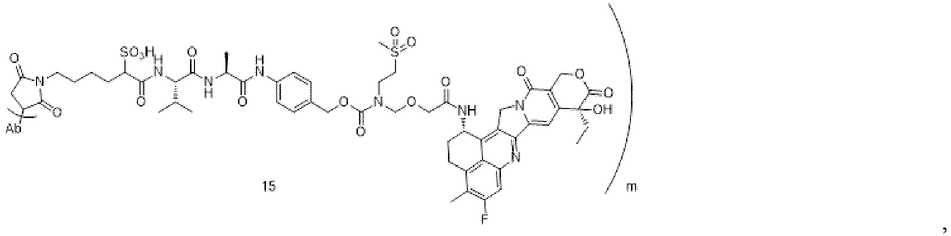 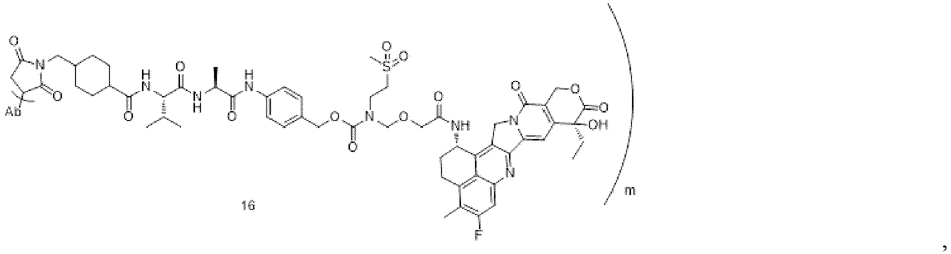 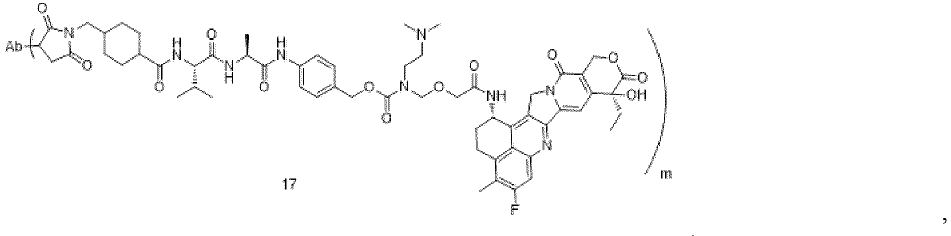 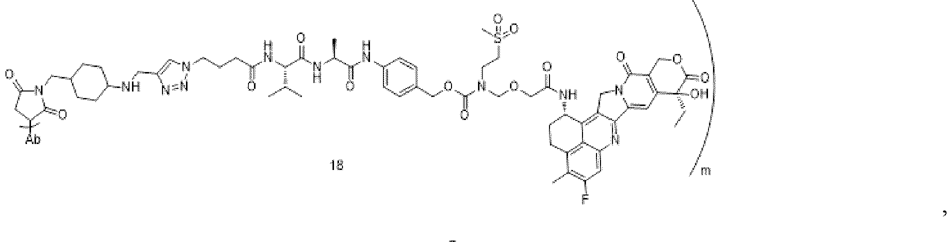 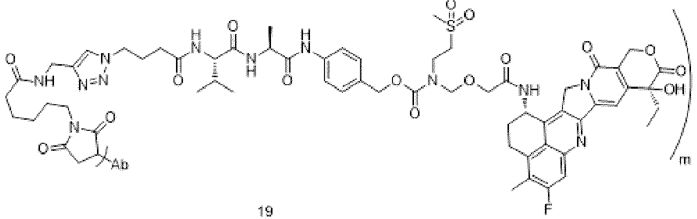 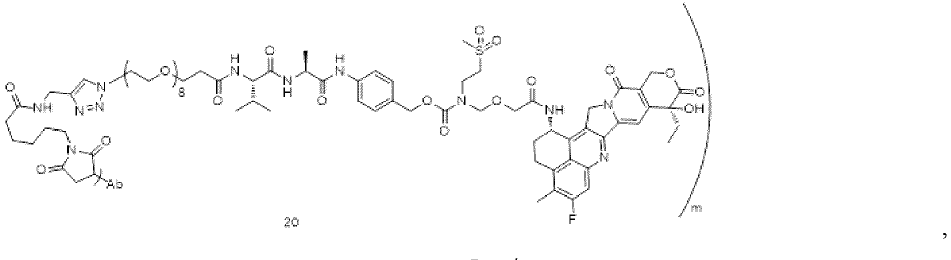 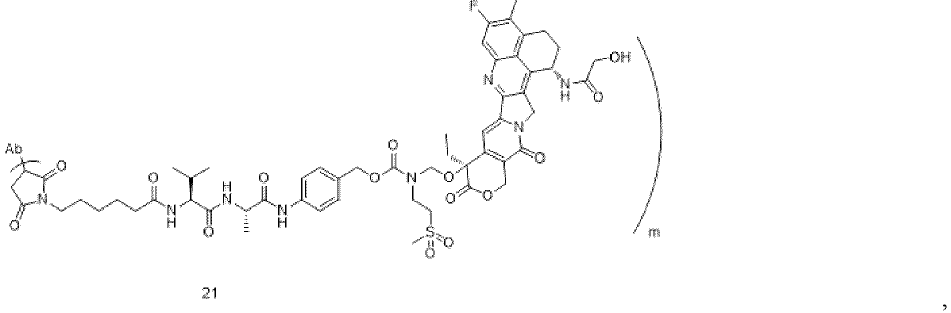 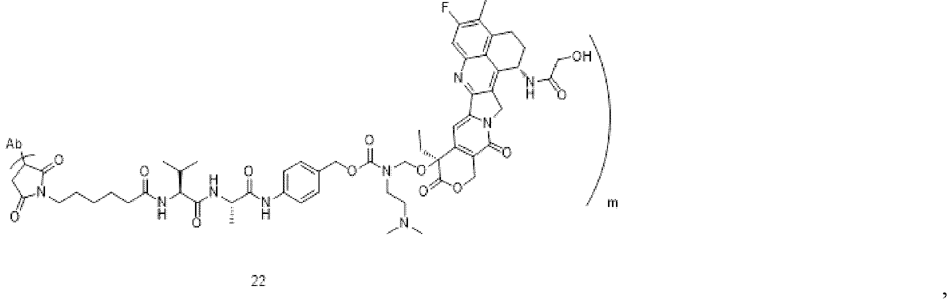 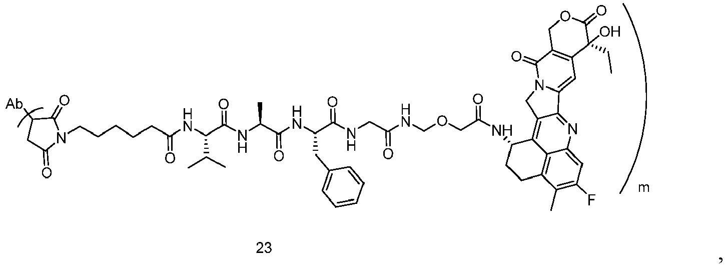 o r 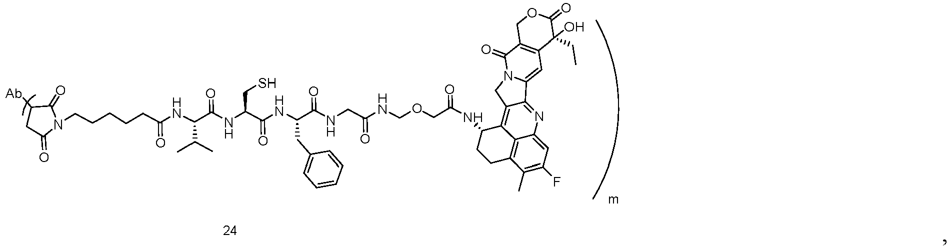
the Ab is an anti-DLL3 antibody, wherein the anti-DLL3 antibody is the anti-DLL3 antibody according to any one of claims 1 to 6, the anti-DLL3 antibody comprising a light chain with an amino acid sequence as shown in SEQ ID NO: 1 and a heavy chain with an amino acid sequence as shown in SEQ ID NO: 2, or comprising a light chain with an amino acid sequence as shown in SEQ ID NO: 3 and a heavy chain with an amino acid sequence as shown in SEQ ID NO: 4;
the m is 7.68, 7.53, 4.43, 7.12, 6.92, 7.43, 7.23, 6.83, 7.32, 7.56, 7.54, 7.47, 5.82, 6.78, 2.28, 6.32, 7.45, 7.65, 7.64, 7.36, 7.75, 7.80, 7.77, or 7.76;
preferably, the antibody-drug conjugate is any one of the following compounds: 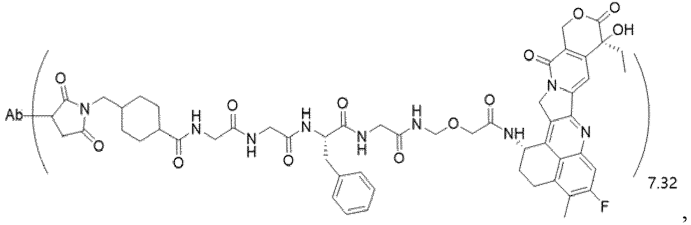 wherein Ab is the anti-DLL3 antibody comprising a VH with an amino acid sequence as shown in SEQ ID NO: 25 and a VL with an amino acid sequence as shown in SEQ ID NO: 26; 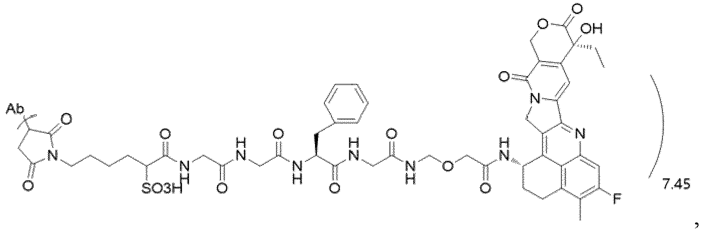 wherein Ab is the anti-DLL3 antibody comprising a VH with an amino acid sequence as shown in SEQ ID NO: 25 for VH and an amino acid sequence as shown in SEQ ID NO: 26; 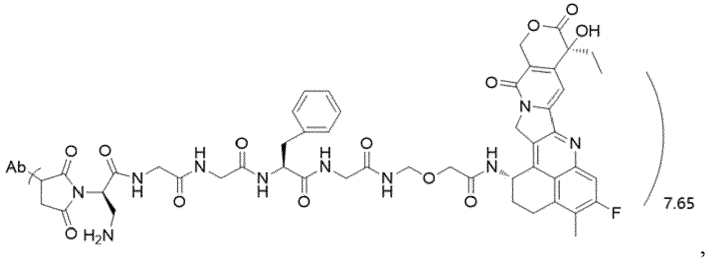 wherein Ab is the anti-DLL3 antibody comprising a VH with an amino acid sequence as shown in SEQ ID NO: 25 and a VL with an amino acid sequence as shown in SEQ ID NO: 26; 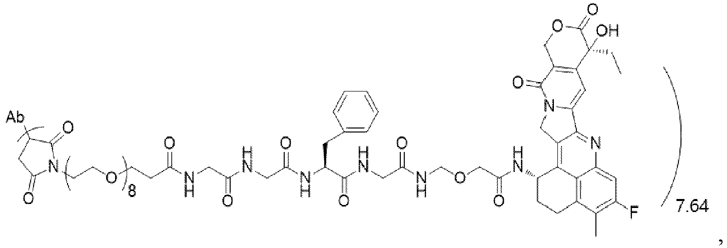 wherein Ab is the anti-DLL3 antibody comprising a VH with an amino acid sequence as shown in SEQ ID NO: 25 and a VL with an amino acid sequence as shown in SEQ ID NO: 26; 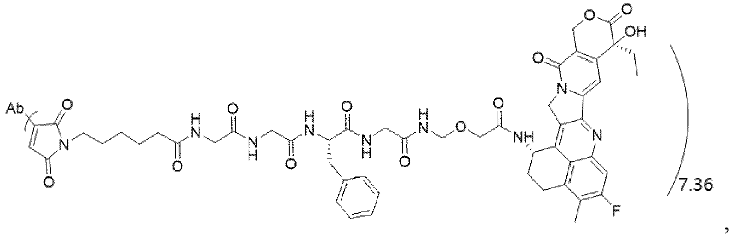 wherein Ab is the anti-DLL3 antibody comprising a VH with an amino acid sequence as shown in SEQ ID NO: 25 and a VL with an amino acid sequence as shown in SEQ ID NO: 26; 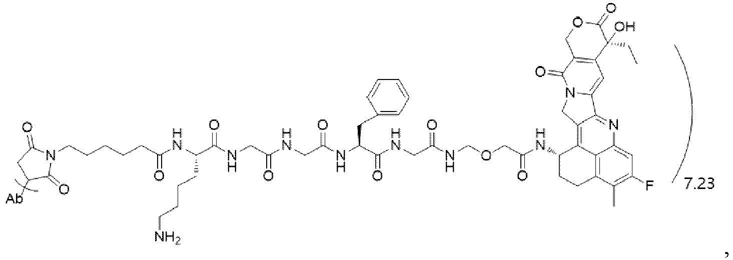 wherein Ab is the anti-DLL3 antibody comprising a VH with an amino acid sequence as shown in SEQ ID NO: 25 and a VL with an amino acid sequence as shown in SEQ ID NO: 26; 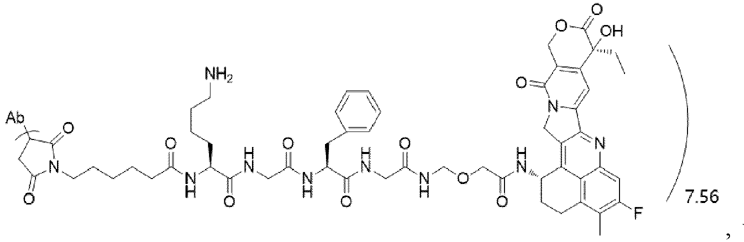 wherein Ab is the anti-DLL3 antibody comprising a VH with an amino acid sequence as shown in SEQ ID NO: 25 and a VL with an amino acid sequence as shown in SEQ ID NO: 26; 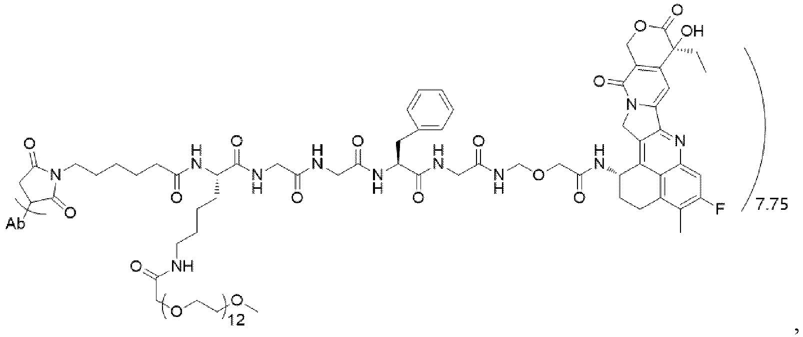 wherein Ab is the anti-DLL3 antibody comprising a VH with an amino acid sequence as shown in SEQ ID NO: 25 and a VL with an amino acid sequence as shown in SEQ ID NO: 26; 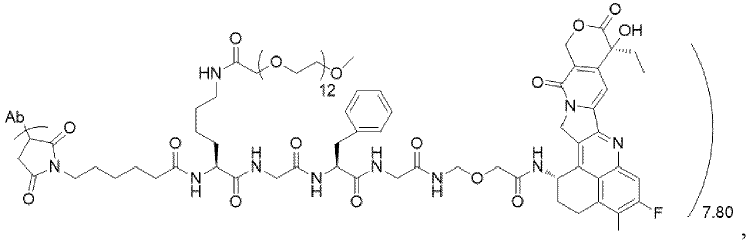 wherein Ab is the anti-DLL3 antibody comprising a VH with an amino acid sequence as shown in SEQ ID NO: 25 and a VL with an amino acid sequence as shown in SEQ ID NO: 26; 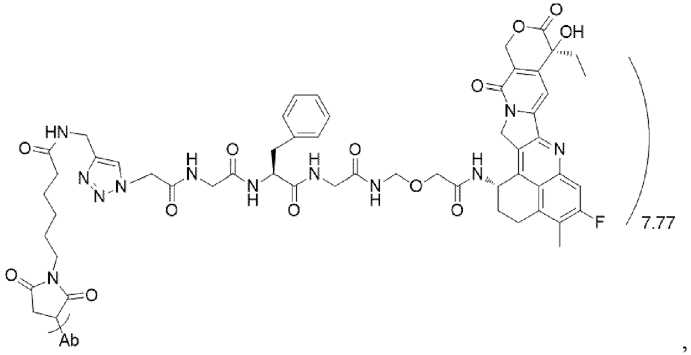 wherein Ab is the anti-DLL3 antibody comprising a VH with an amino acid sequence as shown in SEQ ID NO: 25 and a VL with an amino acid sequence as shown in SEQ ID NO: 26; 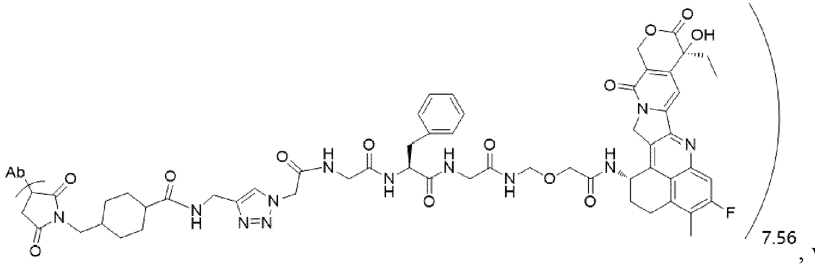 wherein Ab is the anti-DLL3 antibody comprising a VH with an amino acid sequence as shown in SEQ ID NO: 25 and a VL with an amino acid sequence as shown in SEQ ID NO: 26; 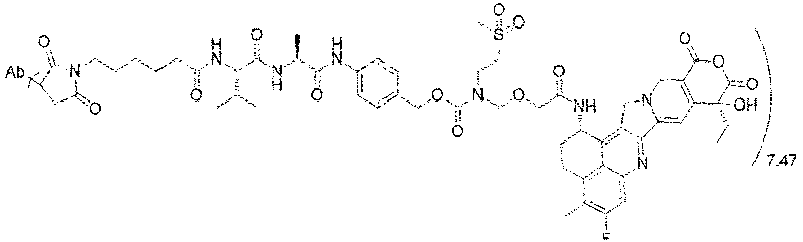 , wherein Ab is the anti-DLL3 antibody comprising a VH with an amino acid sequence as shown in SEQ ID NO: 25 and a VL with an amino acid sequence as shown in SEQ ID NO: 26; 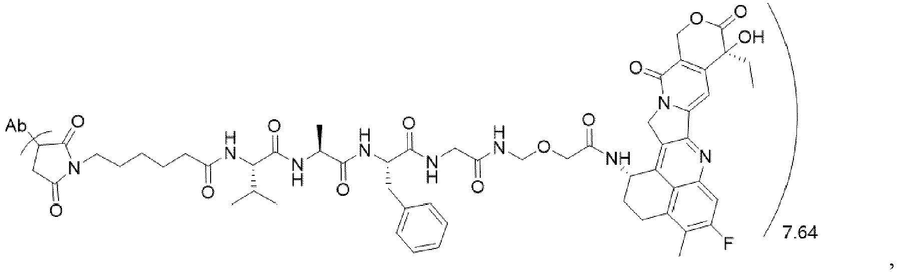 wherein Ab is the anti-DLL3 antibody comprising a VH with an amino acid sequence as shown in SEQ ID NO: 25 and a VL with an amino acid sequence as shown in SEQ ID NO: 26; 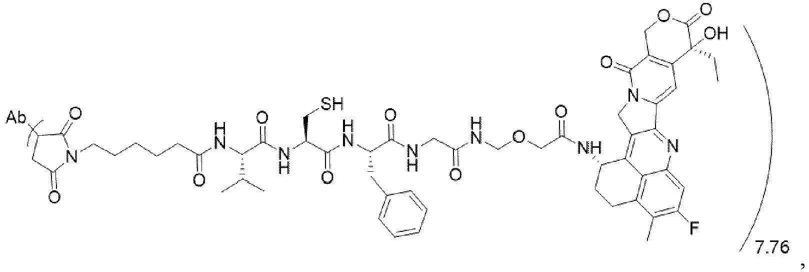 wherein Ab is the anti-DLL3 antibody comprising a VH with an amino acid sequence as shown in SEQ ID NO: 25 and a VL with an amino acid sequence as shown in SEQ ID NO: 26; 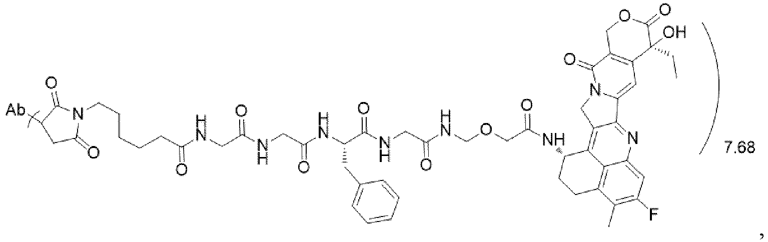 wherein Ab is the anti-DLL3 antibody comprising a VH with an amino acid sequence as shown in SEQ ID NO: 15 and a VL with an amino acid sequence as shown in SEQ ID NO: 16; 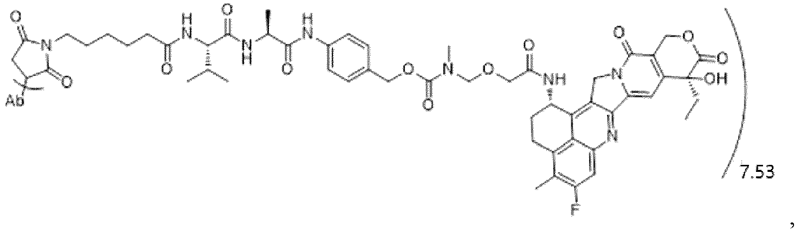 wherein Ab is the anti-DLL3 antibody comprising a VH with an amino acid sequence as shown in SEQ ID NO: 15 and a VL with an amino acid sequence as shown in SEQ ID NO: 16; 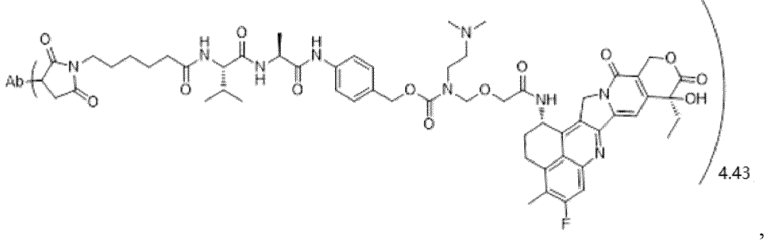 wherein Ab is the anti-DLL3 antibody comprising a VH with an amino acid sequence as shown in SEQ ID NO: 15 and a VL with an amino acid sequence as shown in SEQ ID NO: 16; 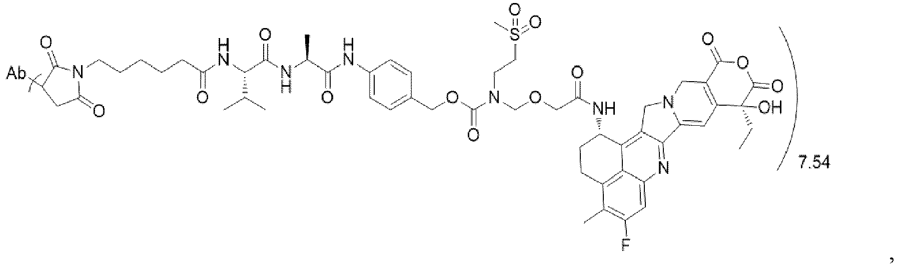 wherein Ab is the anti-DLL3 antibody comprising a VH with an amino acid sequence as shown in SEQ ID NO: 15 and a VL with an amino acid sequence as shown in SEQ ID NO: 16; 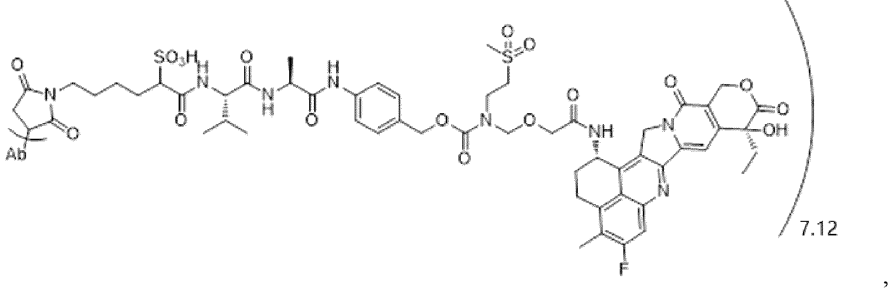 wherein Ab is the anti-DLL3 antibody comprising a VH with an amino acid sequence as shown in SEQ ID NO: 15 and a VL with an amino acid sequence as shown in SEQ ID NO: 16; 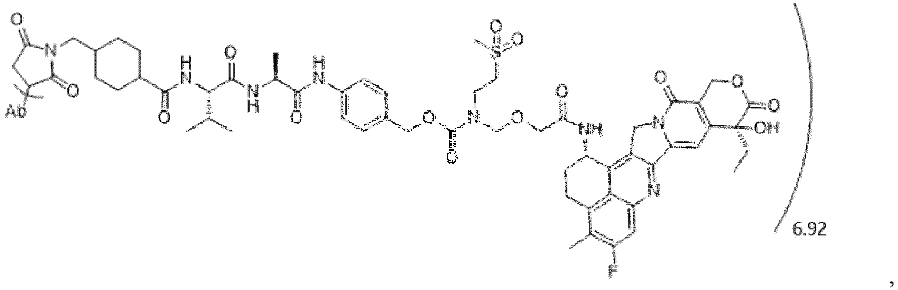 wherein Ab is the anti-DLL3 antibody comprising a VH with an amino acid sequence as shown in SEQ ID NO: 15 and a VL with an amino acid sequence as shown in SEQ ID NO: 16; 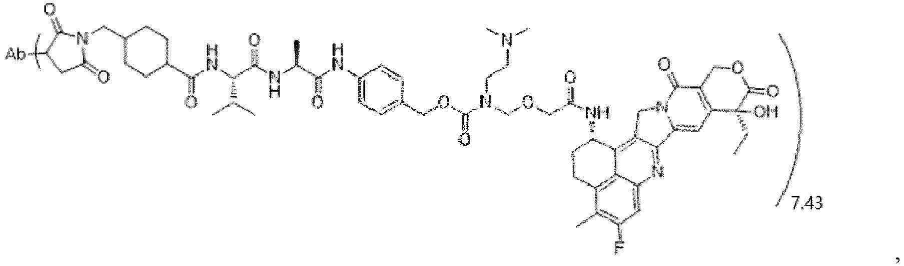 wherein Ab is the anti-DLL3 antibody comprising a VH with an amino acid sequence as shown in SEQ ID NO: 15 and a VL with an amino acid sequence as shown in SEQ ID NO: 16; 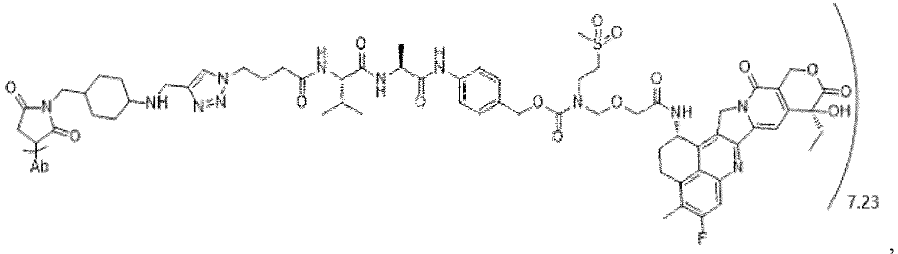 wherein Ab is the anti-DLL3 antibody comprising a VH with an amino acid sequence as shown in SEQ ID NO: 15 and a VL with an amino acid sequence as shown in SEQ ID NO: 16; 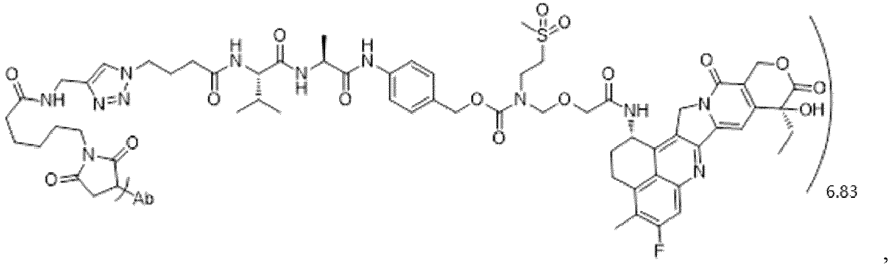 wherein Ab is the anti-DLL3 antibody comprising a VH with an amino acid sequence as shown in SEQ ID NO: 15 and a VL with an amino acid sequence as shown in SEQ ID NO: 16; 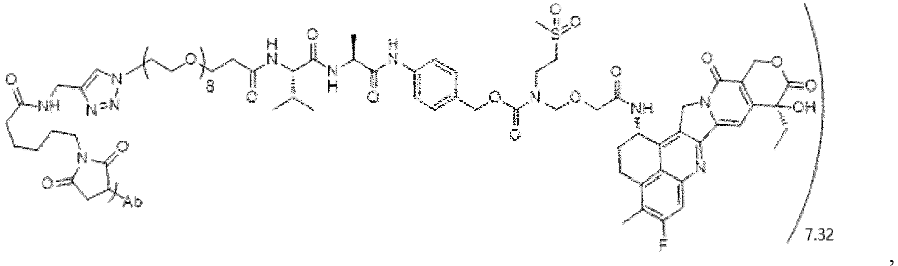 wherein Ab is the anti-DLL3 antibody comprising a VH with an amino acid sequence as shown in SEQ ID NO: 15 and a VL with an amino acid sequence as shown in SEQ ID NO: 16; 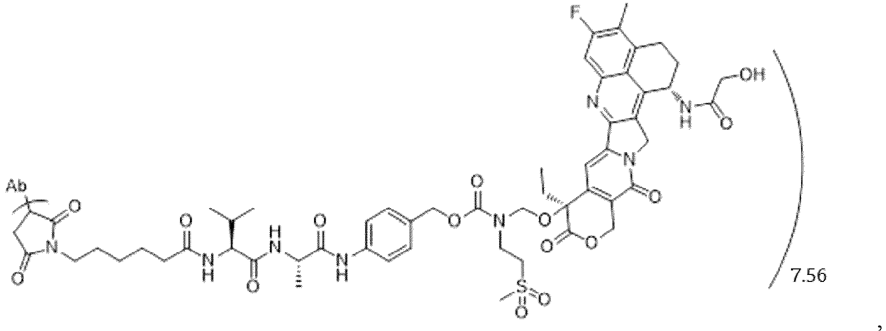 wherein Ab is the anti-DLL3 antibody comprising a VH with an amino acid sequence as shown in SEQ ID NO: 15 and a VL with an amino acid sequence as shown in SEQ ID NO: 16; 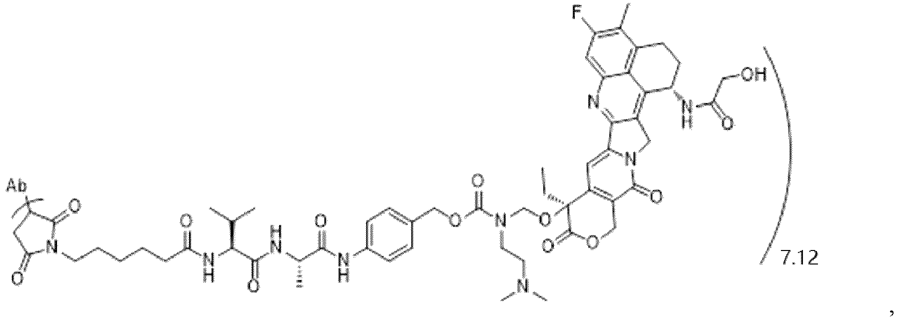 wherein Ab is the anti-DLL3 antibody comprising a VH with an amino acid sequence as shown in SEQ ID NO: 15 and a VL with an amino acid sequence as shown in SEQ ID NO: 16; 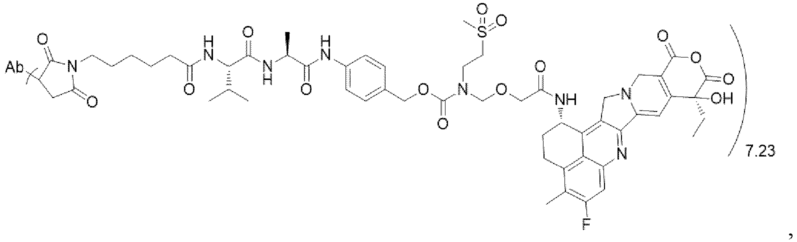 wherein Ab is the anti-DLL3 antibody comprising a heavy chain with an amino acid sequence as shown in SEQ ID NO: 2 and a light chain with an amino acid sequence as shown in SEQ ID NO: 1; 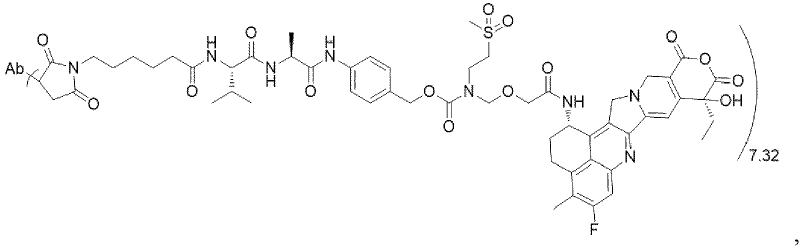 wherein Ab is the anti-DLL3 antibody comprising a heavy chain with an amino acid sequence as shown in SEQ ID NO: 4 and a light chain with an amino acid sequence as shown in SEQ ID NO: 3; 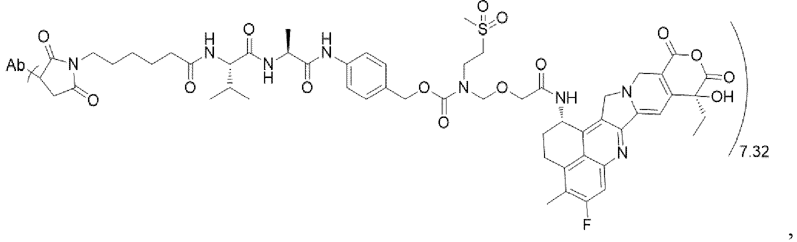 wherein Ab is the anti-DLL3 antibody comprising a VH with an amino acid sequence as shown in SEQ ID NO: 22, and a VL with an amino acid sequence as shown in SEQ ID NO: 23; or 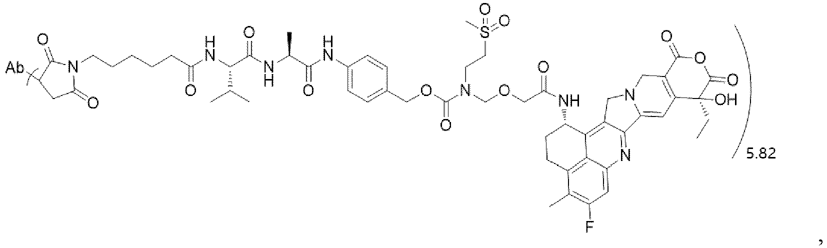 wherein Ab is the anti-DLL3 antibody comprising a VH with an amino acid sequence as shown in SEQ ID NO: 45, and a VL with an amino acid sequence as shown in SEQ ID NO: 46.

14. A chimeric antigen receptor comprising the anti-DLL3 antibody according to any one of claims 1 to 6.

15. A genetically modified cell comprising the anti-DLL3 antibody according to any one of claims 1 to 6; preferably, the genetically modified cell is a eukaryotic cell, preferably an isolated human cell; more preferably an immune cell such as a T cell, or NK cell.

16. A preparation method for the anti-DLL3 antibody comprising following steps: cultivating the transformant according to claim 9, and obtaining the anti-DLL3 antibody from the culture.

17. A pharmaceutical composition comprising the anti-DLL3 antibody according to any one of claims 1 to 6, the antibody-drug conjugate according to any one of claims 10 to 13, the chimeric antigen receptor according to claim 14, and/or the genetically modified cell according to claim 15;
preferably, the pharmaceutical composition is in a liquid, gas, solid, or semi-solid dosage formula, and/or, the pharmaceutical composition can be administered orally, by injection, nasally, transdermally, or via mucosal route;
more preferably, the pharmaceutical composition further comprises a combinational therapeutic agent comprising chemotherapeutic agents, radiation therapy agents, immunosuppressants, and/or cytotoxic drugs.

18. A use of the anti-DLL3 antibody according to any one of claims 1 to 6, the antibody-drug conjugate according to any one of claims 10 to 13, the chimeric antigen receptor according to claim 14, the genetically modified cell according to claim 15, and/or the pharmaceutical composition according to claim 17 in preparation of a medicament, a kit and/or a drug administration device for treatment and/or prevention of a disease related to abnormal expression of DLL3;
the diseases related to abnormal expression of DLL3 is preferably tumor; the tumor is preferably cancer; the cancer is preferably neuroendocrine tumor, more preferably small cell lung cancer.

19. A kit comprising the anti-DLL3 antibody according to any one of claims 1 to 6, the antibody-drug conjugate according to any one of claims 10 to 13, the chimeric antigen receptor according to claim 14, the genetically modified cell according to claim 15, and/or the pharmaceutical composition according to claim 17; and optionally, an instruction manual.

20. A drug administration device comprising: (1) an infusion module for administering the pharmaceutical composition according to claim 17 to a subject in need thereof, and (2) optionally, a pharmacodynamic monitoring module.

21. A method for detecting DLL3 comprising the step of using the anti-DLL3 antibody according to any one of claims 1 to 6 for detection; preferably, the method is used for non-diagnostic and/or non-therapeutic purposes.

22. A method for diagnosing, preventing and/or treating a disease related to abnormal expression of DLL3, comprising administering to a subject in need thereof with the anti-DLL3 antibody according to any one of claims 1 to 6, the antibody-drug conjugate according to any one of claims 10 to 13, the chimeric antigen receptor according to claim 14, the genetically modified cell according to claim 15 and/or the pharmaceutical composition according to claim 17.
